(19) Europäisches Patentamt
European Patent Office
Office européen des brevets

(11) EP 2 051 710 B1

(12) EUROPEAN PATENT SPECIFICATION

(45) Date of publication and mention of the grant of the patent:
**02.11.2011 Bulletin 2011/44**

(21) Application number: **07813637.1**

(22) Date of filing: **01.08.2007**

(51) Int Cl.:
*A61K 31/343* (2006.01) *A61K 31/404* (2006.01)
*A61K 31/4184* (2006.01) *A61K 31/4439* (2006.01)
*A61K 31/4709* (2006.01) *A61K 31/4725* (2006.01)
*A61K 45/06* (2006.01) *A61K 39/395* (2006.01)
*A61P 29/02* (2006.01) *A61P 29/00* (2006.01)

(86) International application number:
**PCT/US2007/074943**

(87) International publication number:
**WO 2008/021729 (21.02.2008 Gazette 2008/08)**

# (54) COMPOSITIONS AND METHODS FOR TREATING, REDUCING, AMELIORATING, OR ALLEVIATING POSTERIOR-SEGMENT OPHTHALMIC DISEASES

ZUSAMMENSETZUNGEN UND VERFAHREN ZUR BEHANDLUNG, VERMINDERUNG, VERBESSERUNG ODER LINDERUNG VON ERKRANKUNGEN DES HINTEREN AUGENSEGMENTS

COMPOSITIONS ET PROCÉDÉS POUR TRAITER, RÉDUIRE, AMÉLIORER OU SOULAGER DES MALADIES OPHTALMIQUES DU SEGMENT POSTÉRIEUR

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **07.08.2006 US 836078 P**

(43) Date of publication of application:
**29.04.2009 Bulletin 2009/18**

(73) Proprietor: **Bausch & Lomb Incorporated**
**Rochester, NY 14604-2701 (US)**

(72) Inventors:
- **KERPPOLA, Raili**
  **Ann Arbor, MI 48104 (US)**
- **PHILLIPS, Gary**
  **Pittsford, NY 14534 (US)**
- **HU, Zhenze**
  **Pittsford, New-York 14534 (US)**
- **WARD, Keith Wayne**
  **Ontario, NY 14519 (US)**

(74) Representative: **Glas, Holger et al**
**Maiwald Patentanwalts GmbH**
**Elisenhof**
**Elisenstrasse 3**
**80335 München (DE)**

(56) References cited:
**WO-A-2004/005229** **WO-A-2004/110385**
**WO-A-2006/050998** **US-A1- 2004 121 014**
**US-B1- 6 316 465**

- **MICHEL SHAWKAT SHAFIK ET AL: "Multifocal choroiditis and panuveitis: immunomodulatory therapy." OPHTHALMOLOGY FEB 2002, vol. 109, no. 2, February 2002 (2002-02), pages 378-383, XP002483909 ISSN: 0161-6420**
- **JAMPOL L M ET AL: "Treatment of juxtafoveal and extrafoveal choroidal neovascularization in the era of photodynamic therapy with verteporfin" AMERICAN JOURNAL OF OPHTHALMOLOGY 2002 US, vol. 134, no. 1, 2002, pages 99-101, XP002483910 ISSN: 0002-9394**
- **TENNANT M T ET AL: "Photodynamic Therapy (PDT) in Multifocal Choroiditis (MFC)." ARVO ANNUAL MEETING ABSTRACT SEARCH AND PROGRAM PLANNER, vol. 2002, 2002, page Abstract No. 595, XP008092817 & ANNUAL MEETING OF THE ASSOCIATION FOR RESEARCH IN VISION AND OPHTHALMOLOGY; FORT LAUDERDALE, FLORIDA, USA; MAY 05-10, 2002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 051 710 B1

## Description

BACKGROUND OF THE INVENTION

The present invention relates to compositions for use in treating, reducing, ameliorating, or alleviating posterior-segment ophthalmic diseases as defined in the appended claims. In particular, the present invention relates to compositions that comprise dissociated glucocorticoid receptor agonists ("DIGRAs") for use in the treatment, reduction, amelioration, or alleviation of posterior-segment ophthalmic diseases using such as defined in the appended claims. In addition, the present invention relates to compositions for use in treating, reducing, ameliorating, or alleviating posterior-segment ophthalmic diseases that have etiology in inflammation as defined in the appended claims.

Ophthalmic conditions may be classified as front-of-eye diseases, such as corneal edema, anterior uveitis, pterygium, corneal diseases, or opacifications with an exudative or inflammatory component, conjunctivitis, allergy- and laser-induced exudation, or back-of-eye diseases such as exudative macular degeneration, macular edema, diabetic retinopathy, age-related macular degeneration, or retinopathy of prematurity. Back-of-eye diseases comprise the largest number of causes for vision loss. There has been growing evidence that many back-of-the eye diseases have etiology in inflammation.

Among the back-of-the-eye diseases, diabetic retinopathy is the leading cause of blindness in adults between the ages of 18 to 72 who suffer from diabetes. In the early stage, the vasculature of the retina is increasingly obstructed by the adhesion of cells involved in immunological response, such as leucocytes, on molecules, such as intercellular adhesion molecule-1 (UICAM-1") or vascular cell adhesion molecule-1 ("VCAM-1"), which are overexpressed on the endothelial layer of inflammed vasculature. The vasculature obstruction results in ischemia and leads to hypoxia condition in the surrounding tissues, especially the retina. In response to such a condition, new blood vessels begin to proliferate uncontrollably. These new blood vessels are typically leaky, resulting in fluid accumulation under the retina and eventually the vision-threatening condition known as macular edema. (See; e.g., A.P. Adamis, British J. Ophthalmol., Vol. 86, 363 (2002); S. Ishida et al., Invest. Ophthalmol. & Visual Sci., Vol. 44, No. 5, 2155 (2003).) Vascular endothelial growth factor ("VEGF'), a hypoxia-induced proinflammatory angiogenic factor, has been found at elevated levels in the diabetic retina during both the nonproliferative and proliferative stage. VEGF also induces the expression of ICAM-1 and VCAM-1 on endothelial cells. (I. Kim et al., Biol. Chem., Vol. 276, No. 10, 7614 (2001).) In addition, experimental investigations in animals have shown that mRNA expression for the proinflammatory cytokines IL-1 (interleukin-1) and TNF-$\alpha$ (tumor necrosis factor-$\alpha$) is increased in the retina early in the course of diabetes, and moreover, inhibition of TNF-$\alpha$ has demonstrated beneficial effects in the prevention of diabetic retinopathy. (J.F. Navarro and C. Mora, Nephrol. Dial. Transplant, Vol. 20, 2601 (2005).) Thus, experimental evidence strongly suggests a central and causal role of chronic inflammation in the pathogenesis of diabetic retinopathy and macular edema. (A.M. Joussen et al., FASEB J., Vol. 18, 1450 (2004).)

Macular degeneration, another back-of-the-eye degenerative condition, is the most common cause of central vision loss in those 50 or older, and its prevalence increases with age. Age-related macular degeneration ("AMD") is the more common form of the condition. The other form, which is sometimes called "juvenile macular degeneration" ("JMD") is most commonly caused by an inherited condition. It is estimated that 50 million people worldwide suffer from AMD. It has recently been discovered that mutations in two genes encoding proteins in the so-called complement cascade account for most of the risk of developing AMD. This complex molecular pathway is the body's first line of defense against invading bacteria, but if overactive, the pathway can produce tissue-damaging inflammation, which underlies the vision-destroying changes that particularly strike the macula. Proteins associated with immune system activity have been found in or near drusen in eyes with AMD. Over time, the drusen grow as they accumulate inflammatory proteins and other materials, and the inflammation persists, causing additional damage to the retina and eventual vision loss. (See; e.g., Science, Vol. 311, 1704 (2006).)

Other back-of-the-eye conditions include "posterior uveitis," which is a term given to a collection of inflammatory conditions associated with the posterior segment of the eye. Posterior uveitis includes, but is not limited to, choroiditis (inflammation of the choroid), retinitis (inflammation of the retina), optic neuritis (inflammation of the optic nerve), and vasculitis (inflammation of the blood vessels at the back of the eye). Ocular complications of posterior uveitis may produce profound and irreversible loss of vision, when unrecognized or treated improperly. The most frequent complications include glaucoma, retinal detachment, neovascularization of the retina or optic nerve, and cystoid macular edema (the most common cause of decreased vision from uveitis).

Thus, it has been established that the cause of a great number of serious back-of-the-eye conditions may be traced to inflammation.

Glucocorticoids (also referred to herein as "corticosteroids") have been under investigation for use as a local therapeutic treatment for diabetic retinopathy. (See; e.g., M.A. Speicher et al., Expert Opinion on Emerging Drugs, Vol. 8, No. 1, 239 (2003); E.A. Felinski and D.A. Antonetti, Curr. Eye Research, Vol. 30, No. 11, (49 (2005); and G.M. Corner and T.A. Ciulla, Expert Opinion on Emerging Drugs, Vol. 10, No. 2, 441 (2005).) Intravitreal injection of corticosteroids

(especially triamcinolone acetonide) has been investigated as a treatment for diabetic macular edema (see; e.g., V. Vasumathy et al., Ophthalmic Practice, Vol. 54, No. 2, 133 (2006); P. Massin et al., Ophthalmology, Vol. 111, No. 2, 218 (2004)). Periocular injection of corticosteroids has been investigated as a treatment for posterior uveitis (see; e.g., W.W. Lai et al., Clin. Experiment Ophthalmol., Vol. 32, No. 6, 563 (2004). A Phase-III clinical trial of periocular injection of triamcinolone acetonide as adjunct therapy to photodynamic therapy ("PDT") for neovascular AMD was completed by Johns Hopkins University School of Medicine and Oregon Health Science University in 2006 (see, www.clinicaltrials.gov). However, steroidal drugs can have side effects that threaten the overall health of the patient.

It is known that certain glucocorticoids have a greater potential for elevating intraocular pressure ("IOP") than other compounds in this class. For example, it is known that prednisolone, which is a very potent ocular anti-inflammatory agent, has a greater tendency to elevate IOP than fluorometholone, which has moderate ocular anti-inflammatory activity. It is also known that the risk of IOP elevations associated with the topical ophthalmic use of glucocorticoids increases over time. In other words, the chronic (i.e., long-term) use of these agents increases the risk of significant IOP elevations. Unlike bacterial infections or acute ocular inflammation associated with physical trauma, which requires short-term therapy on the order of a few weeks, back-of-the-eye conditions require treatment for extended periods of time, generally several months or more. This chronic use of corticosteroids significantly increases the risk of IOP elevations. In addition, use of corticosteroids is also known to increase the risk of cataract formation in a dose- and duration-dependent manner. Once cataracts develop, they may progress despite discontinuation of corticosteroid therapy.

Chronic administration of glucocorticoids also can lead to drug-induced osteoporosis by suppressing intestinal calcium absorption and inhibiting bone formation. Other adverse side effects of chronic administration of glucocorticoids include hypertension, hyperglycemia, hyperlipidemia (increased levels of triglycerides) and hypercholesterolemia (increased levels of cholesterol) because of the effects of these drugs on the body metabolic processes.

Therefore, there is a continued need to provide pharmaceutical compounds and compositions to treat, reduce, or ameliorate back-of-the-eye conditions, which compounds and compositions cause a lower level of at least an adverse side effect than at least a prior-art glucocorticoid used to treat, reduce, or ameliorate the same condition.

Derivatives of 5-substituted quinoline and isoquinoline, a method for their production as well as their use as antiphlogistics are described in WO 2006/050998.

## SUMMARY OF THE INVENTION

The present invention is defined in the appended claims. Subject-matters which are not encompassed by the scope of the claims do not form part of the present invention.

In general, the present invention provides pharmaceutical compounds ans compositions as defined in the claims for use in treating, reducing or ameliorating in a subject a back-of-the-eye condition or disorder as defined in the claims, which compounds and compositions cause a lower level of at least an adverse side effect than at least a prior-art glucocorticoid used to treat, reduce, or ameliorate the same condition or disorder.

In one aspect, such a back-of-the-eye condition or disorder has an etiology in inflammation.

In another aspect, such a condition or disorder is selected from the group consisting of diabetic retinopathy ("DR"), age-related macular degeneration ("AMD," including dry and wet AMD), diabetic macular edema ("DME"), and combinations thereof.

In still another aspect, the pharmaceutical compounds and compositions comprise at least a dissociated glucocorticoid receptor agonist ("DIGRA"), or a pharmaceutically acceptable salt thereof as defined in the claims.

In yet another aspect, a composition for use in treating, reducing, ameliorating, or alleviating a back-of-the-eye condition or disorder that has an etiology in inflammation comprises: (a) a DIGRA or a pharmaceutically acceptable salt thereof; and (b) a material selected from the group consisting of anti-inflammatory agents other than a DIGRA and a pharmaceutically acceptable salt thereof.

In yet another aspect, a pharmaceutical composition of the present invention comprises an ophthalmic topical formulation; injectable formulation; or implantable formulation, system, or device.

In a further aspect, said at least an adverse side effect is demonstrated in vitro or in vivo.

## DETAILED DESCRIPTION OF THE INVENTION

The present invention is defined in the appended claims. Subjet-matters which are not encompassed by the scope of the claims do not form part of the present invention.

As used herein, a dissociated glucocorticoid receptor agonist ("DIGRA") is a compound that is capable of binding to the glucocorticoid receptor (which is a polypeptide) and, upon binding, is capable of producing differentiated levels of transrepression and transactivation of gene expression. A compound that binds to a polypeptide is sometimes herein referred to as a ligand.

As used herein, the term "alkyl" or "alkyl group" means a linear- or branched-chain saturated aliphatic hydrocarbon monovalent group, which may be unsubstituted or substituted. The group may be partially or completely substituted with halogen atoms (F, Cl, Br, or I). Examples of alkyl groups include methyl, ethyl, n-propyl, 1-methylethyl(isopropyl), n-butyl, n-pentyl, 1,1-dimethylethyl (t-butyl). It may be abbreviated as "Alk".

As used herein, the term "alkenyl" or "alkenyl group" means a linear- or branched-chain aliphatic hydrocarbon monovalent radical containing at least one carbon-carbon double bond. This term is exemplified by groups such as ethenyl, propenyl, n-butenyl, isobutenyl, 3-methylbut-2-enyl, n-pentenyl, heptenyl, octenyl, decenyl.

As used herein, the term "alkynyl" or "alkynyl group" means a linear- or branched-chain aliphatic hydrocarbon monovalent radical containing at least one carbon-carbon triple bond. This term is exemplified by groups such as ethynyl, propynyl, n-butynyl, 2-butynyl, 3-methylbutynyl, n-pentynyl, heptynyl, octynyl, decynyl.

As used herein, the term "alkylene" or "alkylene group" means a linear- or branched-chain saturated aliphatic hydrocarbon divalent radical having the specified number of carbon atoms. This term is exemplified by groups such as methylene, ethylene, propylene, n-butylene, and may alternatively and equivalently be denoted herein as -(alkyl)-.

The term "alkenylene" or "alkenylene group" means a linear- or branched-chain aliphatic hydrocarbon divalent radical having the specified number of carbon atoms and at least one carbon-carbon double bond. This term is exemplified by groups such as ethenylene, propenylene, n-butenylene, and may alternatively and equivalently be denoted herein as -(alkylenyl)-.

The term "alkynylene" or "alkynylene group" means a linear- or branched-chain aliphatic hydrocarbon divalent radical containing at least one carbon-carbon triple bond. This term is exemplified by groups such as ethynylene, propynylene, n-butynylene, 2-butynylene, 3-methylbutynylene, n-pentynylene, heptynylene, octynylene, decynylene, and may alternatively and equivalently be denoted herein as - (alkynyl)-.

As used herein, the term "aryl" or "aryl group" means an aromatic carbocyclic monovalent or divalent radical of from 5 to 14 carbon atoms having a single ring (e.g., phenyl or phenylene), multiple condensed rings (e.g., naphthyl or anthranyl), or multiple bridged rings (e.g., biphenyl). Unless otherwise specified, the aryl ring may be attached at any suitable carbon atom which results in a stable structure and, if substituted, may be substituted at any suitable carbon atom which results in a stable structure. Examples of aryl groups include phenyl, naphthyl, anthryl, phenanthryl, indanyl, indenyl, biphenyl. It may be abbreviated as "Ar".

The term "heteroaryl" or "heteroaryl group" means a stable aromatic 5- to 14-membered, monocyclic or polycyclic monovalent or divalent radical, which may comprise one or more fused or bridged ring(s), preferably a 5- to 7-membered monocyclic or 7- to 10-membered bicyclic radical, having from one to four heteroatoms in the ring(s) independently selected from nitrogen, oxygen, and sulfur, wherein any sulfur heteroatoms may optionally be oxidized and any nitrogen heteroatom may optionally be oxidized or be quaternized. Unless otherwise specified, the heteroaryl ring may be attached at any suitable heteroatom or carbon atom which results in a stable structure and, if substituted, may be substituted at any suitable heteroatom or carbon atom which results in a stable structure. Examples of heteroaryls include furanyl, thienyl, pyrrolyl, oxazolyl, thiazolyl, imidazolyl, pyrazolyl, isoxazolyl, isothiazolyl, oxadiazolyl, triazolyl, tetrazolyl, thiadiazolyl, pyridinyl, pyridazinyl, pyrimidinyl, pyrazinyl, triazinyl, indolizinyl, azaindolizinyl, indolyl, azaindolyl, diazaindolyl, dihydroindolyl, dihydroazaindoyl, isoindolyl, azaisoindolyl, benzofuranyl, furanopyridinyl, furanopyrimidinyl, furanopyrazinyl, furanopyridazinyl, dihydrobenzofuranyl, dihydrofuranopyridinyl, dihydrofuranopyrimidinyl, benzothienyl, thienopyridinyl, thienopyrimidinyl, thienopyrazinyl, thienopyridazinyl, dihydrobenzothienyl, dihydrothienopyridinyl, dihydrothienopyrimidinyl, indazolyl, azaindazolyl, diazaindazolyl, benzimidazolyl, imidazopyridinyl, benzthiazolyl, thiazolopyridinyl, thiazolopyrimidinyl, benzoxazolyl, benzoxazinyl, benzoxazinonyl, oxazolopyridinyl, oxazolopyrimidinyl, benzisoxazolyl, purinyl, chromanyl, azachromanyl, quinolizinyl, quinolinyl, dihydroquinolinyl, tetrahydroquinolinyl, isoquinolinyl, dihydroisoquinolinyl, tetrahydroisoquinolinyl, cinnolinyl, azacinnolinyl, phthalazinyl, azaphthalazinyl, quinazolinyl, azaquinazolinyl, quinoxalinyl, azaquinoxalinyl, naphthyridinyl, dihydronaphthyridinyl, tetrahydronaphthyridinyl, pteridinyl, carbazolyl, acridinyl, phenazinyl, phenothiazinyl, and phenoxazinyl.

The term "heterocycle", "heterocycle group", "heterocyclyl", "heterocyclyl group", "heterocyclic", or "heterocyclic group" means a stable non-aromatic 5- to 14-membered monocyclic or polycyclic, monovalent or divalent, ring which may comprise one or more fused or bridged ring(s), preferably a 5- to 7-membered monocyclic or 7- to 10-membered bicyclic ring, having from one to three heteroatoms in at least one ring independently selected from nitrogen, oxygen, and sulfur, wherein any sulfur heteroatoms may optionally be oxidized and any nitrogen heteroatom may optionally be oxidized or be quaternized. As used herein, a heterocyclyl group excludes heterocycloalkyl, heterocycloalkenyl, and heterocycloalkynyl groups. Unless otherwise specified, the heterocyclyl ring may be attached at any suitable heteroatom or carbon atom which results in a stable structure and, if substituted, may be substituted at any suitable heteroatom or carbon atom which results in a stable structure. Examples of heterocycles include pyrrolinyl, pyrrolidinyl, pyrazolinyl, pyrazolidinyl, piperidinyl, morpholinyl, thiomorpholinyl, piperazinyl, tetrahydropyranyl, tetrahydrothiopyranyl, tetrahydrofuranyl, hexahydropyrimidinyl, hexahydropyridazinyl.

The term "cycloalkyl" or "cycloalkyl group" means a stable aliphatic saturated 3- to 15-membered monocyclic or polycyclic monovalent radical consisting solely of carbon and hydrogen atoms which may comprise one or more fused or bridged ring(s), preferably a 5- to 7-membered monocyclic or 7- to 10-membered bicyclic ring. Unless otherwise

specified, the cycloalkyl ring may be attached at any carbon atom which results in a stable structure and, if substituted, may be substituted at any suitable carbon atom which results in a stable structure. Exemplary cycloalkyl groups include cyclopropyl, cyclobutyl, cyclopentyl, cyclohexyl, cycloheptyl, cyclooctyl, cyclononyl, cyclodecyl, norbornyl, adamantyl, tetrahydronaphthyl (tetralin), 1-decalinyl, bicyclo[2.2.2]octanyl, 1-methylcyclopropyl, 2-methylcyclopentyl, 2-methylcyclooctyl.

The term "cycloalkenyl" or "cycloalkenyl group" means a stable aliphatic 5-to 15-membered monocyclic or polycyclic monovalent radical having at least one carbon-carbon double bond and consisting solely of carbon and hydrogen atoms which may comprise one or more fused or bridged ring(s), preferably a 5- to 7-membered monocyclic or 7- to 10-membered bicyclic ring. Unless otherwise specified, the cycloalkenyl ring may be attached at any carbon atom which results in a stable structure and, if substituted, may be substituted at any suitable carbon atom which results in a stable structure. Exemplary cycloalkenyl groups include cyclopentenyl, cyclohexenyl, cycloheptenyl, cyclooctenyl, cyclononenyl, cyclodecenyl, norbornenyl, 2-methylcyclopentenyl, 2-methylcyclooctenyl.

The term "cycloalkynyl" or "cycloalkynyl group" means a stable aliphatic 8-to 15-membered monocyclic or polycyclic monovalent radical having at least one carbon-carbon triple bond and consisting solely of carbon and hydrogen atoms which may comprise one or more fused or bridged ring(s), preferably a 8- to 10-membered monocyclic or 12- to 15-membered bicyclic ring. Unless otherwise specified, the cycloalkynyl ring may be attached at any carbon atom which results in a stable structure and, if substituted, may be substituted at any suitable carbon atom which results in a stable structure. Exemplary cycloalkynyl groups include cyclooctynyl, cyclononynyl, cyclodecynyl, 2-methylcyclooctynyl.

The term "carbocycle" or "carbocyclic group" means a stable aliphatic 3- to 15-membered monocyclic or polycyclic monovalent or divalent radical consisting solely of carbon and hydrogen atoms which may comprise one or more fused or bridged rings, preferably a 5- to 7-membered monocyclic or 7- to 10-membered bicyclic ring. Unless otherwise specified, the carbocycle may be attached at any carbon atom which results in a stable structure and, if substituted, may be substituted at any suitable carbon atom which results in a stable structure. The term comprises cycloalkyl (including spiro cycloalkyl), cycloalkylene, cycloalkenyl, cycloalkenylene, cycloalkynyl, and cycloalkynylene.

The terms "heterocycloalkyl", "heterocycloalkenyl", and "heterocycloalkynyl" mean cycloalkyl, cycloalkenyl, and cycloalkynyl group, respectively, having at least a heteroatom in at least one ring, respectively.

Glucocorticoids ("GCs") are among the most potent drugs used for the treatment of allergic and chronic inflammatory diseases. However, as mentioned above, long-term treatment with GCs is often associated with numerous adverse side effects, such as diabetes, osteoporosis, hypertension, glaucoma, or cataract. These side effects, like other physiological manifestations, are results of aberrant expression of genes responsible for such diseases. Research in the last decade has provided important insights into the molecular basis of GC-mediated actions on the expression of GC-responsive genes. GCs exert most of their genomic effects by binding to the cytoplasmic GC receptor ("GR"). The binding of GC to GR induces the translocation of the GC-GR complex to the cell nucleus where it modulates gene transcription either by a positive (transactivation) or negative (transrepression) mode of regulation. There has been growing evidence that both beneficial and undesirable effects of GC treatment are the results of undifferentiated levels of expression of these two mechanisms; in other words, they proceed at similar levels of effectiveness. Although it has not yet been possible to ascertain the most critical aspects of action of GCs in chronic inflammatory diseases, there has been evidence that it is likely that the inhibitory effects of GCs on cytokine synthesis are of particular importance. GCs inhibit the transcription, through the transrepression mechanism, of several cytokines that are relevant in inflammatory diseases, including IL-1$\beta$ (interleukin-1$\beta$), IL-2, IL-3, IL-6, IL-11, TNF-$\alpha$ (tumor necrosis factor-$\alpha$), GM-CSF (granulocyte-macrophage colony-stimulating factor), and chemokines that attract inflammatory cells to the site of inflammation, including IL-8, RANTES, MCP-1 (monocyte chemotactic protein-1), MCP-3, MCP-4, MIP-1a (macrophage-inflammatory protein-1$\alpha$), and eotaxin. P.J. Barnes, Clin. Sci., Vol. 94, 557-572 (1998). On the other hand, there is persuasive evidence that the synthesis of I$\kappa$B kinases, which are proteins having inhibitory effects on the NF-$\kappa$B proinflammatory transcription factors, is increased by GCs. These proinflammatory transcription factors regulate the expression of genes that code for many inflammatory proteins, such as cytokines, inflammatory enzymes, adhesion molecules, and inflammatory receptors. S. Wissink et al., Mol. Endocrinol., Vol. 12, No. 3, 354-363 (1998); P.J. Barnes and M. Karin, New Engl. J. Med., Vol. 336, 1066-1077 (1997). Thus, both the transrepression and transactivation functions of GCs directed to different genes produce the beneficial effect of inflammatory inhibition. On the other hand, steroid-induced diabetes and glaucoma appear to be produced by the transactivation action of GCs on genes responsible for these diseases. H. Schäcke et al., Pharmacol. Ther., Vol. 96, 23-43 (2002). Thus, while the transactivation of certain genes by GCs produces beneficial effects, the transactivation of other genes by the same GCs can produce undesired side effects. Therefore, it is very desirable to provide pharmaceutical compounds and compositions that produce differentiated levels of transactivation and transrepression activity on GC-responsive genes to treat, reduce, or ameliorate chronic inflammatory conditions.

In general, the present invention provides pharmaceutical compounds and compositions for use in treating, reducing, or ameliorating in a subject a back-of the-eye condition or disorder as defined in the claims which compounds and compositions cause a lower level of at least an adverse side effect than at least a prior-art glucocorticoid used to treat, reduce, or ameliorate the same condition or disorder. In one aspect, such a condition or disorder has an etiology

in inflammation. In another aspect, such a condition or disorder has an etiology in chronic inflammation.

In one aspect, a level of said at least an adverse side effect is determined in vivo or in vitro. For example, a level of said at least an adverse side effect is determined in vitro by performing a cell culture and determining the level of a biomarker associated with said side effect. Such biomarkers can include proteins (e.g., enzymes), lipids, sugars, and derivatives thereof that participate in, or are the products of, the biochemical cascade resulting in the adverse side effect. Representative in vitro testing methods are further disclosed hereinbelow.

In another aspect, said at least an adverse side effect is selected from the group consisting of glaucoma, cataract, hypertension, hyperglycemia, hyperlipidemia (increased levels of triglycerides), and hypercholesterolemia (increased levels of cholesterol). A side effect such as hypertension, hyperglycemia, hyperlipidemia, or hypercholesterolemia can be a systemic side effect. In one embodiment, a level of said at least an adverse side effect is determined at about one day after said compounds or compositions are first administered to, and are present in, said subject. In another embodiment, a level of said at least an adverse side effect is determined about 14 days after said compounds or compositions are first administered to, and are present in, said subject. In still another embodiment, a level of said at least an adverse side effect is determined about 30 days after said compounds or compositions are first administered to, and are present in, said subject. Alternatively, a level of said at least an adverse side effect is determined about 2, 3, 4, 5, or 6 months after said compounds or compositions are first administered to, and are present in, said subject.

In another aspect, said at least a prior-art glucocorticoid used to treat, reduce, ameliorate, or alleviate the same condition or disorder is administered to said subject at a dose and a frequency sufficient to produce an equivalent beneficial effect on said condition or disorder as a compound or composition of the present invention after about the same elapsed time.

In still another aspect, said at least a prior-art glucocorticoid is selected from the group consisting of 21-acetoxypregnenolone, alclometasone, algestone, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, dexamethasone, diflorasone, diflucortolone, difluprednate, enoxolone, fluazacort, flucloronide, flumethasone, flunisolide, fluocinolone acetonide, fluocinonide, fluocortin butyl, fluocortolone, fluorometholone, fluperolone acetate, fluprednidene acetate, fluprednisolone, flurandrenolide, fluticasone propionate, formocortal, halcinonide, halobetasol propionate, halometasone, halopredone acetate, hydrocortarnate, hydrocortisone, loteprednol etabonate, mazipredone, medrysone, meprednisone, methylprednisolone, mometasone furoate, paramethasone, prednicarbate, prednisolone, prednisolone 25-diethylamino-acetate, prednisolone sodium phosphate, prednisone, prednival, prednylidene, rimexolone, tixocortol, triamcinolone, triamcinolone acetonide, triamcinolone benetonide, triamcinolone hexacetonide, their physiologically acceptable salts, combinations thereof, and mixtures thereof. In one embodiment, said at least a prior-art glucocorticoid is selected from the group consisting of dexamethasone, prednisone, prednisolone, methylprednisolone, medrysone, triamcinolone, loteprednol etabonate, physiologically acceptable salts thereof, combinations thereof, and mixtures thereof. In another embodiment, said at least a prior-art glucocorticoid is acceptable for ophthalmic uses.

Also disclosed are pharmaceutical compounds and compositions which comprise at least a mimetic of a glucocorticoid in treating, reducing, ameliorating, or alleviating such a condition or disorder.

In another aspect of the present disclosure, the pharmaceutical compounds and compositions comprise at least a dissociated glucocorticoid receptor agonist ("DIGRA"). As used herein, a DIGRA can comprise any enantiomer of the molecule or a racemic mixture of the enantiomers.

In still another aspect, the pharmaceutical compounds and compositions comprise a prodrug or a pharmaceutically acceptable salt of at least a DIGRA.

In still another aspect, said at least a DIGRA has Formula I.

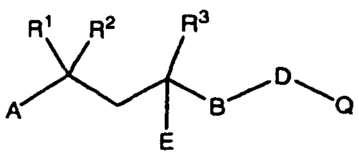

**(I)**

wherein A and Q are independently selected from the group consisting of unsubstituted and substituted aryl and heteroaryl groups, unsubstituted and substituted cycloalkyl and heterocycloalkyl groups, unsubstituted and substituted cycloalkenyl and heterocycloalkenyl groups, unsubstituted and substituted cycloalkynyl and heterocycloalkynyl groups, and unsubstituted and substituted heterocyclic groups; $R^1$ and $R^2$ are independently selected from the group consisting of hydrogen, unsubstituted $C_1$-$C_{15}$ (alternatively, $C_1$-$C_{10}$, or $C_1$-$C_5$, or $C_1$-$C_3$) linear or branched alkyl groups, substituted $C_1$-$C_{15}$ (alternatively, $C_1$-$C_{10}$, or $C_1$-$C_5$, or $C_1$-$C_3$) linear or branched alkyl groups, unsubstituted $C_3$-$C_{15}$ cycloalkyl groups, and substituted $C_3$-$C_{15}$ (alternatively, $C_3$-$C_6$, or $C_3$-$C_5$) cycloalkyl groups; $R^3$ is selected from the group consisting of hydrogen,

unsubstituted $C_1$-$C_{15}$ (alternatively, $C_1$-$C_{10}$, or $C_1$-$C_5$, or $C_1$-$C_3$) linear or branched alkyl groups, substituted $C_1$-$C_{15}$ (alternatively, $C_1$-$C_{10}$, or $C_1$-$C_5$, or $C_1$-$C_3$) linear or branched alkyl groups, unsubstituted $C_3$-$C_{15}$ (alternatively, $C_3$-$C_6$, or $C_3$-$C_5$) cycloalkyl and heterocycloalkyl groups, substituted $C_3$-$C_{15}$ (alternatively, $C_3$-$C_6$, or $C_3$-$C_5$) cycloalkyl and heterocycloalkyl groups, aryl groups, heteroaryl groups, and heterocyclylic groups; B comprises a carbonyl, amino, divalent hydrocarbon, or heterohydrocarbon group; E is hydroxy or amino group; and D is absent or comprises a carbonyl group, -NH-, or -NR'-, wherein R' comprises an unsubstituted or substituted $C_1$-$C_{15}$ (alternatively, $C_1$-$C_{10}$, or $C_1$-$C_5$, or $C_1$-$C_3$) linear or branched alkyl group; and wherein $R^1$ and $R^2$ together may form an unsubstituted or substituted $C_3$-$C_{15}$ cycloalkyl group.

In one embodiment, B can comprise one or more unsaturated carbon-carbon bonds.

In another embodiment, B can comprise an alkylenecarbonyl, alkyleneoxycarbonyl, al ky lenecarbony lox y, alkyleneoxycarbonylamino, alkyleneamino, alkenylenecarbonyl, alkenyleneoxycarbonyl, alkenylenecarbonyloxy, alkenyleneoxycarbonylamino, alkenyleneamino, alkynylenecarbonyl, alkynyleneoxycarbonyl, alkynylenecarbonyloxy, alkynyleneoxycarbonylamino, alkynyleneamino, arylcarbonyloxy, aryloxycarbonyl, or ureido group.

In still another embodiment, A and Q are independently selected from the group consisting of aryl and heteroaryl groups substituted with at least a halogen atom, cyano group, hydroxy group, or $C_1$-$C_{10}$ alkoxy group (alternatively, $C_1$-$C_5$ alkoxy group, or $C_1$-$C_3$ alkoxy group); $R^1$, $R^2$, and $R^3$ are independently selected from the group consisting of unsubstituted and substituted $C_1$-$C_5$ alkyl groups (preferably, $C_1$-$C_3$ alkyl groups); B is a $C_1$-$C_5$ alkylene group (alternatively, $C_1$-$C_3$ alkyl groups); D is the -NH- or -NR'- group, wherein R' is a $C_1$-$C_5$ alkyl group (preferably, $C_1$-$C_3$ alkyl group); and E is the hydroxy group.

In yet another embodiment, A comprises a dihydrobenzofuranyl group substituted with a halogen atom; Q comprises a quinolinyl or isoquinolinyl group substituted with a $C_1$-$C_{10}$ alkyl group; $R^1$ and $R^2$ are independently selected from the group consisting of unsubstituted and substituted $C_1$-$C_5$ alkyl groups (preferably, $C_1$-$C_3$ alkyl groups); B is a $C_1$-$C_3$ alkylene group; D is the -NH- group; E is the hydroxy group; and $R^3$ comprises a completely halogenated $C_1$-$C_{10}$ alkyl group (preferably, completely halogenated $C_1$-$C_5$ alkyl group; more preferably, completely halogenated $C_1$-$C_3$ alkyl group).

In still another embodiment, A comprises a dihydrobenzofuranyl group substituted with a fluorine atom; Q comprises a quinolinyl or isoquinolinyl group substituted with a methyl group; $R^1$ and $R^2$ are independently selected from the group consisting of unsubstituted and substituted $C_1$-$C_5$ alkyl groups; B is a $C_1$-$C_3$ alkylene group; D is the -NH- group; E is the hydroxy group; and $R^3$ comprises a trifluoromethyl group.

In a further embodiment, said at least a DIGRA has Formula II or III.

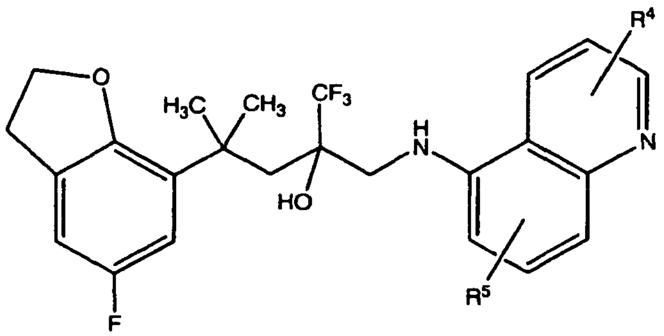

(II)

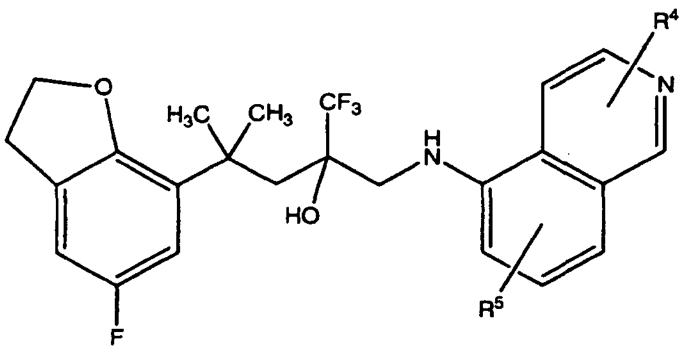

(III)

wherein $R^4$ and $R^5$ are independently selected from the group consisting of hydrogen, halogen, cyano, hydroxy, $C_1$-$C_{10}$ (alternatively, $C_1$-$C_5$ or $C_1$-$C_3$) alkoxy groups, unsubstituted $C_1$-$C_{10}$ (alternatively, $C_1$-$C_5$ or $C_1$-$C_3$) linear or branched

alkyl groups, substituted $C_1$-$C_{10}$ (alternatively, $C_1$-$C_5$ or $C_1$-$C_3$) linear or branched alkyl groups, unsubstituted $C_3$-$C_{10}$ (alternatively, $C_3$-$C_6$ or $C_3$-$C_5$) cyclic alkyl groups, and substituted $C_3$-$C_{10}$ (alternatively, $C_3$-$C_6$ or $C_3$-$C_5$) cyclic alkyl groups.

In still another embodiment, said at least a DIGRA has Formula IV.

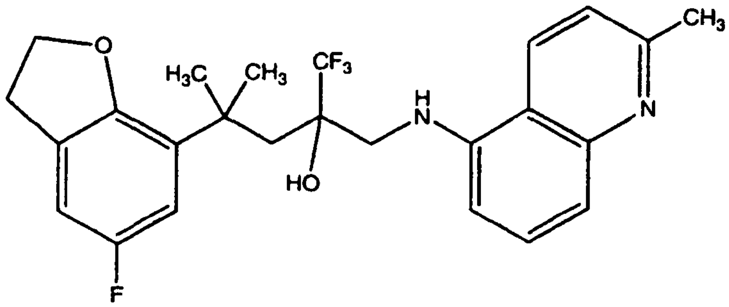

(IV)

Methods for preparing compounds of Formula I, II, III, or IV are disclosed, for example, in U.S. Patents 6,897,224; 6,903,215; 6,960,581. Still other methods for preparing such compounds also can be found in U.S. Patent Application Publication 2006/0116396 or PCT Patent Application WO 2006/050998 A1.

Examples of compounds having Formula I include 5-[4-(5-fluoro-2,3-dihydrobenzofuran-7-yl)-2-hydroxy-4-methyl-2-trifluoromethyl-pentylamino]-2-methylquinoline, 5-[4-(5-fluoro-2,3-dihydrobenzofuran-7-yl)-2-hydroxy-4-methyl-2-trifluoromethyl-pentylamino]-1-methylisoquinoline, 5-[4-(5-fluoro-2,3-dihydrobenzofuran-7-yl)-2-hydroxy-4-methyl-2-trifluoromethyl-pentylamino]isoquinol-1(2H)-one, 5-[4-(5-fluoro-2,3-dihydrobenzofuran-7-yl)-2-hydroxy-4-methyl-2-trifluoromethyl-pentylamino]-2,6-dimethylquinoline, 5-[4-(5-fluoro-2,3-dihydrobenzofuran-7-yl)-2-hydroxy-4-methyl-2-trifluoromethyl-pentylamino]-6-chloro-2-methylquinoline, 5-[4-(5-fluoro-2,3-dihydrobenzofuran-7-yl)-2-hydroxy-4-methyl-2-trifluoromethyl-pentylamino]isoquinoline, 5-[4-(5-fluoro-2,3-dihydrobenzofuran-7-yl)-2-hydroxy-4-methyl-2-trifluoromethyt-pentylamino]quinoline, 5-[4-(2,3-dihydro-5-fluoro-7-benzofuranyl)-2-hydroxy-4-methyl-2-trifluoromethyl-pentylamino]quinolin-2[1H]-one, 6-fluro-5-[4-(5-fluoro-2,3-dihydrobenzofuran-7-yl)-2-hydroxy-4-methyl-2-trifluoromethyl-pentylamino]-2-methylquinoline, 8-fluoro-,5-[4-(5-fluoro-2,3-dihydrobenzofuran-7-yl)-2-hydroxy-4-methyl-2-trifluoromethyl-pentylamino]-2-methylquinoline, 5-[4-(5-fluoro-2,3-dihydrobenzofuran-7-yl)-2-hydroxy-4-methyl-2-trifluoromethyl-pentylamino]-2-methylisoquinol-1-[2h]-one, and enantiomers thereof.

In yet another embodiment, said at least a DIGRA has Formula I, wherein

(a) A is an aryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is the trifluoromethyl group;

(d) B is $C_1$-$C_5$ alkyl, $C_2$-$C_5$ alkenyl, or $C_2$-$C_5$ alkynyl, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q is an azaindolyl group optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl,

aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, halogen, hydroxy, oxo, cyano, amino, and trifluoromethyl.

Examples of these compounds include 1,1,1-trifluoro-4-(5-fluoro-2-methoxyphenyl)-4-methyl-2-(1H-pyrrolo [2,3-c] pyridin-2-ylmethyl)pentan-2-ol; 1,1,1-trifluoro-4-(5-fluoro-2-methoxyphenyl)-4-methyl-2-(1H-pyrrolo[3,2-c] pyridin-2-ylmethyl)pentan-2-ol; 1,1,1-trifluoro-4-methyl-4-phenyl-2-(1H-pyrrolo[2,3-c]pyridin-2-ylmethyl)pentan-2-ol; 1,1,1-trifluoro-4-(5-fluoro-2-methoxyphenyl)-4-methyl-2-(1H-pyrrolo[2,3-c] pyridin-2-ylmethyl)pentan-2-ol; 1,1,1-trifluoro-4-methyl-4-phenyl-2-(1H-pyrrolo[3,2-c]pyridin-2-ylmethyl)pentan-2-ol; 1,1,1-trifluoro-4-(4-fluoro-2-methoxyphenyl)-4-methyl-2-(1H-pyrrolo[3,2-c] pyridin-2-ylmethyl)pentan-2-ol; 5-fluoro-2-[4,4,4-trifluoro-3-hydroxy-1,1-dimethyl-3-(1H-pyrrolo [2,3-c]pyridin-2-ylmethyl)butyl]phenol; 4-fluoro-2-[4,4,4-triftuoro-3-hydroxy-1,1-dimethyl-3-(1H-pyrrolo[2,3-c]pyridin-2-ylmethyl)butyl]phenol; 1,1,1-trifluoro-4-(5-fluoro-2-methoxyphenyl)-4-methyl-2-(1H-pyrrolo[3,2-c] pyridin-2-ylmethyl) pentan-2-ol; 1,1,1-trifluoro-4-(5-fluoro-2-methoxyphenyl)-4-methyl-2-(3-methyl-1H-pyrrolo[2,3,c]pyridin-2-ylmethyl) pentan-2-ol; and 4-fluoro-2-[4,4,4-trifluoro-3-hydroxy-1,1-dimethyl-3-(1H-pyrrolo[2,3-c]pyridin-2-ylmethyl)butyl]phenol.

In still another embodiment, said at least a DIGRA has Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) B is the methylene or carbonyl group;

(d) $R^3$ is a carbocycle, heterocyclyl, aryl, heteroaryl, carbocycle-$C_1$-$C_8$ alkyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups;

(e) D is the -NH- group;

(f) E is the hydroxy group; and

(g) Q comprises a methylated benzoxazinone.

Examples of these compounds include 2-benzyl-4-(5-fluoro-2-methoxyphenyl)-2-hydroxy-4-methylpentanoic acid(4-methyl-1-oxo-1H-benzo[d][1,2]oxazin-6-yl)amide; 2-benzyl-4-(5-fluoro-2-hydroxyphenyl)-2-hydroxy-4-methylpentanoic acid(4-methyl-1-oxo-1H-benzo[d][1,2]oxazin-6-yl)amide; 2-cyclohexylmethyl-4-(5-fluoro-2-methoxyphenyl)-2-hydroxy-4-methylpentanoic acid(4-methyl-1-oxo-1H-benzo[d][1,2]oxazin-6-yl)amide; 2-cyclohexylmethyl-4-(5-fluoro-2-hydroxyphenyl)-2-hydroxy-4-methylpentanoic acid(4-methyl- 1-oxo-1H-benzo[d][1,2]oxazin-6-yl)amide; 2-benzyl-2-hydroxy-4-methyl-4-methylpentanoic acid(4-methyl-1-oxo-1H-benzo[d][1,2]oxazin-6-yl)amide; and 2-cyclohexylmethyl-2-hydroxy-4-methylpentanoic acid(4-methyl-1-oxo-1H-benzo[d][1,2]oxazin-6-yl)amide.

In still another embodiment, said at least a DIGRA has Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$

alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is $C_1$-$C_5$ alkyl, $C_2$-$C_5$ alkenyl, or $C_2$-$C_5$ alkynyl, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q is an aryl or heteroaryl group one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, and trifluoromethyl.

Examples of these compounds include 2-(3,5-difluorobenzyl)-1,1,1-trifluoro-4-(5-fluoro-2-methoxyphenyl)-4-methylpentan-2-ol; 2-biphenyl-4-ylmethyl-1,1,1-trifluoro-4-(5-fluoro-2-methoxyphenyl)-4-methylpentan-2-ol; 2-(3,5-dimethylbenzyl)-1,1,1-trifluoro-4-(5-fluoro-2-methoxyphenyl)-4-methylpentan-2-ol; 2-(3-bromobenzyl)-1,1,1-trifluoro-4-(5-fluoro-2-methoxyphenyl)-4-methylpentan-2-ol; 2-(3,5-dichlorobenzyl)-1,1,1-trifluoro-4-(5-fluoro-2-methoxyphenyl)-4-methylpentan-2-ol; 2-(3,5-bis-trifluoromethylbenzyl)-1,1,1,trifluoro-4-(5-fluoro-2-methoxyphenyl)-4-methylpentan-2-ol; 1,1,1-trifluoro-4-(5-fluoro-2-methoxyphenyl)-2-(3-fluoro-5-trifluoromethylbenzyl)-4-methylpentan-2-ol; 2-(3-chloro-2-fluoro-5-trifluoromethylbenzyl-)-1,1,1-trifluoro-4-(5-fluoro-2-methoxyphenyl)-4-methylpentan-2-ol; 4-[4-(5-fluoro-2-methoxyphenyl)-2-hydroxy-4-methyl-2-trifluoromethylpentyl]benzonitrile; 2-(3,5-dibromobenzyl)-1,1,1-trifluoro-4-(5-fluoro-2-methoxyphenyl)-4-methylpentan-2-ol; 1,1,1-trifluoro-4-(5-fluoro-2-methoxyphenyl)-2-(2-fluoro-3-trifluoromethylbenzyl)-4-methylpentan-2-ol; 1,1,1-trifluoro-4-(5-fluoro-2-methoxyphenyl)-2-(2-fluoro-5-trifluoromethylbenzyl)-4-methylpentan-2-ol.

In still another embodiment, said at least a DIGRA has Formula I, wherein

(a) A is an aryl, heteroaryl, or $C_5$-$C_{15}$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen, $C_1$-$C_5$ alkyl, $C_5$-$C_{15}$ arylalkyl, or $R^1$ and $R^2$ together with the carbon atom they are commonly attached to form a $C_3$-$C_8$ spiro cycloalkyl ring;

(c) $R^3$ is the trifluoromethyl group;

(d) B is the carbonyl group or methylene group, which is optionally independently substituted with one or two substituent groups selected from $C_1$-$C_5$ alkyl, hydroxy, and halogen;

(e) D is absent;

(f) E is the hydroxy group or amino group wherein the nitrogen atom is optionally independently mono- or di-substituted by $C_1$-$C_5$ alkyl; and

(g) Q comprises a pyrrolidine, morpholine, thiomorpholine, piperazine, piperidine, 1H-pyridin-4-one, 1H-pyridin-2-one, 1H-pyridin-4-ylideneamine, 1H-quinolin-4-ylideneamine, pyran, tetrahydropyran, 1,4-diazepane, 2,5-diazabicyclo[2.2.1]heptane, 2,3,4,5-tetrahydrobenzo[b][1,4]diazepine, dihydroquinoline, tetrahydroquinoline, 5,6,7,8-tetrahydro-1H-quinolin-4-one, tetrahydroisoquinoline, decahydroisoquinoline, 2,3-dihydro-1H-isoindole, 2,3-dihydro-1H-indole, chroman, 1,2,3,4-tetrahydroquinoxaline, 1,2-dihydroindazol-3-one, 3,4-dihydro-2H-benzo[1,4]oxazine, 4H-benzo[1,4]thiazine, 3,4-dihydro-2H-benzo[1,4]thiazine, 1,2-dihydrobenzo[d] [1,3]oxazin4-one, 3,4-dihydrobenzo [1,4]oxazin4-one, 3H-quinazolin4-one, 3,4-dihydro-1H-quinoxalin-2-one, 1H-quinnolin-4-one, 1H-quinazolin4-one, 1H-[1,5]naphthyridin-4-one, 5,6,7,8-tetrahydro-1H-[1,- 5]naphthyridin-4-one, 2,3-dihydro-1H-[1,5]naphthyridin-4-one, 1,2-dihydropyrido[3,2-d][1,3]oxazin-4-one, pyrrolo[3,4-c]pyridine-1,3-dione, 1,2-dihydropyrrolo[3,4-c]pyridin-3-one, or tetrahydro[b][1,4]diazepinone group, each optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, oxo, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from $C_1$-$C_3$ alkyl, $C_1$-$C_3$ alkoxy, $C_1$-$C_3$ alkoxycarbonyl, acyl, aryl, benzyl, heteroaryl, heterocyclyl, halogen, hydroxy, oxo, cyano, amino wherein the nitrogen atom is optionally independently mono- or di-substituted by $C_1$-$C_5$ alkyl, or ureido wherein either nitrogen atom is optionally independently substituted with $C_1$-$C_5$ alkyl.

Examples of these compounds include 2-(2,6-dimethylmorpholin-4-ylmethyl)-1,1,1-trifluoro-4-(5-fluoro-2-methoxyphenyl)-4-methylpentan-2-ol; 1-[4-(5-fluoro-2-methoxyphenyl)-2-hydroxy-4-methyl-2-trifluoromethylpentyl]-1H-quinolin-4-one; 1-[4-(5-fluoro-2-methoxyphenyl)-2-hydroxy-4-methyl-2-trifluoromethylpentyl]-3,5-dimethylpiperidin-4-one; 1-[4-(5-fluoro-2-methoxyphenyl)-2-hydroxy-4-methyl-2-trifluoromethylpentyl]-3-methyl-1H-quinolin-4-one; 1-[4-(5-fluoro-2-methoxyphenyl)-2-hydroxy-4-methyl-2-trifluoromethylpentyl]-2,3-dihydro-IH-quinolin-4-one; 1-[4-(4-fluorophenyl)-2-hydroxy-4-methyl-2-trifluoromethylpentyl]-1H-quinolin-4-one; 1-[4-(3-fluorophenyl)-2-hydroxy-4-methyl-2-trifluoromethylpentyl]-1H-quinolin-4-one; 1-[4-(4-fluoro-2-hydroxyphenyl)-2-hydroxy-4-methyl-2-trifluoromethylpentyl]-1H-quinolin-4-one; 1-[4-phenyl-2-hydroxy-4-methyl--2-trifluoromethylpentyl]-1H-quinolin-4-one; 1-[4-(5-fluoro-2,3-dihydrobenzofuran-7-yl)-2-hydroxy-4-methyl-2-trifluoromethylpentyl]-1H-quinolin-4-one; 1-[4-(5-bromo-2,3-dihydrobenzofuran-7-yl)-2-hydroxy-4-methyl-2-trifluoromethylpentyl]-1H-quinolin-4-one; 1-[4-(5-methyl-2,3-dihydrobenzofuran-7-y-1)-2-hydroxy-4-methyl-2-trifluoromethylpentyl]-1H-quinolin-4-one; 1-[4-(5-chloro-2,3-dihydrobenzofuran-7-yl)-2-hydroxy-4-methyl-2-trifluoromethylpentyl]-1H-quinolin-4-one; 1-[4-(2,3-dihydrobenzofuran-7-yl)-2-hydroxy-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-1H-[1,5]naphthyridin-4-one; 1-[4-(5-fluoro-2-methoxyphenyl)-2-hydroxy-2,4-dimethylpentyl]-3,5-dimethyl-1H-pyridin-4-one; 1-[2-hydroxy-4-(2-methoxy-5-thiophen-2-ylphenyl)-4-methyl-2-trifluoromethylpentyl]-1H-quinolin-4-one; 1-[4-(6-bromobenzo[1,3]dioxol-4-yl)-2-hydroxy-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-3-methyl-1H-quinolin-4-one; 1-[2-hydroxy-4-(4-hydroxybiphenyl-3-yl)-4-methyl-2-trifluoromethylpentyl]-1H-quinolin-4-one; 1-{4-(5-(3,5-dimethylisoxazol-4-yl)-2-hydroxyphenyl]-2-hydroxy-4-methyl-2-trifluoromethylpentyl}-1H-quinolin-4-one; 1-[2-hydroxy-4-(2-hydroxy-5-thiophen-3-ylphenyl)-4-methyl-2-trifluoromethylpentyl]-1H-quinolin-4-one; 1-{4-[5-(3,5-dimethylisoxazol-4-yl)-2-methoxyphenyl]-2-hydroxy-4-methyl-2-trifluoromethylpentyl}-1H-quinolin-4-one; 1-[2-hydroxy-4-methyl-4-(3-pyridin-3-ylphenyl)-2-trifluoromethylpentyl]-1H-quinolin-4-one; 4-methoxy-3-[4,4,4-trifluoro-3-hydroxy-1,1-dimethyl-3-(4-oxo-4H-quinolin-1-ylmethyl)butyl]benzaldehyde; 1-[2-hydroxy-4-(2-methoxy-5-thiophen-3-ylphenyl)-4-methyl-2-trifluoromethylpentyl]-1H-quinolin-4-one; 1-[4-(5-furan-3-yl-2-methoxyphenyl)-2-hydroxy-4-methyl-2-trifluoromethylpentyl]-1H-quinolin-4-one; 1-[2-hydroxy-4-(4-methoxybiphenyl-3-yl)-4-methyl-2-trifluoromethylpentyl]-1H-quinolin-4-one; 1-[4-(5-acetyl-2-hydroxyphenyl)-2-hydroxy-4-methyl-2-trifluoromethylpentyl]-1H-quinolin-4-one; 1-[3,3,3-trifluoro-2-(6-fluoro-4-methylchroman-4-ylmethyl)-2-hydroxypropyl]-1H-quinolin-4-one; 1-(4-{3-[1-(benzyloxyimino)ethyl]phenyl}-2-hydroxy-4-methyl-2-trifluoromethylpentyl)-1H-quinolin-4-one; 1-[4-(5-acetyl-2-methoxyphenyl)-2-hydroxy-4-methyl-2-trifluoromethylpentyl]-1H-quinolin-4-one; 1-(2-hydroxy-4-{3-[1-(methoxyimino)ethyl]phenyl}-4-methyl-2-trifluoromethylpentyl)-1H-quinolin-4-one; 1-[4-(5-bromo-2-hydroxyphenyl)-2-hydroxy-4-methyl-2-trifluoromethylpentyl]-1H-quinolin-4-one; 1-(2-hydroxy-4-{3-[1-(hydroxyimino)ethyl]phenyl}-4-methyl-2-trifluoromethylpentyl)- 1H-quinoiin-4-one; 1-[4-(5-bromo-2-methoxyphenyl)-2-hydroxy-4-methyl-2-trifluoromethylpentyl]-1H-quinolin-4-one; 1-[4-(3,5-difluorophenyl)-2-hydroxy-4-methyl-2-trifluoromethylpentyl]-1H-quinolin-4-one; 1-[4-(3,5-dimethylphenyl)-2-hydroxy-4-methyl-2-trifluoromethylpentyl]-1H-quinolin-4-one; 1-{2-hydroxy-4-methyl-4-[3-(2-methyl-[1,3]dioxolan-2-yl)phenyl]-2-trifluoromethylpentyl}-1H-quinolin-4-one; 1-[4-(2,3-dihydrobenzofuran-7-yl)-2-hydroxy-4-methyl-2-trifluoromethylpentyl]-1H-[1,5]naphthyridin-4-one; 1-[4-(3-[1,3]dioxan-2-ylphenyl)-2-hydroxy-4-methyl-2-trifluoromethylpentyl]-1H-quinolin-4-one; 1-{4-[3-(3,5-dimethylisoxazol-4-yl)phenyl]-2-hydroxy-4-methyl-2-trifluoromethylpentyl-1H-quinolin-4-one; 1-[4-(2,3-dihydrobenzofuran-7-yl)-2-hydroxy-4-methyl-2-trifluoromethylpentyl]-3,5-dimethyl-1H-pyridin-4-one ; 1-[4-(5-fluoro-2-methoxyphenyl)-2-hydroxy-4-methyl-2-trifluoromethylpentyl]-2-hydroxymethyl-3,5-dimethyl-1H-pyridin-4-one; 1-[4-(5-fluoro-2-hydroxyphenyl)-2-hydroxy-4-methyl-2-trifluoromethylpentyl]-3-hydroxymethyl-1H-quinolin-4-one; 1-[4-(3-bromophenyl)-2-hydroxy-4-methyl-2-trifluoromethylpentyl]-1H-quinolin-4-one; 1-[4-(5-fluoro-2-methoxyphenyl)-2-hydroxy-4-methyl-2-trifluoromethylpentyl]-6-methyl-1H-quinolin-4-one; 6-chloro-1-[4-(5-fluoro-2-hydroxyphenyl)-2-hydroxy-4-methyl-2-trifluoromethylpentyl]-1H-quinolin-4-one; 1-[-4-(2-difluoromethoxy-5-fluorophenyl)-2-hydroxy-4-methyl-2-trifluoromethylpentyl]-1H-quinolin-4-one; 1-(4-biphenyl-3-yl-2-hydroxy-4-methy-2-trifluoromethylpentyl)-1H-quinolin-4-one; 1-[2-hydroxy-4-(2-hydroxy-5-methylphenyl)-4-methyl-2-trifluoromethylpentyl]-1H-quinolin-4-one; 1-[2-hydroxy-4-(3-isopropoxyphenyl)-4-methyl-2-trifluoromethylpentyl]-1H-quinolin-4-one; 1-[4-(3-ethoxyphenyl)-2-hydroxy-4-methyl-2-trifluoromethylpentyl]-1H-quinolin-4-one; 1-[2-hydroxy-4-(2-methoxy-5-methylphenyl)-4-methyl-2-trifluoromethylpentyl]-1H-quinolin-4-one; 1-[4-(2,5-dimethylphenyl)-2-hydroxy-4-methyl-2-trifluoromethylpentyl]-1H-quinolin-4-one; 1-[2-hydroxy-4-(3-methoxyphenyl)-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-1,2-dihydroindazol-3-one; 7-fluoro-1-[4-(5-fluoro-2-hydroxyphenyl)-2-hydroxy-4-methyl-2-trifluoromethylpentyl]-1H-quinolin-4-one; 1-[4-(5-fluoro-2-hydroxyphenyl)-2-hydroxy-4-methyl-2-trifluoromethylpentyl]-3,5-dimethyl-1H-pyridin-4-one; 7-fluoro-1-[4-(5-fluoro-2-methoxyphenyl)-2-hydroxy-4-methyl-2-tritluoromethylpentyl]-1H-quinolin-4-one; 1-(2-hydroxy-4-methyl-4-phenyl-2-trifluoromethylhexyl)-1H-quinolin-4-one; 1-[4-(4-fluoro-2-methylphenyl)-2-hydroxy-4-methyl-2-trifluoromethylpentyl]-H-quinolin-4-one; 1-[4-(3,4-dimethylphenyl)-2-hydroxy-4-methyl-2-trifluoromethylpentyl]- 1H-quinolin-4-one; 8-fluoro-1-[4-(5-fluoro-2-hydroxyphenyl)-2-hydroxy-4-methyl-2-trifluoromethylpentyl]- 1H-quinolin-4-one; 6-fluoro-1-[4-(5-fluoro-2-hydroxyphenyl)-2-hydroxy-4-methyl-2-trifluoromethylpentyl]-1H-quinolin-4-one; 7-chloro-1-[4-(5-fluoro-2-hydroxyphenyl)-2-hydroxy-4-methyl-2-trifluoromethylpentyl]-IH-quinolin-4-one; 1-[4-(5-fluoro-2-isopropoxyphenyl)-2-hydroxy-4-methyl-2-trifluoromethylpentyl]-H-quinolin-4-one; 1-[4-(2-ethoxy-5-fluorophenyl)-2-hydroxy-4-methyl-2-trifluoromethylpentyl]-1H-quinolin-4-one; 8-fluoro-1-[4-(5-fluoro-2-methoxyphenyl)-2-hydroxy-4-methyl-2-trifluoromethylpentyl]-1H-quinolin-4-one; 6-fluoro- -[4-(5-fluoro-2-methoxyphenyl)-2-hydroxy-4-methyl-2-trifluoromethylpentyl]-1H-quinolin-4-one; 1-[2-hydroxy-4-(5-methanesulfonyl-2,3-dihydrobenzofuran-7-yl)-4-methyl-2-trifluoromethylpentyl]-1H-quinolin-4-one; 1-[2-hydroxy-4-methyl-4-(5-methylsulfanyl-2,3-dihydrobenzofuran-7-yl)-2-trifluoromethylpentyl]-1H-quinolin-4-one; 7-chloro-1-[4-(5-fluoro-2-methoxyphenyl)-2-hydroxy-4-methyl-2-trifluoromethylpentyl]-1H-quinolin-4-one; 3-chloro-1-[4-(5-fluoro-2-methoxyphenyl)-2-hydroxy-4-methyl-2-trifluoromethylpentyl]-5-trifluoromethyl-1H-pyridin-2-one; 1-[2-hydroxy-4-(5-methanesulfonyl-2,3-dihydrobenzofuran-7-yl)-4-methyl-2-trifluoromethylpentyl]-3-methyl-1H-quinolin-4-one; 1-[2-hydroxy-4-(2-methoxy-5-pyridin-3-ylphenyl)-4-methyl-2-trifluoromethylpentyl]-1H-quinolin-4-one; 1-[2-hydroxy-4-(2-hydroxy-3,5-dimethylphenyl)-4-methyl-2-trifluoromethylpentyl]-H-quinolin-4-one; 1-[4-(3-[1,3]dioxan-2-yl-4-fluorophenyl)-2-hydroxy-4-methyl-2-trifluoromethylpentyl]-1H-quinolin-4-one; 2-(1,1-dioxo-2,3-dihydro- 1H- 1$\lambda^6$-benzo[1,4]thiazin-4-ylmethyl)-1,1,1-trifluoro-4-(5-fluoro-2-methoxyphenyl)-4-methylpentan-2-ol; 2-(2,3-dihydrobenzo[1,4]oxazin4-ylmethyl)-1,1,1-trifluoro-4-(5-fluoro-2-methoxyphenyl)-4-methylpentan-2-ol; 1-[4-(5-fluoro-2-hydroxyphenyl)-2-hydroxy-4-methyl-2-trifluoromethylpentyl]-H-quinolin-4-one; 1-[4-(5-fluoro-2-hydroxyphenyl)-2-hydroxy-4-methyl-2-trifluoromethylpentyl]-H-[1,5]naphthyridin-4-one; 1-[4-(5-fluoro-2-methylphenyl)-2-hydroxy-4-methyl-2-trifluoromethylpentyl]-H-quinolin-4-one; 1-[4-(2,4-dimethylphenyl)-2-hydroxy-4-methyl-2-trifluoromethylpentyl]-1H-quinolin-4-one; 1-[4-(4-fluoro-2-methoxyphenyl)-2-hydroxy-4-methyl-2-trifluoromethylpentyl]-H-quinolin-4-one; 1-[4-(3-fluoro-4-methoxyphenyl)-2-hydroxy-4-methyl-2-trifluoromethylpentyl]-1H-quinolin-4-one; 1-(4-benzo[1,3]dioxol-4-yl-2-hydroxy-4-methyl-2-trifluoromethylpentyl)-1H-quinolin-4-one; 1-[4-(5-fluoro-2-methoxyphenyl)-2-hydroxy-4-methyl-2-trifluoromethylpentyl]-1,2-dihydroindazol-3-one; 1,1,1-trifluoro-4-(5-fluoro-2-methoxyphenyl)-4-methyl-2-(1-oxo-2,3-dihydro-1H-1$\lambda^4$-benzo[1,4-]thiazin-4-ylmethyl)pentan-2-ol; 1-[4-(5-fluoro-2-methoxyphenyl)- 2- hydroxy- 4- methyl- 2- trifluoromethylpentyl]- 2- hydroxymethyl- 3,5- dimethyl- 1H- pyridin-- 4- one; 1-[4-(2,3-dihydrobenzofuran-7-yl)-2-hydroxy-4-methyl-2-trifluoromethylpentyl]-3-methyl-1H-quinolin-4-one; 1-[2-hydroxy-4-(2-methoxy-3,5-dimethylphenyl)-4-methyl-2-trifluoromethylpentyl]-1H-quinolin-4-one; 1-[2-hydroxy-4-(2-hydroxy-5-pyridin-3-ylphenyl)-4-methyl-2-trifluoromethylpentyl]-1H-quinolin-4-one; and 1-[2-hydroxy-4-(2-hydroxy-5-pyridin-5-ylphenyl)-4-methyl-2-trifluoromethylpentyl]-1H-quinolin-4-one.

In still another embodiment, said at least a DIGRA has Formula I, wherein A, R', $R^2$, B, D, E, and Q have the meanings disclosed immediately above, and $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_l$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl.

In still another embodiment, said at least a DIGRA has Formula I, wherein

(a) A is an aryl, heteroaryl, or $C_5$-$C_{15}$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is the carbonyl group;

(e) D is the -NH- group;

(f) E is the hydroxy group; and

(g) Q comprises an optionally substituted phenyl group having the formula

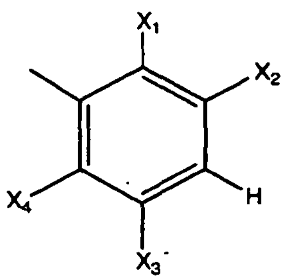

wherein $X_1$, $X_2$, $X_3$ and $X_4$ are each independently selected from the group consisting of hydrogen, halogen, hydroxy, trifluoromethyl, trifluoromethoxy, $C_1$-$C_5$ alkyl, $C_2$-$C_5$ alkenyl, $C_2$-$C_5$ alkynyl, $C_1$-$C_5$ alkoxy, $C_1$-$C_5$ alkylthio wherein the sulfur atom is optionally oxidized to a sulfoxide or sulfone, $C_1$-$C_5$ alkanoyl, $C_1$-$C_5$ alkoxycarbonyl, $C_1$-$C_5$ acyloxy, $C_1$-$C_5$ alkanoylamino, $C_1$-$C_5$ carbamoyloxy, urea, aryl, and amino wherein the nitrogen atom may be independently mono- or di-substituted by $C_1$-$C_5$ alkyl, and wherein said aryl group is optionally substituted by one or more hydroxy

or $C_1$-$C_5$ alkoxy groups, and wherein either nitrogen atom of the urea group may be independently substituted by $C_1$-$C_5$ alkyl; or Q is an aromatic 5- to 7-membered monocyclic ring having from one to four heteroatoms in the ring independently selected from nitrogen, oxygen, and sulfur, optionally independently substituted with one to three substituent groups selected from the group consisting of hydrogen, halogen, hydroxy, trifluoromethyl, trifluoromethoxy, $C_1$-$C_5$ alkyl, $C_2$-$C_5$ alkenyl, $C_2$-$C_5$ alkynyl, $C_1$-$C_5$ alkoxy, $C_1$-$C_5$ alkylthio wherein the sulfur atom is optionally oxidized to a sulfoxide or sulfone, $C_1$-$C_5$ alkanoyl, $C_1$-$C_5$ alkoxycarbonyl, $C_1$-$C_5$ acyloxy, $C_1$-$C_5$ alkanoylamino, $C_1$-$C_5$ carbamoyloxy, urea, aryl optionally substituted by one or more hydroxy or $C_1$-$C_5$ alkoxy groups, and amino wherein the nitrogen atom may be independently mono- or di-substituted by $C_1$-$C_5$ alkyl, and wherein either nitrogen atom of the urea group may be independently substituted by $C_1$-$C_5$ alkyl.

Examples of these compounds include 4-(5-fluoro-2-hydroxyphenyl)-2-hydroxy-4-methyl-2-trifluoromethyl-pentanoic acid (3,5-dichloro-phenyl)-amide; 4-(5-fluoro-2-hydroxy-phenyl)-2-hydroxy-4-methyl-2-trifluoromethyl-pentanoic acid (3-chloro-phenyl)-amide; 4-(5-fluoro-2-hydroxy-phenyl)-2-hydroxy-4-methyl-2-trifluoromethyl-pentanoic acid (2-chloro-phenyl)-amide; 4-(5-fluoro-2-hydroxy-phenyl)-2-hydroxy-4-methyl-2-trifluoromethyl-pentanoic acid (2,6-dichloro-pyrimidin-4-yl)-amide; 4-(5-fluoro-2-hydroxy-phenyl)-2-hydroxy-4-methyl-2-trifluoromethyl-pentanoic acid (2,6-dichloro-pyridin-4-yl)-amide; 4-(5-fluoro-2-hydroxy-phenyl)-2-hydroxy-4-methyl-2-trifluoromethyl-pentanoic acid (2,3-dichloro-phenyl)-amide; 4-(5-fluoro-2-hydroxy-phenyl)-2-hydroxy-4-methyl-2-trifluoromethyl-pentanoic acid (3,5-dimethylphenyl)-amide; 4-(5-fluoro-2-hydroxy-phenyl)-2-hydroxy-4-methyl-2-trifluoromethyl-pentanoic acid (3,5-bis-trifluoromethyl-phenyl)-amide; 4-(5-fluoro-2-hydroxy-phenyl)-2-hydroxy-4-methyl-2-trifluoromethyl-pentanoic acid (2,5-dichloro-phenyl)-amide; 4-(5-fluoro-2-hydroxy-phenyl)-2-hydroxy-4-methyl-2-trifluoromethyl-pentanoic acid (3-bromo-phenyl)-amide; 4-(5-fluoro-2-hydroxy-phenyl)-2-hydroxy-4-methyl-2-trifluoromethyl-pentanoic acid (3,5-difluoro-phenyl)-amide; 4-(5-fluoro-2-hydroxyphenyl)-2-hydroxy-4-methyl-2-trifluoromethyl-pentanoic acid (3,5-dibromo-phenyl)-amide.

In still another embodiment, said at least a DIGRA has Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein $R^3$ cannot be trifluoromethyl;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises an azaindolyl group optionally independently substituted with one to three substituent groups,

wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from $C_1$-$C_3$ alkyl, $C_1$-$C_3$ alkoxy, halogen, hydroxy, oxo, cyano, amino, or trifluoromethyl.

Examples of these compounds include 1,1,1-trifluoro-4-(5-fluoro-2-methoxyphenyl)-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-(1H-pyrrolo[2,3-b]pyridin-2-ylmethyl)pentan-2-ol; 1,1,1-trifluoro-4-(5-fluoro-2-methoxyphenyl)-4-methyl-2-(1H-pyrrolo[3,2-c]pyridin-2-ylmethyl)pentan-2-ol; 1,1;1-trifluoro-4-(5-fluoro-2-methoxyphenyl)-4-methyl-2-(1H-pyrrolo[3,2-b]pyridin-2-ylmethyl)pentan-2-ol; 4-fluoro-2-[4,4,4-trifluoro-3-hydroxy-1,1-dimethyl-3-(1H-pyrrolo[2,3-c]pyridin-2-ylmethyl)butyl]phenol; 4-fluoro-2-[4,4,4-trifluoro-3-hydroxy-1,1-dimethyl-3-(1H-pyrrolo[2,3-b]pyridin-2-ylmethyl)butyl]phenol; 4-fluoro-2-[4,4,4-trifluoro-3-hydroxy-1,1-dimethyl-3-(1H-pyrrolo[3,2-c]pyridin-2-ylmethyl)butyl]phenol; 4-fluoro-2-[4,4,4-trifluoro-3-hydroxy-1,1-dimethyl-3-(1H-pyrrolo[3,2-b]pyridin-2-ylmethyl)butyl]phenol; 1,1,1-trifluoro-4-(3-fluorophenyl)-4-methyl-2-(1H-pyrrolo[2,3-c]pyridin-2-ylmethyl)pentan-2-ol; 1,1,1-trifluoro-4-(4-fluorophenyl)-4-methyl-2-(1H-pyrrolo[2,3-c]pyridin-2-ylmethyl)pentan-2-ol; 4-(2,3-dihydrobenzofuran-7-yl)-1,1,1-trifluoro-4-methyl-2-(1H-pyrrolo[2,3-c]pyridin-2-yelmethyl)pentan-2-ol; 4-(2,3-dihydrobenzofuran-7-yl)-1,1,1-trifluoro-4-methyl-2-(1H-pyrrolo[3,2-c]pyridin-2-yelmethyl)pentan-2-ol; 1,1,1-trifluoro-4-methyl-4-phenyl-2-(1H-pyrrolo[2,3-c]pyridine-2-ylmethyl)pentan-2-ol; 1,1,1-trifluoro-4-(4-fluoro-2-methoxyphenyl)-4-methyl-2-(1H-pyrrolo[2,3-c]pyridin-2-ylmethyl)pentan-2-ol; 1,1,1-trifluoro-4-(4-fluoro-2-methoxyphenyl)-4-methyl-2-(1H-pyrrolo[3,2-c]pyridin-2-ylmethyl)pentan-2-ol; 1,1,1-trifluoro-4-methyl-4-phenyl-2-(1H-pyrrolo[3,2-c]pyridin-2-ylmethyl)pentan-2.ol; 1,1,1-trifluoro-4-(4-fluorophenyl)-4-methyl-2-(1H-pyrrolo[3,2-c]pyridin-2-ylmethyl)pentan-2-ol; 5-fluoro-2-[4,4,4-trifluoro-3-hydroxy-1,1-dimethyl-3-(1H-pyrrolo[2,3-c]pyridin-2-ylmethyl)butyl]phenol; 1,1,1-trifluoro-4-(5-fluoro-2-methylphenyl)-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-(3-methyl-1H-pyrrolo[2,3-c]pyridin-2-ylmethyl)pentan-2-ol; 4-fluoro-2-[4,4,4-trifluoro-3-hydroxy-1,1-dimethyl-3-(3-methyl-1H-pyrrolo[2,3-c]pyridin-2-ylmethyl)butyl] phenol; 5-fluoro-2-[4,4,4-trifluoro-3-hydroxy-1,1-dimethyl-3-(1H-pyrrolo[3,2-c]pyridin-2-ylmethyl)butyl]pentan-2-ol; 1,1,1-trifluoro-4-(5-fluoro-2,3-dihydrobenzofuran-7-yl)-4-methyl-2-(1H-pyrrolo[2,3-c]pyridine-2-ylmethy()pentan-2-ol; 4-fluoro-2-[4,4,4-trifluoro-3-hydroxy-1,1-dimethyl-3-(1H-pyrrolo[2,3-c]-[3-methylpyridin]-2-ylmethyl)butyl]phenol; 4-fluoro-2-[4,4,4-trifluoro-3-hydroxy-1,1-dimethyl-3-(1H-pyrrolo[2,3-c]-[2-fluoropyridin]-2-ylmethyl)butyl]phenol; and 4-fluoro-2-[4,4,4-trifluoro-3-hydroxy-1,1-dimethyl-3-(1H-pyrrolo[2,3-c]-[2-trifluoromethylpyridin]-2-ylmethyl)butyl]phenol.

In still another embodiment, said at least a DIGRA has Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises a heteroaryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, or trifluoromethyl.

Examples of these compounds include 4-cyclohexyl-1,1,1-trifluoro-4-methyl-2-quinolin-4-ylmethylpentan-2-ol; 4-pyrimidin-5-yl-2-[4,4,4-trifluoro-3-hydroxy-1,1-dimethyl-3-(1H-pyrrolo[2,3-c]pyridin-2-ylmethyl)butyl]phenol; 4-pyrimidin-5-yl-2-[4,4,4-.trifluoro-3-hydroxy- 1,1-dimethyl-3-(1H-pyrrolo[3,2-c]pyridin-2-ylmethyl)butyl]phenol; 1,1,1-trifluoro-4-(5-fluoro-2-methoxyphenyl)-4-methyl-2-(3-methyl-1H-pyrrolo[3,2-c]pyridin-2-ylmethyl)pentan-2-ol; 1,1,1-trifluoro-4-(5-fluoro-2,3-dihydrobenzofuran-7-yl)-4-methyl-2-(1H-pyrrolo[3,2-c]pyridin-2-ylmethyl)pentan-2-ol; 1,1,1-trifluoro-4-(5-fluoro-2-methylphenyl)-4-methyl-2-(3-methyl-1H-pyrrolo[2,3-c]pyridin-2-ylmethyl)pentan-2-ol; 2-(4,6-dimethyl-1H-pyrrolo[3,2-c]pyridin-2-ylmethyl)-1,1,1-trifluoro-4-(5-fluoro-2-methoxyphenyl)-4-methylpentan-2-ol 2-(5,7-dimethyl-1H-pyrrolo[2,3-c]pyridin-2-ylmethyl)-1,1,1-trifluoro-4-(5-fluoro-2-methoxyphenyl)-4-methylpentan-2-ol; 2-[4-(5-fluoro-2-methoxyphenyl)-2-hydroxy-4-methyl-2-trifluoromethylpentyl]-1H-pyrrolo[3,2-b]pyridine-5-carbonitrile; 1,1,1-trifluoro-4-(5-fluoro-2-methoxyphenyl)-4-methyl-2-(6-methyl- 1H-pyrrolo[3,2-c]pyridin-2-ylmethyl)pentan-2-ol; 1,1,1-trifluoro-4-(5-fluoro-2-methoxyphenyl)-4-methyl-2-(4-methyl-1H-pyrrolo[3,2-c]pyridiln-2-ylmethyl)pentan-2-ol; 2-[4-(5-fluoro-2-methoxyphenyl)-2-hydroxy-4-methyl-2-trifluoromethylpentyl]-4-methyl-1H-pyrrolo[3,2-c)pyridine-6-carbonitrile; 2-[4-(5-fluoro-2-methoxyphenyl)-2-hydroxy-4-methyl-2-trifluoromethylpentyl]- 1H-pyrrolo[2,3-c]pyridine-5-carbonitrile; 2-[4-(5-fluoro-2-methoxyphenyl)-2-hydroxy-4-methyl-2-trifluoromethylpentyl]-1H-pyrrolo[3,2-c]pyridine-4-carbonitrile; 1,1,1-trifluoro-4-(5-fluoro-2-methoxyphenyl)-4-methyl-2-(5H-pyrrolo[3,2-d]pyrimidin-6-ylmethyl)pentan-2-ol; 1,1,1-trifluoro-4-(5-fluoro-2-methoxyphenyl)-4-methyl-2-thieno[2,3-d]pyridazin-2-ylmethylpentan-2-ol; 1,1,1-trifluoro-4-(5-fluoro-2-methoxyphenyl)-4-methyl-2-(5H-pyrrolo[3,2-c]pyridazin-6-ylmethyl)pentan-2-ol; 1,1,1-trifluoro-4-(5-fluoro-2-methoxyphenyl)-4-methyl-2-(2-methyl-5H-pyrrolo[3,2-d]pyrimidin-6-ylmethyl)pentan-2-ol; 1,1,1-trifluoro-4-(5-fluoro-2-methylphenyl)-4-methyl-2-(1H-pyrrolo[2,3-d]pyridazin-2-ylmethyl)pentan-2-ol; 2-(4,6-dimethyl-H-pyrrolo[3,2-c]pyridin-2-ylmethyl)-1,1,1-trifluoro-4-(5-fluoro-2-methylphenyl)-4-methylpentan-2-ol; 4-(5-chloro-2,3-dihydrobenzofuran-7-yl)-2-(4,6-dimethyl- 1H-pyrrolo[3,2-c]pyridin-2-ylmethyl)-1,1,1-trifluoro-4-methylpentan-2-ol; 2-[4-(5-fluoro-2-methylphenyl)-2-hydroxy-4-methyl-2-trifluoromethylpentyl]-1H-pyrrolo[3,2-b]pyridine-5-carbonitrile; 4-(5-chloro-2,3-dihydrobenzofuran-7-yl)-1,1,1-trifluoro-4-methyl-2-(3-methyl-1H-pyrrolo[2,3-c]pyridin-2-ylmethyl)pentan-2-ol; 1,1,1-trifluoro-4-(5-fluoro-2-methylphenyl)-4-methyl-2-(5H-pyrrolo[3,2-c]-pyridazin-6-ylmethyl)pentan-2-ol; 4-(5-chloro-2,3-dihydrobenzofuran-7-yl)-1,1,1-trifluoro-4-methyl-2-(5H-pyrrolo[3,2-c]pyridazin-6-ylmethyl)pentan-2-ol; 4-(5-chloro-2,3-dihydrobenzofuran-7-yl)-1,1,1-trifluoro-4-methyl-2-(1-H-pyrrolo[2,3-d]pyridazin-2-ylmethyl)pentan-2-ol; 1,1,1-trifluoro-4-(5-fluoro-2-methoxyphenyl)-2-(7-fluoro-1H-pyrrolo[2,3-c]pyridin-2ylmethyl)-4-methylpentan-2-ol; 1,1,1-trifluoro-4-(5-fluoro-2-methoxyphenyl)-4-methyl-2-(4-methyl-1H-pyrrolo[2,3-c]pyridin-2-ylmethyl)pentan-2-ol; 2-(5,7-dichloro-1H-pyrrolo[2,3-c]pyridin-2-ylmethyl)-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-(5-trifluoromethyl-1H-pyrrolo[2,3-c]pyridin-2-ylmethyl)pentan-2-ol; 1,1,1-trifluoro-4-(5-fluoro-2-methoxyphenyl)-2-(5-methoxy-1H-pyrrolo[2,3-c]pyridin- 2-ylmethyl)-4-methylpentan-2-ol; 1,1,1-trifluoro-4-(5-fluoro-2-methylphenyl)-4-methyl-2-(4-methyl-1H-pyrrolo[2,3-c]pyridin-2-ylmethyl)pentan-2-ol; 1,1,1-trifluoro-4-(5-fluoro-2-methylphenyl)-2-(5-isopropoxy-1H-pyrrolo[2,3-c]pyridin-2-ylmethyl)-4-methylpentan-2-ol ; 1,1,1-trifluoro-4-(5-fluoro-2-methylphenyl)-2-(5-methoxy-1H-pyrrolo[2,3-c]pyridin-2-ylmethyl)-4-methylpentan-2-ol; 4-(5-chloro-2,3-dihydrobenzofuran-7-yl)-1,1,1-trifluoro-2-(5-methoxy-1H-pyrrolo[2,3-c]pyridin-2-ylmethyl)-4-methylpentan-2-ol; 1, 1, 1-difluoro-4-(5-fluoro-2-methylphenyl)-2-(7-fluoro-1H-pyrrolo[2,3-c] pyridin-2-ylmethyl)-4-methylpentan-2-ol; 4-(5-chloro-2,3-dihydrobenzofuran-7-yl)-1-trifluoro-4-methyl-2-(5-trifluoromethyl-1H-pyrrolo[2,3-c]pyridin-2-ylmethyl)pentan-2-ol; 1,1,1-trifluoro-4-(5-fluoro-2-methylphenyl)-4-methyl-2-(5-trifluoromethyl- 1H-pyrrolo[2,3-c]pyridin-2-ylmethyl)pentan-2-ol; 4-(5-chloro-2,3-dihydrobenzofuran-7-yl)-1,1,1-trifluoro-2-(5-isopropoxy-1H-pyrrolo[2,3-c]pyridin-2-ylmethyl)-4-methylpentan-2-ol; 4-(5-chloro-2,3-dihydrobenzofuran-7-yl)-1,1,1-trifluoro-2-(7-fluoro-1H-pyrrolo[2,3-c]pyridin-2-ylmethyl)-4-methylpentan-2-ol; 4-(5-chloro-2,3-dihydrobenzofuran-7-yl)-2-(5-dimethylamino-1H-pyrrolo[2,3-c]pyridin-2-ylmethyl)-1,1,1-trifluoro-4-methylpentan-2-ol; 4-(5-chloro-2,3-dihydrobenzofuran-7-yl)-1,1,1-trifluoro-4-methyl-2-(5-piperidin-1-yl-1H-pyrrolo[2,3-c]pyridin-2-ylmethyl)pentan-2-ol; 4-(5-chloro-2,3-dihydrobenzofuran-7-yl)-1,1,1-trifluoro-4-methyl-2-(5-morpholin-4-yl-1H-pyrrolo[2,3-c]pyridin-2-ylmethyl)pentan-2-ol; 1,1,1-trifluoro-4-(5-fluoro-2-methylphenyl)-4-methyl-2-(5-piperidin-1-yl-1H-pyrrolo[2,3-c]pyridin-2-ylmethyl)pentan-2-ol; 4-(5-chloro-2,3-dihydrobenzofuran-7-yl)-2-(5-ethoxy-1H-pyrrolo[2,3-c]pyridin-2-ylmethyl)-1,1,1-trifluoro-4-methylpentan-2-ol; 2-(5-benzyloxy-1H-pyrrolo[2,3-c]pyridin-2-ylmethyl)-1,1,1-trifluoro-4-(5-fluoro-2-methylphenyl)-4-methylpentan-2-ol; 2-(5-benzyloxy-1H-pyrrolo[2,3-c]pyridin-2-ylmethyl)-4-(5-chloro-2,3-dihydrobenzofiran-7-yl)-1,1,1-trifluoro-4-methylpentan-2-ol; 1,1,1-trifluoro-4-(5-fluoro-2-methoxyphenyl)-2-(5-chloro-1H-pyrrolo[2,3-c-]pyridin-2-ylmethyl)-4-methylpentan-2-ol ; 1,1,1-trifluoro-4-(5-fluoro-2-methoxyphenyl)-4-methyl-2-[5-(methylamino)- 1H-pyrrolo[2,3-c]pyridin-2-ylmethyl]pentan-2-ol; 1,1,1-trifluoro-4-(5-fluoro-2-methoxyphenyl)-4-methyl-2-(5-amino-1H-pyrrolo[2,3-c]pyridin-2-ylmethyl)pentan-2-ol; 1,1,1-trifluoro-4-(5-fluoro-2-methylphenyl)-4-methyl-2-(6-amino-1H-pyrrol- o[2,3-c]pyridin-2-ylmethyl)pentan-2-ol; 4-(5-chloro-2,3-dihydrobenzofuran-7-yl)-1,1,1-trifluoro-2-(5-amino-1H-pyrrolo[2,3-c]pyridin-2-ylmethyl)-4-methylpentan-2-ol; 4-(5-chloro-2,3-dihydrobenzofuran-7-yl)-1,1,1-trifluoro-4-methyl-2-(5-methylamino-1H-pyrrolo[2,3-c]pyridin-2-ylmethyl)pentan-2-ol; 7-[4-(5-fluoro-2-methoxyphenyl)-2-hydroxy-4-methyl-2-trifluoromethylpentyl]-1H-pyrrolo[2,3-b]pyridin-7-ium chloride; 6-[4-(5-fluoro-2-methoxyphenyl)-2-hydroxy-4-methyl-2-trifluoromethylpentyl]-2-methyl- 1H-pyrrolo[2,3-c]pyridin-6-ium chloride; 4-(5-bromo-2,3-dihydrobenzofuran-7-yl)-1,1,1-trifluoro-4-methyl-2-(1H-pyrrolo[2,3-c]pyridin-2-ylmethyl)pentan-2-ol; 1,1,1-trifluoro-4-methyl-4-(5-methyl-2,3-dihydrobenzofuran-7-yl)-2-(1H-pyrrolo[2,3-c]pyridin-2-ylmethyl)pentan-2-ol; 4-(5-chloro-2,3-dihydrobenzofuran-7-yl)-1,1,1-trifluoro-4-methyl-2-(1H-pyrrolo[2,3-c]pyridin-2-ylmethyl)pentan-2-ol; 1,1,1-trifluoro-4-(5-fluoro-2-methoxyphenyl)-4-methyl-2-pyrrolo[2,3-b] pyridin-1-ylmethylpentan-2-ol ; 1,1,1-trifluoro-4-(5-fluoro-2-methoxyphenyl)-4-methyl-2-(6-oxy-1H-pyrrolo[2,3-c]pyridin-2-ylmethyl)pentan-2-ol; 1,1,1-trifluoro-4-(5-fluoro-2-methoxyphenyl)-4-methyl-2-pyrrolo[2,3-c]pyridin-1-ylmethylpentan-2-ol; 2-benzo[b]thiophen-2-ylmethyl-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-thieno[2,3-c]pyridin-2-ylmethylpentan-2-ol; 1,1,1-trifluoro-4-(5-fluoro-2-methoxyphenyl)-2-indazol-1-ylmethyl-4-methylpentan-2-ol; 1,1,1-trifluoro-4-(5-fluoro-2-methoxyphenyl)-4-methyl-2-pyrazolo[1,5-a]pyridin-2-ylmethylpentan-2-ol; 4-(5-chloro-2,3-dihydrobenzofuran-7-yl)-2,4-dimethyl-1-thieno[2,3-c]pyridin-2-ylpentan-2-ol; 4-(5-fluoro-2-methylphenyl)-2,4-dimethyl-1-thieno[2,3-c]pyridin-2-ylpentan-2-ol; 1,1,1-trifluoro-4-(5-fluoro-2-methoxyphenyl)-2-furo[2,3-c]pyridin-2-ylmethy- 1-4-methylpentan-2-ol; 4-(5-chloro-2,3-dihydrobenzofuran-7-yl)-1-furo[2,3-c]pyridin-2-yl-2,4-dimethylpentan-2-ol; 4-(5-fluoro-2-methylphenyl)-1-furo-[2,3-c] pyridin-2-yl-2,4-dimethylpentan-2-ol; 1,1,1-trifluoro-4-(5-fluoro-2-methylphenyl)-4-methyl-2-(1H-pyrrolo[3,2-c]pyridin-2-ylmethyl)pentan-2-ol-; 1,1,1-trifluoro-4-methyl-4-(5-methyl-2,3-dihydrobenzofuran-7-yl)-2-(1H-pyrrolo[3,2-c]pyridin-2-ylmethyl)pentan-2-ol; 4-(5-chloro-2,3-dihydrobenzofuran-7-yl)-1,1,1-trifluoro-4-methyl-2-(1H-pyrrolo[3,2-c]pyridin-2-ylmethyl)pentan-2-ol; 4-(5-bromo-2,3-dihydrobenzofuran-7-yl)-1,1,1-trifluoro-4-methyl-2-(1H-pyrrolo[3,2-c]pyridin-2-ylmethyl)pentan-2-ol; 2-(3-dimethylaminomethyl-1H-pyrrolo[3,2-c]pyridin-2-ylmethyl)-1,1,1-trifluoro-4-(5-fluoro-2-methoxyphenyl)-4-methylpentan-2-ol; 1,1,1-trifluoro-4-(5-fluoro-2-methoxyphenyl)-4-methyl-2-pyrrolo[3,2-c]pyridin-l-ylmethylpentan-2-ol; 1,1,1-trifluoro-4-(5-fluoro-2-methoxyphenyl)-4-methyl-2-pyrolo[3,2-b]pyridin-1-ylmethylpentan-2-ol; 1,1,1-trifluoro-4-(5-fluoro-2-methoxyphenyl)-2-furo[3,2-c]pyridin-2-ylmethyl-4-methylpentan-2-ol; 4-(5-chloro-2,3-dihydrobenzofuran-7-yl)-1,1,1-trifluoro-4-methyl-2-pyrrolo[3,2-b]pyridin-1-ylmethylpentan-2-ol; 1,1,1-trifluoro-4-(5-fluoro-2-methoxyphenyl)-4-methyl-2-thieno[3,2-c]pyridin-2-ylmethylpentan-2-ol; 4-(5-chloro-2,3-dihydrobenzofuran-7-yl)-1,1,1-trifluoro-4-methyl-2-thieno[3,2-c] pyridin-2-ylmethylpentan-2-ol; 1,1,1-trifluoro-4-(5-fluoro-2-methylphenyl)-4-methyl-2-pyrrolo[3,2-b]pyridin-1-ylmethylpentan-2-ol; 1,1,1-trifluoro-4-(5-fluoro-2-methylphenyl)-4-methyl-2-thieno[3,2-c]pyridin-2-ylmethylpentan-2-ol; 4-fluoro-2-(4,4,4-trifluoro-3-hydroxy-1,1-dimethyl-3-thieno[3,2-c]pyridin-2-ylmethylbutyl)phenol; 4-fluoro-2-(4,4,4-trifluoro-3-furo[3,2-c]pyridin-2-ylmethyl-3-hydroxy-1,1-dimethylbutyl)phenol; 4-fluoro-2-(4,4,4-trifluoro-3-hydroxy-1,1-dimethyl-3-pyrrolo[3,2-b]pyridin-1-ylmethylbutyl)phenol; 2-[4-(5-fluoro-2-hydroxyphenyl)-2-hydroxy-4-methyl-2-trifluoromethylpentyl]- 1H-indole-6-carboxylic acid; 2-[4-(5-fluoro-2-hydroxyphenyl)-2-hydroxy-4-methyl-2-trifluoromethylpentyl]-1H-indole-6-carboxylic acid dimethylamide; {2-[4-(5-fluoro-2-hydroxyphenyl)-2-hydroxy-4-methyl-2-trifluoromethylpentyl]-1H-indol-6-yl}morpholin-4-ylmethanone; 2-[4-(5-fluoro-2-methoxyphenyl)-2-hydroxy-4-methyl-2-trifluoromethylpentyl]- I H-indole-6-carboxylic acid dimethylamide; {2-[4-(5-fluoro-2-methoxyphenyl)-2-hydroxy-4-methyl-2-trifluoromethylpentyl]- 1H-indol-6-yl}morpholin-4-ylmethanone; 2-[4-(5-fluoro-2-hydroxyphenyl)-2-hydroxy-4-methyl-2-trifluoromethylpentyl]-1H-indole-6-carboxylic acid amide; 2-[4-(5-fluoro-2-methoxyphenyl)-2-hydroxy-4-methyl-2-trifluoromethylpentyl]-1H-indole-6-carboxylic acid amide; 4-fluoro-2-[4,4,4-trifluoro-3-hydroxy-1,1-dimethyl-3-(5-nitro-1H-indol-2-ylmethyl)butyl]phenol; 2-[4-(5-fluoro-2-methoxyphenyl)-2-hydroxy-4-methyl-2-trifluoromethylpentyl]-1H-indole-6-carbonitrile; 2-[4-(5-fluoro-2-hydroxyphenyl)-2-hydroxy-4-methyl-2-trifluoromethylpentyl]-1H-indole-6-carbonitrile; N-{2-[4-(5-fluoro-2-methoxyphenyl)-2-hydroxy-4-methyl-2-trifluoromethylpentyl]-1H-indol-5-yl}acetamide; 1,1,1-trifluoro-4-(4-fluoro-2-methoxyphenyl)-2-(7-fluoro-4-methyl- 1H-indo- 1-2-ylmethyl)-4-methylpentan-2-ol; 5-fluoro-2-[4,4,4-trifluoro-3-(7-fluoro-4-methyl- 1H-indol-2-ylmethyl)-3-hydroxy-1,1-dimethylbutyl]phenol; 2-[4-(3-[1,3]dioxolan-2-ylphenyl)-2-hydroxy-4ymethyl-2-trifluoromethylpentyl]- 1H-indole-5-carbonitrile; 2-[4-(5-fluoro-2-methoxyphenyl)-2-hydroxy-4-methyl-2-trifluoromethylpentyl]-1H-indole-5-carboxylic acid-2-trimethylsilanylethyl ester; 2-[4-(5-fluoro-2-methoxyphenyl)-2-hydroxy-4-methyl-2-trifluoromethylpentyl]-1H-indole-5-carboxylic acid; 2-[4-(4-fluoro-2-hydroxyphenyl)-2-hydroxy-4-methyl-2-trifluoromethylpenty-1]-4-methyl-1H-indole-6-carbonitrile; {2-[4-(5-Fluoro-2-methoxyphenyl)-2-hydroxy-4-methyl-2-trifluoromethylpentyl]- l H-indol-5-yl}piperidin-1-ylmethanone; 2-[4-(5-fluoro-2-methoxyphenyl)-2-hydroxy-4-methyl-2-trifluoromethylpentyl]-1H-indole-5-carboxylic acid methylamide; {2-[4-(5-fluoro-2-methoxyphenyl)-2-hydroxy-4-methyl-2-tifluoromethylpentyl}-1H-indol-5-yl}pyrrolidin-1-ylmethanone; 1-{2-[4-(5-fluoro-2-methoxyphenyl)-2-hydroxy-4-methyl-2-trifluoromethylpentyl}1H-indole-5-carbonyl}piperidin-4-one; 2-[4-(5-fluoro-2-methoxyphenyl)-2-hydroxy-4-methyl-2-trifluoromethylpentyl]-1H-indole-5-carboxylic acid (2-hydroxyethyl)amide; {2-[4-(5-fluoro-2-methoxyphenyl)- 2- hydroxy- 4- methyl- 2- trifluoromethylpentyl]- 1H- indol- 5- yl} (4- hydroxypiperidin- 1- yl) methanone; {2-[4-(5-fluoro-2-methoxyphenyl)-2-hydroxy-4-methyl-2-trifluoromethylpentyl]-1H-indol-5-yl}(3-hydroxypyrrolidin-1-yl) methanone; 2-[4-(5-fluoro-2-methoxyphenyl)-2-hydroxy-4-methyl-2-trifluoromethylpentyl]-1H-indole-5-carboxylic acid cyanomethylamide; 2-[4-(5-fluoro-2-methoxyphenyl)-2-hydroxy-4-methyl-2-trifluoromethylpentyl]-1H-indole-5-carboxylic acid (2-dimethylaminoethyl)amide; {2-[4-(5-fluoro-2-methoxyphenyl)-2-hydroxy-4-methyl-2-trifluoromethylpentyl]-1H-indol-5-yl}(4-methylpiperazin-1-yl)methanone; ({2-[4-(5-fluoro-2-methoxyphenyl)-2-hydroxy-4-methyl-2-trifluoromethylpentyl]-1H-indole-5-carbonyl}amino)acetic acid methyl ester; 2-[4-(5-fluoro-2-methoxyphenyl)-2-hydroxy-4-methyl-2-trifluoromethylpentyl]-1H-indole-5-carboxylic acid carbamoylmethylamide; 4-({2-[4-(5-fluoro-2-methoxyphenyl)-2-hydroxy-4-methyl-2-trifluoromethylpentyl]-1H-indote-5-carbonyl amino)butyric acid methyl ester; ({2-[4-(5-fluoro-2-methoxyphenyl)-2-hydroxy-4-methyl-2-trifluoromethylpentyl]-1H-indole-5-carbonyl}amino)acetic acid; 4-({2-[4-(5-fluoro-2-methoxyphenyl)-2-hydroxy-4-methyl-2-trifluoromethylpentyl]-1H-indole-5-carbonyl}amino)butyric acid; 2-[4-(3-dimethylaminomethylphenyl)-2-hydroxy-4-methyl-2-trifluoromethylpentyl]-1H-indote-5-carbonitrile; 4-fluoro-2-[4,4,4-trifluoro-3-hydroxy-1,1-dimethyl-3-(5-trifluoromethyl-1H-indol-2-ylmethyl)butyl]phenol; 2-[4-(5-bromo-2,3-dihydrobenzofuran-7-yl)-2-hydroxy-4-methyl-2-trifluoromethylpentyl]-4-methyl-1H-indole-6-carbonitrile; 2-[2-hydroxy-4-(5-methanesulfonyl-2,3-dihydrobenzofuran-7-yl)-4-methyl-2-trifluoromethylpentyl]-4-methyl-1H-indole-6-carbonitrile; 2-[4-(5-bromo-2,3-dihydrobenzofuran-7-yl)-2-hydroxy-4-methyl-2-trifluoromethylpentyl]-1H-indole-5-carboxylic acid; 2-[4-(5-bromo-2,3-dihydrobenzofuran-7-yl)-2-hydroxy-4-methyl-2-trifluoromethylpentyl]-1H-indole-5-carboxylic acid amide; 2-[4-(5-bromo-2,3-dihydrobenzofuran-7-yl)-2-hydroxy-4-methyl-2-trifluoromethylpentyl]-1H-indole-5-carboxylic acid dimethylamide; 2-[4-(5-Bromo-2,3-dihydrobenzofuran-7-yl)-2-hydroxy-4-methyl-2-trifluoromethylpentyl]-1H-indole-5-carboxylic acid cyanomethylamide; {2-[4-(5-bromo-2,3-dihydrobenzofuran-7-yl)-2-hydroxy-4-methyl-2-trifluoromethylpentyl]-1H-indol-5-yl}pyrrolidin-1-ylmethanone; {2-[4-(5-bromo-2,3-dihydrobenzofuran-7-yl)-2-hydroxy-4-methyl-2-trifluoro-methylpentyl]-1H-indol-5-yl}morpholin-4-ylmethanone; 2-[4-(5-fluoro-2-methoxyphenyl)-2-hydroxy-4-methyl-2-trifluoromethylpentyl]-1H-indole-5-carboxylic acid amide; {2-[4-(5-fluoro-2-methoxyphenyl)-2-hydroxy-4-methyl-2-trifluoromethylpentyl]-1H-indol-5-yl}morpholin-4-ylmethanone; 2-(4-benzo[1,3]dioxol-4-yl-2-hydroxy-4-methyl-2-trifluoromethylpentyl)-4-methyl-1H-indole-6-carbonitrile; 1,1,1-trifluoro-4-methyl-4-phenyl-2-quinolin-4-ylmethylhexan-2-ol; 2-[2-hydroxy-4-methyl-4-(5-methylsulfanyl-2- ,3-dihydrobenzofuran-7-yl)-2-trifluoromethylpentyl]-1H-indole-3-carbonitrile; 7-(4,4,4-trifluoro-3-hydroxy-1,1-dimethyl-3-quinolin-4-ylmethylbutyl)-2,3-dihydrobenzofuran-5-carbonitrile; 2-[2-hydroxy-4-(5-methanesulfonyl-2,3-dihydrobenzofuran-7-yl)-4-methyl-2-trifluoromethylpentyl]-1H-indole-3-carbonitrile; 2-[2-hydroxy-4-(2-hydroxy-5-methylphenyl)-4-methyl-2-trifluoro-methylpentyl]-4-methyl-1H-indole-6-carbonitrile; 1,1,1-trifluoro-4-(5-fluoro-2,3-dihydrobenzofuran-7-yl)-4-methyl-2-(5-methylsulfanyl-1H-indol-2-ylmethyl)pentan-2-ol; 2-[2-hydroxy-4-(2-methoxy-5-methylsulfanylphenyl)-4-methyl-2-trifluoromethylpentyl]-1H-indole-3-carbonitrile; 2-[2-Hydroxy-4-(5-methanesulfonyl-2-methoxyphenyl)-4-methyl-2-trifluoromethylpentyl]-1H-indole-3-carbonitrile; 2-[4-(5-fluoro-2,3-dihydrobenzofuran-7-yl)-2-hydroxy-4-methyl-2-trifluoromethylpentyl]-1H-indole-5-sulfonic acid dimethylamide; 1,1,1-trifluoro-4-(5-fluoro-2,3-dihydrobenzofuran-7-y- 1)-4-methyl-2-(5-phenyl-1H-indol-2-ylmethyl)pentan-2-ol; 2-[4-(5-tert-butyl-2-hydroxyphenyl)-2-hydroxy-4-methyl-2-trifluoromethylpentyl]-1H-indole-3-carbonitrile; 2-[2-hydroxy-4-(2-hydroxy-5-isopropylphenyl)-4-methyl-2-trifluoromethylpentyl]- 1H-indole-3-carbonttnie; 2-[2-hydroxy-4-(2-hydroxy-3,5-dimethylphenyl)-4-methyl-2-trifluoromethylpentyl]- 1H-indole-3-carbonitrite; 2-[2-hydroxy-4-(5-hydroxy-2,4-dimethylphenyl)-4-methyl-2-trifluoromethylpentyl]-1H-indole-3-carbonitrile; 2-[4-(5-tert-butyl-2-methoxyphenyl)-2-hydroxy-4-methyl-2-trifluoromethylpentyl]-1H-indole-3-carbonitrile; 2-[4-(5-tert-butyl-2-methoxyphenyl)-2-hydroxy-4-methyl-2-trifluoromethylpentyl]-1-methyl-1H-indole-3-carbonitrile; 2-[2-hydroxy-4-(5-isopropyl-2-methoxyphenyl)-4-methyl-2-trifluoromethylpentyl]-1H-indole-3-carbonitrile; 2-[2-hydroxy-4-(5-isopropyl-2-methoxyphenyl)-4-methyl-2-trifluoromethylpentyl]-1-methyl-1H-indole-3-carbonitrile; 2-[2-hydroxy-4-(2-hydroxy-5-methanesulfonylphenyl)-4-methyl-2-trifluoromethylpentyl]-1H-indole-3-carbonitrile; 2-[2-hydroxy-4-(2-methoxy-5-methylphenyl)-4-methyl-2-trifluoromethylpentyl]-4-methyl-1H-indole-6-carbonitrile; 1,1,1-trifluoro-4-methyl-2-quinolin-4-ylmethyl-4-o-tolylpentan-2-ol; 1,1,1-trifluoro-4-methyl-2-quinolin-4-ylmethyl-4-m-tolylpentan-2-ol; 1,1,1-trifluoro-4-(2-fluorophenyl)-2-(1H-indol-2-ylmethyl)-4-methylpentan-2-ol; 1,1,1-trifluoro-4-(2-fluorophenyl)-4-methyl-2-quinolin-4-ylmethylpentan-2-ol; 1,1,1-trifluoro-4-(3-fluorophenyl)-2-(1H-indol-2-ylmethyl)-4-methylpentan-2-ol; 1,1,1-trifluoro-4-(3-fluorophenyl)-4-methyl-2-quinolin-4-ylmethylpentan-2-ol; 1,1,1-trifluoro-4-(4-fluorophenyl)-2-(1H-indol-2-ylmethyl)-4-methylpentan-2-ol; 1,1,1-trifluoro-4-(4-fluorophenyl)-4-methyl-2-quinolin-4-ylmethylpentan-2-ol; 3-(4,4,4-trifluoro-3-hydroxy-1,1-dimethyl-3-quinolin-4-ylmethylbutyl)phenol; 1,1,1-trifluoro-4-methyl-2-quinolin-4-ylmethyl-4-(2-trifluoromethylphenyl)pentan-2-ol; 1,1,1-trifluoro-2-(1H-indol-2-ylmethyl)-4-methyl-4-(4-trifluoromethylphenyl)pentan-2-ol; 1,1,1-trifluoro-4-methyl-2-quinolin-4-ylmethyl-4-(4-trifluoromethylphenyl)pentan-2-ol; 4-(3-chlorophenyl)-1,1,1-trifluoro-2-(1H-indol-2-ylmethyl)-4-methylpentan-2-ol; 4-(3-chlorophenyl)-1,1,1,-trifluoro-4-methyl-2-quinolin-4-ylmethylpentan-2-ol; 4-(4-dimethylaminophenyl)-1,1,1-trifluoro-2-(1H-indol-2-ylmethyl)-4-methylpentan-2-ol; 4-biphenyl-3-yl-1,1,1-trifluoro-4-methyl-2-quinolin-4-ylmethylpentan-2-ol: 4-(3-bromophenyl)-1,1,1-trifluoro-2-(1H-indol-2-ylmethyl)-4-methylpentan-2-ol; 4-(2-difluoromethoxy-5-fluorophenyl)-1,1,1-trifluoro-2-(1H-indol-2-ylmethyl)-4-methylpentan-2-ol; 4-biphenyl-3-yl-1,1,1-trifluoro-2-(1H-indol-2-ylmethyl)-4-methylpentan-2-ol; 4-(4-dimethylaminophenyl)-1,1,1-trifluoro-4-methyl-2-quinolin-4-ylmethylpentan-2-ol; 2-[4-(5-fluoro-2-methylphenyl)-2-hydroxy-4-methyl-2-trifluoromethylpentyl]-1,6-dihydropyrrolo[2,3-c]pyridin-5-one; 2-[4-(5-Fluoro-2-methylphenyl)-2-hydroxy-4-methyl-2-trifluoromethylpentyl]-6-methyl-1,6-dihydropyrrolo[2,3-c]pyridin-5-one; 2-[4-(5-fluoro-2-methyl- phenyl)-2-hydroxy-4-methyl-2-trifluoromethylpentyl]-4-methyl-1,4-dihydropyrrolo[3,2-b]pyridin-5-one; 1,1,1-trifluoro-4-(5-fluoro-2-methylphenyl)-2-(6-methoxy-1H-pyrolo[3,2-c]pyridin-2-ylmethyl)-4-methylpentan-2-ol; 2-[4-(5-fluoro-2-methylphenyl)-2-hydroxy-4-methyl-2-trifluoromethylpentyl]-5-methyl-1,5-dihydropyrrolo[3,2-c]pyridin-6-one; 2-[4-(5-fluoro-2-methyl-phenyl)-2-hydroxy-4-methyl-2-trifluoromethylpentyl]-1,3a-dihydropyrrolo[3,-2-c]pyridin-6-one; 2-[4-(5-fluoro-2-methylphenyl)-2-hydroxy-4-methyl-2-trifluoromethylpentyl]-1,7-dihydropyrrolo[3,2-c]pyridine-4,6-dione; 6-[4-(5-fluoro-2-methylphenyl)-2-hydroxy-4-methyl-2-trfluoromethylpentyl]-3-methyl- 1,7-dihydropyrrolo[2,3-d]pyrimidine-2,4-dione; 2-[4-(5-chloro-2,3-dihydrobenzofuran-7-yl)-2-hydroxy-4-methyl-2-trifluoro-methylpentyl]-1,6-dihydropyrrolo[2,3-c]pyridin-5-one; 2-[4-(5-chloro-2,3-dihydrobenzofuran-7-yl)-2-hydroxy-4-methyl-2-trifluoromethylpentyl]-6-methyl-1,6-dihydropyrrolo[2,3-c]pyridin-5-one; 2-[4-(5-chloro-2,3-dihydrobenzofuran-7-yl)-2-hydroxy-4-methyl-2-trifluoromethylpentyl]-1,4-dihydropyrrolo[3,2-b]pyridin-5-one; 2-[4-(5-chloro-2,3-dihydrobenzofiran-7-yl)-2-hydroxy-4-methyl-2-trifluoromethylpentyl]-4-methyl-1,4-dihydropyrrolo[3,2-b]pyridin-5-one; 2-[4-(5-chloro-2,3-dihydrobenzofuran-7-yl)-2-hydroxy-4-methyl-2-trifluoro-methylpentyl]-1,5-dihydropyrrolo[3,2-c]pyridin-6-one; 2-[4-(5-chloro-2,3-dihydrobenzofuran-7-yl)-2-hydroxy-4-methyl-2-trifluoromethylpentyl]-5-methyl-1,5-dihydropyrrolo[3,2-c]pyridin-6-one; 4-(5-chloro-2,3-dihydrobenzofuran-7-yl)-1,1,1-trifluoro-2-(6-methoxy-5,6-dihydro-1H-pyrrolo[3,2-c]pyridin-2-ylmethyl)-4-methylpentan-2-ol; 2-[4-(5-chloro-2,3-dihydrobenzofuran-7-yl)-2-hydroxy-4-methyl-2-trifluoromethylpentyl]-1,7-dihydropyrrolo[3,2-c]pyridine-4,6-dione; 6-[4-(5-chloro-2,3-dihydrobenzofuran-7-yl)-2-hydroxy-4-methyl-2-trifluoromethylpentyl]-3-methyl-1,7-dihydropyrrolo[2,3-d]pyrimidine-2,4-dione; 2-[4-(3-dimethylaminomethylphenyl)-2-hydroxy-4-methyl-2-trifluoromethylpentyl]-1H-indole-5-carbonitrile; 1,1,1-trifluoro-2-(1H-indol-2-ylmethyl)-4-methyl-4-(3-morpholin-4-ylmethylphenyl)pentan-2-ol; 1,1,1-trifluoro-4-methyl-4-(3-morpholin-4-ylmethylphenyl)-2-(1H-pyrrolo[2-,3-d]pyridazin-2-ylmethyl)pentan-2-ol; 1,1,1-trifluoro-4-(5-fluoro-2-methylphenyl)-4-methyl-2-(5-morpholin-4-ytmethyl-1H-indol-2-ylmethyl)pentan-2-ol; 1,1,1-trifluoro-4-(5-fluoro-2-methylphenyl)-4-methyl-2-(5-morpholin-4-ylmethyl-1H-pyrrolo[2,3-c]pyridin-2-ylmethyl)pentan-2-ol; {2-[4-(5-fluoro-2-methylphenyl)-2-hydroxy-4-methyl-2-trifuoromethylpentyl]-1H-indol-5-yl)phenylmethanone; {2-[4-(5-fluoro-2-methylphenyl)-2-hydroxy-4-methyl-2-trifluoromethylpenty-1]-1H-pyrrolo[2,3-c]pyridin-5-yl}phenylmethanone; {2-[4-(5- fluoro- 2- methylphenyl)- 2- hydroxy- 4- methyl- 2- trifluoromethylpentyl]- 1H- indol- 5- yl} furan- 2- ylmethanone; {2-[4-(5-fluoro-2-methylphenyl)-2-hydroxy-4-methyl-2-trifluoromethylpentyl]-1H-pyrrolo[2,3-c]pyridin-5-yl}furan-2-ylmethanone; 1,1,1-trifluoro-2-(1H-indol-2-ylmethyl)-4-methyl-4-pyridin-2-ylpentan-2-ol; 1,1,1-trifluoro.4-methyl-4-pyridin-4-yl-2-quinolin-4-ylmethylpentan-2-ol; 2-(2,6-dimethylpyridin-4-ylmethyl)-1,1,1-trifluoro-4-(5-fluoro-2-methoxyphenyl)-4-methylpentan-2-ol; 2-[3-(2,6-dimethylpyridin-4-ylmethyl)-4,4,4-trifluoro-3-hydroxy-1,1-dimethylbutyl]-4-fluorophenol; 1,1,1-trifluoro-4,4-dimethyl-5-phenyl-2-quinolin-4-ylmethylpentan-2-ol; 1,1,1-trifluoro-4-(5-fluoro-2-methoxyphenyl)-4-methyl-2-pyridin-4-ylmethylpentan-2-ol; 4-fluoro-2-[4,4,4-trifluoro-3-(2-fluoropyridin-4-ylmethyl)-3-hydroxy-1,1-dimethylbutyl]phenol; 2-[3-(2-bromopyridin-4-ylmethyl)-4,4,4-trifluoro-3-hydroxy-1,1-dimethylbutyl]-4-fluorophenol; 2-(6,8-dimethylquinolin-4-ylmethyl)-1,1,1-trifluoro-4-(5-fluoro-2-methoxy- phenyl)-4-methylpentan-2-ol; 4-[4-(5-fluoro-2-methoxyphenyl)-2-hydroxy-4-methyl-2-trifluoromethylpentyl]pyridine-2-carbonitrile; 2,6-dichloro-4-[4-(5-fluoro-2-methoxyphenyl)-2-hydroxy-4-methyl-2-trifluoromethylpentyl]nicotinonitrile; 4-[4-(5-fluoro-2-methoxyphenyl)-2-hydroxy-4-methyl-2-trifluoromethylpentyl]quinolin-2-ol; 2,6-dichloro-4-[4-(5-fluoro-2-hydroxyphenyl)-2-hydroxy-4-methyl-2-trifluoromethylpentyl]nicotinonitrile; 2-(2-chloro-8-methylquinolin-4-ylmethyl)-1,1,1-trifluoro-4-(5-fluoro-2-methoxyphenyl)-4-methylpentan-2-ol; 2-(2,6-dichloroquinolin-4-ylmethyl)-1,1,1-trifluoro-4-(5-fluoro-2-methoxyphenyl)-4-methylpentan-2-ol; 2-[3-(2-chloro-8-methylquinolin-4-ylmethyl)-4,4,4-trifluoro-3-hydroxy-1,1-dimethylbutyl]-4-fluorophenol; 2-[3-(2,6-dichloroquinolin-4-ylmethyl)-4,4,4-trifluoro-3-hydroxy-1,1-dimethylbutyl]-4-fluorophenol; 4-(2,3-dihydrobenzofuran-7-yl)-2-(2,6-dimethylpyridin-4-ylmethyl)-1,1,1-trifluoro-4-methylpentan-2-ol; 2-(2,6-dimethylpyridin-4-ylmethyl)-1,1,1-trifluoro-4-(3-fluorophenyl)-4-methylpentan-2-ol; 2-(2,6-dimethylpyridin-4-ylmethyl)-1,1,1-trifluoro-4-(4-fluorophenyl)-4-methylpentan-2-ol; 1,1,1-trifluoro-4-(5-fluoro-2-methylphenyl)-4-methyl-2-quinolin-4-ylmethylpentan-2-ol; 2-(2,6-dimethylpyridin-4-ylmethyl)-1,1,1-trifluoro-4-(5-fluoro-2-methylphenyl)-4-methylpentan-2-ol; 2-(2,6-dimethylpyridin-4-ylmethyl)-1,1,1-trifluoro-4-methyl-4-m-tolylpentan-2-ol; 1,1,1-trifluoro-4-(5-fluoro-2-methoxyphenyl)-4-methyl-2-(2-methylquinolin-4-ylmethyl)pentan-2-ol; 4-fluoro-2-(4,4,4-trifluoro-3-hydroxy-1,1,1-dimethyl-3-quinolin-4-ylmethylbutyl) phenol; 4-fluoro-2-[4,4,4-trifluoro-3-hydroxy-1,1-dimethyl-3-(2-methylquinolin-4-ylmethyl)butyl]phenol; 2-(2,6-dimethylpyridin-4-ylmethyl)-1,1,1-trifluoro-4-(4-fluoro-2-methoxyphenyl)-4-methylpentan-2-ol; 1,1,1-trifluoro-4-(5-fluoro-2-methoxyphenyl)-4-methyl-2-(7-methylquinolin-4-ylmethyl)pentan-2-ol; 2-[3-(2,6-dimethylpyridin-4-ylmethyl)-4,4,4-trifluoro-3-hydroxy-1,1-dimethylbutyl]-5-fluorophenol; and 2-(5,7-dimethylquinolin-4-ylmethyl)-1,1,1-trifluoro-4-(5-fluoro-2-methoxyphenyl)-4-methylpentan-2-ol.

In still another embodiment, said at least a DIGRA has Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises a heteroaryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, or trifluoromethyl.

Examples of these compounds include 2-cyclopropyl-4-(5-fluoro-2-methoxyphenyl)-4-methyl-1-(1H-pyrrolo[3,2-c]pyridin-2-yl)pentan-2-ol; 4-(5-fluoro-2-methoxyphenyl)-2-hydroxy-4-methyl-2-(1H-pyrrolo[2,3-c]pyridin-2-ylmethyl)pentanoic acid; 4-(5-fluoro-2-methoxyphenyl)-2-hydroxy-4-methyl-2-(1H-pyrrolo[2,3-c]pyridin-2-ylmethyl)pentanoic acid methyl ester; 2-cyclopropyl-4-(5-fluoro-2-methylphenyl)-4-methyl-1-(1H-pyrrolo[2,3-c]pyridin-2-yl)pentan-2-ol; 4-(5-chloro-2,3-dihydrobenzofuran-7-yl)-2-cyclopropyl-4-methyl-1-(1H-pyrrolo[2,3-c]pyridin-2-yl)pentan-2-ol; 2-cyclopropyl-4-(5-fluoro-2-methylphenyl)-4-methyl-1-(1H-pyrrolo[3,2-c]pyridin-2-yl)pentan-2-ol; 4-(5-chloro-2,3-dihydrobenzofuran-7-yl)-2-cyclopropyl-4-methyl-1-(1H-pyrrolo[3,2-c]pyridin-2-yl)pentan-2-ol; 4-(5-fluoro-2-methoxyphenyl)-2,4-dimethyl-1-(1H-pyrrolo[2,3-c]pyridin-2-yl)pentan-2-ol; 5-(5-fluoro-2-methoxyphenyl)-2,5-dimethyl-3-(1H-pyrrolo[2,3-c]pyridin-2-ylmethyl)hexan-3-ol; 5-(5-fluoro-2-methoxyphenyl)-2,2,5-trimethyl-3-(1H-pyrrolo[2,3-c]pyridin-2-ylmethyl)hexan-3-ol; 2-cyclohexyl-4-(5-fluoro-2-methoxyphenyl)-4-methyl-1-(1H-pyrrolo[2,3-c]pyridin-2-yl)pentan-2-ol; 2-cyclopentyl-4-(5-fluoro-2-methoxyphenyl)-4-methyl-1-(1H-pyrrolo[2,3-c]pyridin-2-yl)pentan-2-ol; 5-(5-fluoro-2-methoxyphenyl)-5-methyl-3-(1H-pyrrolo[2,3-c]pyridin-2-ylmethyl)hexan-3-ol; 2-(5-fluoro-2-methoxyphenyl)-2,6-dimethyl-4-(1H-pyrrolo[2,3-c]pyridin-2-ylmethyl)heptan-4-ol; 2-(5-fluoro-2-methoxyphenyl)-2,5,5-trimethyl-4-(1H-pyrrolo[2,3-c]pyridin-2-ylmethyl)heptan-4-ol; 1,1-difluoro-4-(5-fluoro-2-methoxyphenyl)-4-methyl-2-(1H-pyrrolo[2,3-c]pyridin-2-ylmethyl)pentan-2-ol; 1-cyclohexyl-4-(5-fluoro-2-methoxyphenyl)-4-methyl-2-(1H-pyrrolo[2,3-c]pyridin-2-ylmethyl)pentan-2-ol; 5-(5-fluoro-2-methylphenyl)-2,5-dimethyl-3-(1H-pyrrolo[2,3-c]pyridin-2-ylmethyl)hexan-3-ol; 5-(5-fuoro-2-methylphenyl-)-2,2,5-trimethyl-3-(1H-pyrrolo[2,3-c]pyridin-2-ylmethyl)hexan-3-ol; 5-(5-chloro-2,3-dihydrobenzofuran-7-yl)-2,5-dimethyl-3-(1H-pyrrolo[2,3-c]pyridin-2-ylmethyl)hexan-3-ol; 2-cyclobutyl-4-(5-fluoro-2-methoxyphenyl)-4-methyl-1-(1H-pyrrolo[2,3-c]pyridin-2-yl)pentan-2-ol; 2-(5-fluoro-2-methoxyphenyl)-2,6,6-trimethyl-4-(1H-pyrrolo[2,3-c]pyridin-2-ylmethyl)heptan-4-ol; 5-(5-fluoro-2-methoxyphenyl)-5-methyl-3-(1H-pyrrolo[2,3-c]pyridin-2-ylmethyl)hex-1-en-3-ol; 5-(5-fluoro-2-methoxyphenyl)-5-methyl-3-(1H-pyrrolo[2,3-c]pyridin-2-ylmethyl)hex-1-yn-3-ol; 1-fluoro-4-(5-fluoro-2-methoxyphenyl)-4-methyl-2-(1H-pyrrolo[2;3-c]pyridin-2-ylmethyl)pentan-2-ol; 2,2-difluoro-5-(5-fluoro-2-methoxyphenyl)-5-methyl-3-(1H-pyrolo[2,3-c]pyridin-2-ylmethyl)hexan-3-ol; 2-fluoro-5-(5-fluoro-2-methoxyphenyl)-2,5-dimethyl-3-(1H-pyrrolo[2,3-c]pyridin-2-ylmethyl)hexan-3-ol; 2-fluoro-5-(5-fluoro-2-methoxyphenyl)-5-methyl-3-(1H-pyrrolo[2,3-c]pyridin-2-ylmethyl)hexan-3-ol; 5-(5-fluoro-2-methoxyphenyl)-2,5-dimethyl-3-(1H-pyrrolo[2,3-c]pyridin-2-ylmethyl)hex-1-en-3-ol; 1,1,1-trifluoro-5-(5-fluoro-2-methoxyphenyl)-5-methyl-3-(1H-pyrrolo[2,3-c]pyridin-2-ylmethyl)hexan-3-ol; 4-(5-fluoro-2-methoxyphenyl)-4-methyl-2-phenyl-1-(1H-pyrrolo[2,3-c]pyridin-2-yl)pentan-2-ol; 5-(5-chloro-2,3-dihydrobenzofuran-7-yl)-2,2,5-trimethyl-3-(1H-pyrrolo[2,3-c]pyridin-2-ylmethyl)hexan-3-ol; 5-(5-fluoro-2-methylphenyl)-2,2,5-trimethyl-3-thieno[2,3-c]pyridin-2-ylmethylhexan-3-ol; 1,1-difluoro-4-(5-fluoro-2-methoxyphenyl)-4-methyl-2-(1H-pyrrolo[3,2-c]pyridin-2-ylmethyl)pentan-2-ol; 5-(5-fluoro-2-methoxyphenyl)-2,5-dimethyl-3-(1H-pyrrolo[3,2-c]pyridin-2-ylmethyl)hexan-3-ol; 5-(5-fluoro-2-methoxyphenyl)-2,2,5-trimethyl-3-(1H-pyrrolo[3,2-c]pyridin-2-ylmethyl)hexan-3-ol; 2-(1-fluorocyclopropyl)-4-(5-fluoro-2-methoxyphenyl)-4-methyl-1-(1H-pyrrolo[2,3-c]pyridin-2-yl)pentan-2-ol; 2-(1-fluorocyclopropyl)-4-(4-fluorophenyl)-4-methyl-1-quinolin-4-ylpentan-2-ol; 2-[4,4-difluoro-3-hydroxy-1,1-dimethyl-3-(1H-pyrrolo[3,2-c]pyridin-2-ylmethyl)butyl]-4-fluorophenol; 5-(5-chloro-2,3-dihydrobenzofuran-7-yl)-2,5-dimethyl-3-(1H-pyrrolo [3,2-c] pyridin-2-ylmethyl)hexan-3-ol; 5-(5-fluoro-2-methylphenyl)-2,5-dimethyl-3-(1H-pyrrolo[3,2-c]pyridin-2-ylmethyl)hexan-3-ol; 5-(5-fluoro-2-methylphenyl)-2,2,5-trimethyl-3-(1H-pyrrolo[3,2-c]pyridin-2-ylmethyl)hexan-3-ol; 4-(5-chloro-2,3-dihydrobenzofuran-7-yl)-1,1-difluoro-4-methyl-2-(1H-pyrrolo[3,2-c]pyridin-2-ylmethyl)pentan-2-ol; 4-(5-chloro-2,3-dihydrobenzofuran-7-yl)-1,1-difluoro-4-methyl-2-pyrrolo[3,2-b]pyridin-1-ylmethylpentan-2-ol; 5-(5-chloro-2,3-dihydrobenzofuran-7-yl)-2,2,5-trimethyl-3-(1H-pyrrolo[3,2-c]pyridin-2-ylmethyl)hexan-3-ol; 5-(5-fluoro-2-methylphenyl)-2,2,5-trimethyl-3-(3-methyl-1H-pyrrolo[2,3-c]pyridin-2-ylmethyl)hexan-3-ol; 5-(5-chloro-2,3-dihydrobenzofuran-7-yl)-2,5-dimethyl-3-(3-methyl-1H-pyrrolo[2,3-c]pyridin-2-ylmethyl)hexan-3-ol; 5-(5-chloro-2,3-dihydrobenzofuran-7-yl)-2,5-dimethyl-3-(5-phenyl-1H-pyrrolo[2,3-c]pyridin-2-ylmethyl)hexan-3-ol; 5-(5-fluoro-2-methylphenyl)-2,2,5-trimethyl-3-(5-phenyl-1H-pyrrolo[2,3-c]pyridin-2-ylmethyl)hexan-3-ol; 5-(5-fluoro-2-methylphenyl)-2,5-dimethyl-3-(5-phenyl-1H-pyrrolo[2,3-c]pyridin-2-ylmethyl)hexan-3-ol; 5-(5-fluoro-2-methylphenyl)-5-methyl-3-(5-phenyl-1H-pyrrolo[2,3-c]pyridin-2-ylmethyl)hexan-3-ol; 4-(5-fluoro-2-methylphenyl)-2,4-dimethyl-1-(5-phenyl-1H-pyrrolo[2,3-c]pyridin-2-yl)pentan-2-ol; 4-(5-chloro-2,3-dihydrobenzofuran-7-yl)-1,1-difluoro-4-methyl-2-(6-methyl-1H-pyrrolo[3,2-c]pyridin-2-ylmethyl)pentan-2-ol; 5-(5-fluoro-2-methylphenyl)-2,5-dimethyl-3-(5-pyridin-3-yl-1H-pyrrolo[2,3-c]pyridin-2-ylmethyl)hexan-3-ol; 5-(5-chloro-2,3-dihydrobenzofuran-7-yl)-5-methyl-3-(5-phenyl-1H-pyrrolo[2,3-c]pyridin-2-ylmethyl)hexan-3-ol; 4-(5-chloro-2,3-dihydrobenzofuran-7-yl)-2,4-dimethyl-1-(5-phenyl-1H-pyrrolo[2,3-c]pyridin-2-yl)pentan-2-ol; 1,1-difluoro-4-(5-methanesulfonyl-2,3-dihydrobenzofuran-7-yl)-4-methyl-2-(1H-pyrrolo[2,3-c]pyridin-2-ylmethyl)pentan-2-ol; 5-(5-chloro-2,3-dihydrobenzofuran-7-yl)-2,5-dimethyl-3-(5-pyridin-3-yl-1H-pynrolo[2,3-c]pyridin-2-ylmethyl)hexan-3-ol; 2-(5-bromo-1H-indol-2-ylmethyl)-1,1-difluoro-4-(5-methanesulfonyl-2,3-dihydrobenzofuran-7-yl)-4-methylpentan-2-ol; and 2-[2-difluoromethyl-2-hydroxy-4-(5-methanesulfonyl-2,3-dihydrobenzofuran-7-yl)-4-methylpentyl]-4-methyl-1H-indole-6-carbonitrile.

In still another embodiment, said at least a DIGRA has Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently $C_1$-$C_5$ alkyl, wherein one or both are independently substituted with hydroxy, $C_1$-$C_5$ alkoxy, $C_1$-$C_5$ alkylthio wherein the sulfur atom is optionally oxidized to a sulfoxide or sulfone, amino wherein the nitrogen atom is optionally independently mono- or di-substituted by $C_1$-$C_5$ alkyl or aryl;

(c) $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises a heteroaryl group optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, acyl, $C_1$-$C_3$ silanyloxy, $C_1$-$C_5$ alkoxycarbonyl, carboxy, halogen, hydroxy, oxo, cyano, heteroaryl, heterocyclyl, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, or trifluoromethyl.

In still another embodiment, said at least a DIGRA has Formula I, wherein

(a) A is an aryl, heteroaryl, heterocyclyl, or $C_3$-$C_8$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen, $C_1$-$C_5$ alkyl, $C_1$-$C_5$ arylalkyl, or $R^1$ and $R^2$ together with the carbon atom they are commonly attached to form a $C_3$-$C_8$ spiro cycloalkyl ring;

(c) B is the carbonyl group or methylene group, which is optionally independently substituted with one or two substituent groups selected from the group consisting of $C_1$-$C_3$ alkyl, hydroxy, and halogen;

(d) $R^3$ is the trifluoromethyl group;

(e) D is absent;

(f) E is the hydroxy group or amino group wherein the nitrogen atom is optionally independently mono- or di-substituted by $C_1$-$C_5$ alkyl; and

(g) Q comprises a 5- to 7-membered heterocyclyl ring fused to a 5- to 7-membered heteroaryl or heterocyclyl ring, each optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, oxo, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, $C_1$-$C_3$ alkoxycarbonyl, acyl, aryl, benzyl, heteroaryl, heterocyclyl, halogen, hydroxy, oxo, cyano, amino wherein the nitrogen atom is optionally independently mono- or di-substituted by $C_1$-$C_5$ alkyl, and ureido wherein either nitrogen atom is optionally independently substituted with $C_1$-$C_5$ alkyl or trifluoromethyl, wherein Q cannot be 1H-[1,5]naphthyridin-4-one.

Examples of these compounds include 4-[4-(5-fluoro-2-methoxyphenyl)-2-hydroxy-4-methyl-2-trifluoromethylpentyl]-4H-thieno[3,2-b]pyridin-7-one; 4-[4-(5-fluoro-2-hydroxyphenyl)-2-hydroxy-4-methyl-2-trifluoromethylpenty-1]-4H-thieno[3,2-b]pyridin-7-one; 4-[4-(2,3-dihydrobenzofuran-7-yl)-2-hydroxy-4-methyl-2-trifluoromethylpentyl]-4H-thieno[3,2-b]pyridin-7-one; 1-[4-(5-fluoro-2-methoxyphenyl)-2-hydroxy-4-methyl-2-trifluoromethylpentyl]-1H-[1,6]naphthyridin-4-one; 1-[4-(5-fluoro-2-hydroxyphenyl)-2-hydroxy-4-methyl-2-trifluoromethylpentyl]-1H-[1,6]naphthyridin-4-one; 4-[4-(5-fluoro-2-methylphenyl)-2-hydroxy-4-methyl-2-trifluoromethylpentyl]-4H-thieno[3,2-b]pyridin-7-one; 4-[2-hydroxy-4-(5-methanesulfonyl-2,3-dihydrobenzofuran-7-yl)-4-methyl-2-trifluoromethylpentyl]-4H-thieno[3,2-b]pyridin-7-one; 1-[2-hydroxy-4-(5-methanesulfonyl-2,3-dihydrobenzofuran-7-yl)-4-methyl-2-trifluoromethylpentyl]-1H-[1,6]naphthyridin-4-one; 1-[4-(5-fluoro-2-methylphenyl)-2-hydroxy-4-methyl-2-trifluoromethylpentyl]-1H-[1,6]naphthyridin-4-one; 4-[2-hydroxy-4-(2-methoxy-3-methylphenyl)-4-methyl-2-trifluoromethylpentyl]-4H-thieno[3,2-b]pyridin-7-one; 4-[2-hydroxy-4-(2-methoxyphenyl)-4-methyl-2-trifluoromethylpentyl]-4H-thieno[3,2-b]pyridin-7-one; 4-[4-(3-bromo-2-methoxyphenyl)-2-hydroxy-4-methyl-2-trifluoromethylpentyl]-4H-thieno[3,2-b]pyridin-7-one; 4-[2-hydroxy-4-(2-hydroxy-3-methylphenyl)-4-methyl-2-trifluoromethylpentyl]-4H-thieno[3,2-b]pyridin-7-one; 4-[4-(3-bromo-2-hydroxyphenyl)-2-hydroxy-4-methyl-2-trifluoromethylpentyl]-4H-thieno[3,2-b]pyridin-7-one; 3-bromo-1-[4-(5-chloro-2,3-dihydrobenzofuran-7-yl)-2-hydroxy-4-methyl-2-trifluoromethylpentyl]-1H-[1,6]naphthyridin-4-one; 6-chloro-4-[4-(2,3-dihydrobenzofuran-7-yl)-2-hydroxy-4-methyl-2-trifluoromethylpentyl]-4H-thieno[3,2-b]pyridin-7-one; 6-bromo-4-[4-(2,3-dihydrobenzofuran-7-yl)-2-hydroxy-4-methyl-2-trifluoromethylpentyl]-4H-thieno[3,2-b]pyridin-7-one; 3-chloro-1-[4-(5-fluoro-2-hydroxyphenyl)-2-hydroxy-4-methyl-2-trifluoromethylpentyl]-1H-[1,6]naphthyridin-4-one; 1-[4-(5-chloro-2,3-dihydrobenzofuran-7-yl)-2-hydroxy-4-methyl-2-trifluoromethylpentyl]-3-methyl-1H-[1,6]naphthyridin-4-one; 1-[4-(5-Chloro-2,3-dihydrobenzofuran-7-yl)-2-hydroxy-4-methyl-2-trifluoromethylpentyl]-3-methyl-1H-[1,7]naphthyridin-4-one; 1-[2-hydroxy-4-(2-methoxy-3,5-dimethylphenyl)-4-methyl-2-trifluoromethylpentyl]-3-methyl-1H-[1,6]naphthyridin-4-one; 1-[2-hydroxy-4-(2-methoxy-3,5-dimethylphenyl)-4-methyl-2-trifluoromethylpentyl]-3-methyl-1H-[1,7]naphthyridin-4-one; 1-[2-hydroxy-4-(2-hydroxy-3,5-dimethylphenyl)-4-methyl-2-trifluoromethylpentyl]-3-methyl-1H-[1,6]naphthyridin-4-one; 1-[4-(5-fluoro-2-methylphenyl)-2-hydroxy-4-methyl-2-trifluoromethylpentyl]-1H-[1,8]naphthyridin-4-one; 1-[4-(5-fluoro-2-methylphenyl)-2-hydroxy-4-methyl-2-trifluoromethylpentyl]-1H-[1,7]naphthyridin-4-one; 4-[4-(5-fluoro-2-hydroxyphenyl)-2-hydroxy-4-methyl-2-trifluoromethylpenty-1]-4H-thiazolo[4,5-b]pyridin-7-one; 4-[4-(5-fluoro-2-hydroxyphenyl)-2-hydroxy-4-methyl-2-trifluoromethylpentyl]-4H-oxazolo[4,5-b]pyridin-7-one; 4-[4-(5-fluoro-2-methylphenyl)-2-hydroxy-4-methyl-2-trifluoromethylpentyl]-4H-furo[3,2-b]pyridin-7-one; 7-[4-(5-fluoro-2-methylphenyl)-2-hydroxy-4-methyl-2-trifluoromethylpentyl]-7H-thieno[2,3-b]pyridin-4-one; 4-[4-(5-fluoro-2-hydroxyphenyl)-2-hydroxy-4-methyl-2-trifluoromethylpentyl]-4H-oxazolo[5,4-b]pyridin-7-one; 4-[4-(5-fluoro-2-hydroxyphenyl)-2-hydroxy-4-methyl-2-trifluoromethylpentyl]-4H-thiazolo[5,4-b]pyridin-7-one; 7-[4-(5-fluoro-2-methylphenyl)-2-hydroxy-4-methyl-2-trifluoromethylpentyl]-7H-furo[2,3-b]pyridin-4-one; 4-[4-(5-fluoro-2-methylphenyl)-2-hydroxy-4-methyl-2-trifluoromethylpentyl]-1,4-dihydropyrrolo[3,2-b]pyridin-7-one; 1-[4-(5-fluoro-2-hydroxyphenyl)-2-hydroxy-4-methyl-2-trifluoromethylpentyl]-5,6,7,8-tetrahydro-1H-[1,6]naphthyridin-4-one; 1-[4-(5-fluoro-2-methylphenyl)-2-hydroxy-4-methyl-2-trifluoromethylpentyl]-6-methyl-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-1H-[1,8]naphthyridin-4-one; 1-[2-hydroxy-4-(5-methanesulfonyl-2,3-dihydrobenzofuran-7-yl)-4-methyl-2-trifluoromethylpentyl]-1H-[1,7]naphthyridin-4-one; 4-[2-hydroxy-4-(5-methanesulfonyl-2,3-dihydrobenzofuran-7-yl)-4-methyl-2-trifluoromethylpentyl]-4-H-thiazolo[4,5-b]pyridin-7-one; 4-[4-(2,3-dihydrobenzofuran-7-yl)-2-hydroxy-4-methyl-2-trifluoromethylpentyl]-4H-oxazolo[4,5-b]pyridin-7-one; 4-[2-hydroxy-4-(5-methanesulfonyl-2,3-dihydrobenzofuran-7-yl)-4-methyl-2-trifluoromethylpentyl]-4H-furo[3,2-b]pyridin-7-one; 7-[4-(2,3-dihydrobenzofuran-7-yl)-2-hydroxy-4-methyl-2-trifluoromethylpentyl]-7H-thieno[2,3-b]pyridin-4-one; 4-[2-hydroxy-4-(5-methanesulfonyl-2,3-dihydrobenzofuran-7-yl)-4-methyl-2-trifluoromethylpentyl]-4H-oxazolo[5,4-b]pyridin-7-one; 4-[2-hydroxy-4-(5-methanesulfonyl-2,3-dihydrobenzofuran-7-yl)-4-methyl-2-trifluoromethylpentyl]-4H-thiazolo[5,4-b]pyridin-7-one; 7-[4-(2,3-dihydrobenzofuran-7-yl)-2-hydroxy-4-methyl-2-trifluoromethylpentyl]-7H-furo[2,3-b]pyridin-4-one; 4-[4-(2,3-dihydrobenzofuran-7-yl)-2-hydroxy-4-methyl-2-trifluoromethylpentyl]-1,4-dihydropyrrolo[3,2-b]pyridin-7-one; 1-[2-hydroxy-4-(5-methanesulfonyl-2,3-dihydrobenzofuran-7-yl)-4-methyl-2-trifluoromethylpentyl]-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-6-methyl-5,6,7,8-tetrahydro-1H-[1,6]naphthyridin-4-one; 1-[4-(2,3-dihydrobenzofuran-7-yl)-2-hydroxy-4-methyl-2-trifluoromethylpentyl]-5-methyl-5,6,7,8-tetrahydro-1H-[1,5]naphthyridin-4-one; 1-[4-(2,3-dihydrobenzofuran-7-yl)-2-hydroxy-4-methyl-2-trifluoromethylpentyl]-5-methyl-5,6,7,8-tetrahydro-1H-[1,5]naphthyridin-4-one; 4-[2-hydroxy-4-(4-methoxybiphenyl-3-yl)-4-methyl-2-trifluoromethylpentyl]-4H-thieno[3,2-b]pyridin-7-one; 4-[2-hydroxy-4-(2-methoxy-5-pyridin-3-ylphenyl)-4-methyl-2-trifluoromethylpentyl]-4H-thieno[3,2-b]pyridin-7-one; 4-[2-hydroxy-4-(2-methoxy-5-pyrimidin-5-ylphenyl)-4-methyl-2-trifluoromethylpentyl]-4H-thieno[3,2-b]pyridin-7-one; 4-[2-hydroxy-4-(2-methoxy-5-thiophen-3-ylphenyl)-4-methyl-2-trifluoromethylpentyl]-4H-thieno[3,2-b]pyridin-7-one; 4-[2-hydroxy-4-(4-hydroxybiphenyl-3-yl)-4-methyl-2-trifluoromethylpentyl]-4H-thieno[3,2-b]pyridin-7-one; 4-[2-hydroxy-4-(2-hydroxy-5-pyridin-3-ylphenyl)-4-methyl-2-trifluoromethylpentyl]-4H-thieno[3,2-b]pyridin-7-one; 4-[2-hydroxy-4-(2-hydroxy-5-pyrimidin-5-ylphenyl)-4-methyl-2-trifluoromethylpentyl]-4H-thieno[3,2-b]pyridin-7-one; 4-[2-Hydroxy-4-(2-hydroxy-5-thiophen-3-ylphenyl)-4-methyl-2-trifluoromethylpentyl]-4H-thieno[3,2-b]pyridin-7-one; 1-[2-hydroxy-4-(4-methoxybiphenyl-3-yl)-4-methyl-2-trifluoromethylpentyl]-1H-[1,6]naphthyridin-4-one; 1-[2-hydroxy-4-(2-methoxy-5-pyridin-3-ylphenyl)-4-methyl-2-trifluoromethylpentyl]-1H-[1,6]naphthyridin-4-one; 1-[2-hydroxy-4-(2-methoxy-5-pyrimidin-5-ylphenyl)-4-methyl-2-trifluoromethylpentyl]-1H-[1,6]naphthyridin-4-one; 1-[2-hydroxy-4-(2-methoxy-5-thiophen-3-ylphenyl)-4-methyl-2-trifluoromethylpentyl]-1H-[1,6]naphthyridin-4-one- ; 1-[2-hydroxy-4-(2-methoxy-5-thiophen-3-ylphenyl)-4-methyl-2-trifluoromethylpentyl]-1H-[1,6]naphthyridin-4-one; 1-[2-hydroxy-4-(2-hydroxy-5-pyridin-3-ylphenyl)-4-methyl-2-trifluoromethylpentyl]-1H-[1,6]naphthyridin-4-one; 1-[2-hydroxy-4-(2-hydroxy-5-pyrimidin-5-ylphenyl)-4-methyl-2-trifluoromethylpentyl]-1H-[1,6]naphthyridin-4-one; 1-[2-hydroxy-4-(2-hydroxy-5-thiophen-3-yphenyl)-4-methyl-2-trifluoromethylpentyl]-1H-[1,6]naphthyridin-4-one; 5-[4-(5-fluoro-2-methoxyphenyl)-2-hydroxy-4-methyl-2-trifluoromethylpentyl]-5H-pyrido[3,2-d]pyrimidin-8-one; 1-[4-(5-fluoro-2-methoxyphenyl)-2-hydroxy-4-methyl-2-trifluoromethylpentyl]-1H-pyrido[2,3-d]pyridazin-4-one; 5-[4-(5-fluoro-2-methoxyphenyl)-2-hydroxy-4-methyl-2-trifluoromethylpenty-1]-5H-pyrido[3,2. c]pyridazin-8-one; 4-[4-(2-fifluoromethoxy-3-methylphenyl- )-2-hydroxy-4-methyl-2-trifluoromethylpentyl]-4H-thieno[3,2-b]pyridin-7-one; 3-chloro-1-[4-(2,3-dihydrobenzofuran-7-yl)-2-hydroxy-4-methyl-2-trifluoromethylpentyl]-1H-[1,6]naphthyridin-4-one; 4-(4-benzo[1,3]dioxol-4-yl-2-hydroxy-4-methyl-2-trifluoromethylpentyl)-6-bromo-4H-thieno[3,2-b]pyridin-7-one; 4-(4-benzo[1,3]dioxol-4-yl-2-hydroxy-4-methyl-2-trifluoromethylpentyl)-6-chloro-4H-thieno[3,2-b]pyridin-7-one; 6-chloro-4-[2-hydroxy-4-methyl-4-(5-pyridin-3-yl-2,3-dihydrobenzofuran-7-yl)-2-trifluoromethylpentyl]-4H-thieno[3,2-b]pyridin-7-one; 1-(4-benzo[1,3]dioxol-4-yl-2-hydroxy-4-methyl-2-trifluoromethylpentyl)-3-chloro-1H-[1,6]naphthyridino4-one; 6-chloro-4-[2-hydroxy-4-methyl-4-(5-pyrimidin-5-yl-2,3-dihydrobenzofuran-7-yl)-2-trifluoromethylpentyl]-4H-thieno[3,2-b]pyridin-7-one; 3-chloro-1-[2-hydroxy-4-methyl-4-(5-pyrimidin-5-yl-2,3-dihydrobenzofuran-7-yl)-2-trifluoromethylpentyl]-1H-[1,6]naphthyridin-4-one; 3-chloro-1-[2-hydroxy-4-methyl-4-(5-pyridin-3-yl-2,3-dihydrobenzofuran-7-yl)-2-trifluoromethylpentyl]-1H-[1,6]naphthyridin-4-one; 4-[2-hydroxy-4-methyl-4-(5-pyrimidin-5-yl-2,3-dihydrobenzofuran-7-yl)-2-trifluoromethylpentyl]-4H-thieno[3,2-b]pyridin-7-one; 1-[2-hydroxy-4-methyl-4-(5-pyrimidin-5-yl-2,3-dihydrobenzofuran-7-yl)-2-trifluoromethylpentyl]-1H-[1,6]naphthyridin-4-one; 6-chloro-4-[2-hydroxy-4-(2-methoxy-5-pyridin-3-ylphenyl)-4-methyl-2-trifluoromethylpentyl]-4H-thieno[3,2-b]pyridin-7-one; 6-chloro-4-[2-hydroxy-4-(2-methoxy-5-pyrimidin-5-ylphenyl)-4-methyl-2-trifluoromethylpentyl]-4H-thieno[3,2-b]pyridin-7-one; 6-chloro-4-[2-hydroxy-4-(2-hydroxy-5-pyridin-3-ylphenyl)-4-methyl-2-trifluoromethylpentyl]-4H-thieno[3,2-b]pyridin-7-one; 6-chloro-4-[2-hydroxy-4-(-2-hydroxy-5-pyrimidin-5-ylphenyl)-4-methyl-2-trifluoromethylpentyl]-4H-thieno[3,2-b]pyridin-7-one; 4-(4-biphenyl-3-yl-2-hydroxy-4-methyl-2-trifluoromethylpentyl)-6-chloro-4H-thieno[3,2-b]pyridin-7-one; 4-(4-biphenyl-3-yl-2-hydroxy-4-methyl-2-trifluoromethylpentyl)-4H-thieno[3,2-b]pyridin-7-one; 3-chloro-1-{4-[5-(5-chloropyridin-3-yl)-2,3-dihydrobenzofuran-7-yl]-2-hydroxy-4-methyl-2-trifluoromethylpentyl}-1H-[1,6]naphthyridin-4-one; 6-chloro-4-{4-[5-(2,6-dimethylpyridin-4-yl)-2-methoxyphenyl]-2-hydroxy-4-methyl-2-trifluoromethylpentyl}-4H-thieno[3,2-b]pyridin-7-one- ; 4-[2-hydroxy-4-(2-hydroxy-5-pyridin-2-ylphenyl)-4-methyl-2-trifluoromethylpentyl]-4H-thieno[3,2-b]pyridin-7-one; 6-chloro-4-[2-hydroxy-4-methyl-4-(5-pyrazin-2-yl-2,3-dihydrobenzofuran-7-yl)-2-trifluoromethylpentyl]-4H-thieno[3,2-b]pyridin-7-one; 3-chloro-1-[2-hydroxy-4-methyl-4-(5-pyrimidin-2-yl-2,3-dihydrobenzofuran-7-yl)-2-trifluoromethylpentyl]-1H-[1,6]naphthyridin-4-one; 5-{7-[3-(6-chloro-7-oxo-7H-thieno[3,2-b]pyridin-4-ylmethyl)-4,4,- 4-trifluoro-3-hydroxy-1,1-dimethylbutyl]-2,3-dihydrobenzofuran-5-yl}nicotinonitrile; 4-{4-Methoxy-3-[4,4,4-trifluoro-3-hydroxy-1,1-dimethyl-3-(7-oxo-7H-thieno[3,2-b]pyridin-4-ylmethyl)butyl]phenyl}pyridine-2-carbonitrile; 6-chloro-4-{4-[5-(2-fluoro-6-methylpyridin-4-yl)-2-methoxyphenyl]-2-hydroxy-4-methyl-2-trifluoromethylpentyl}-4H-thieno[3,2-b]pyridin-7-one; 3-chloro-1-{2-hydroxy-4-[5-(1H-imidazol-4-yl)-2,3-dihydrobenzofuran-7-yl]-4-methyl-2-trifluoromethylpentyl}-1H-[1,6]naphthyridin-4-one; 6-chloro-4-[2-hydroxy-4-methyl-4-(5-morpholin-4-yl-2,3-dihydrobenzofuran-7-yl)-2-trifluoromethylpentyl]-4H-thieno[3,2-b]pyridin-7-one; and 1-[2-hydroxy-4-methyl-4-(5-piperidin-1-yl-2,3-dihydrobenzofuran-7-yl)-2-trifluoromethylpentyl]-1H-[1,6]naphthyridin-4-one.

In yet another embodiment, said at least a DIGRA has Formula I, wherein A, B, D, E, $R^1$, and $R^2$ have the meanings disclosed immediately above, and $R^3$ is hydrogen, $C_1$-$C_8$ alkyl, $C_2$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl.

In yet another embodiment, said at least a DIGRA has Formula I, wherein

(a) A is an aryl, heteroaryl, heterocyclyl, or $C_3$-$C_8$ cycloalkyl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl;

(c) $R^3$ is the trifluoromethyl group;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is the hydroxy group; and

(g) Q comprises an indolyl group optionally substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone, wherein each substituent group of Q is optionally independently substituted with one to three substituent groups selected from the group consisting of $C_1$-$C_3$ alkyl, $C_1$-$C_3$ alkoxy, halogen, hydroxy, oxo, cyano, amino, and trifluoromethyl.

Examples of these compounds include 4-(5-bromo-2,3-dihydrobenzofuran-7-yl)-1,1,1-trifluoro-2-(1H-indol-2-ylmethyl)-4-methylpentan-2-ol; 1,1,1-trifluoro-2-(1H-indol-2-ylmethyl)-4-methyl-4-pyridin-2-ylpentan-2-ol; 4-(2,3-dihydro-5-cyanobenzofuran-7-yl)-1,1,1-trifluoro-2-(1H-indol-2-yl-methyl)-4-methylpentan-2-ol; 4-(2,3-dihydrobenzofuran-7-yl)-1,1,1-trifluoro-2-(1H-indol-2-ylmethyl)-4-methylpentan-2-ol; 1,1,1-trifluoro-4-(5-fluoro-2,3-dihydrobenzofuran-7-yl)-2-(1H-indol-2-ylmethyl)-4-methylpentan-2-ol; 1,1,1-trifluoro-2-(1H-indol-2-ylmethyl)-4-methyl-4-(5-methyl-2,3-dihydrobenzofuran-7-yl)pentan-2-ol; 4-(2,3-dihydrobenzofuran-5-yl)-1,1,1-trifluoro-2-(1H-indol-2-ylmethyl)-4-methylpentan-2-ol; 2-[4-(2,3-dihydrobenzofuran-7-yl)-2-hydroxy-4-methyl-2-trifluoromethylpentyl]-1H-indole-3-carbonitrile; 2-[4-(5-fluoro-2,3-dihydrobenzofuran-7-yl)-2-hydroxy-4-methyl-2-trifluoromethylpentyl]-1H-indole-3-carbonitrile; 2-[4-(5-bromo-2,3-dihydrobenzofuran-7-yl)-2-hydroxy-4-methyl-2-trifluoromethylpentyl]-1H-indole-3-carbonitrile; 2-[4-(2,3-dihydrobenzofuran-7-yl)-2-hydroxy-4-methyl-2-trifluoromethylpentyl]-4-methyl-1H-indole-6-carbonitrile; 2-[4-(2,3-dihydrobenzofuran-7-yl)-2-hydroxy-4-methyl-2-trifluoromethylpentyl]-1H-indole-5-carbonithile; 4-(2,3-dihydrobenzofuran-7-yl)-1,1,1-trifluoro-2-(7-fluoro-1H-indol-2-ylmethyl)4-methylpentan-2-ol; 1-[4-(2,3-dihydrobenzofuran-7-yl)-2-hydroxy-4-methyl-2-trifluoromethylpentyl]-1H-indole-3-carbonitrile; 4-(2,3-dihydrobenzofuran-7-yl)-1,1,1-trifluoro-4-methyl-2-(5-trifluoromethyl-1H-indol-2-ylmethyl)pentan-2-ol; and 1,1,1-trifluoro-2-(1H-indol-2-ylmethyl)-4-methyl-4-thiophen-3-ylpentan-2-ol.

In a further embodiment, said at least a DIGRA has Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(d) B is the methylene or carbonyl group;

(e) D is the -NH- group;

(f) E is the hydroxy group; and

(g) Q comprises the group

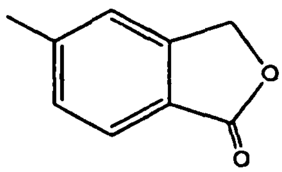
.

Examples of these compounds include 2-benzyl-2-hydroxy-4-methyl-4-phenylpentanoic acid (1-oxo-1,3-dihydroisobenzofuran-5-yl)amide; 2-hydroxy-4-methyl-2,4-diphenylpentanoic acid (1-oxo-1,3-dihydroisobenzofuran-5-yl) amide; 2-hydroxy-4-methyl-2-phenethyl-4-phenylpentanoic acid (1-oxo-1,3-dihydroisobenzofuran-5-yl)amide; 2-hydroxy-2-(3-methoxybenzyl)$_4$-methyl-4-phenylpentanoic acid (1-oxo-1,3-dihydroisobenzofuran-5-yl)amide; 2-hydroxy-2-(4-methoxybenzyl)-4-methyl-4-phenylpentanoic acid (1-oxo-1,3-dihydroisobenzofuran-5-yl)amide; 2-hydroxy-2-[2-(4-methoxyphenyl)ethyl]4-methyl-4-phenylpentanoic acid (1-oxo-1,3-dihydroisobenzofuran-5-yl)amide; 2-cyclohexylmethyl-2-hydroxy-4-methyl-4-phenylpentanoic acid (1-oxo-1,3-dihydroisobenzofuran-5-yl)amide; 2-(4-tert-butylbenzyl)-2-hydroxy-4-methyl-4-phenylpentanoic acid (1-oxo-1,3-dihydroisobenzofuran-5-yl)amide; 2-biphenyl-4-ylmethyl-2-hydroxy-4-methyl-4-phenylpentanoic acid (1-oxo-1,3-dihydroisobenzofuran-5-yl)amide; 2-hydroxy-4-methyl-2-naphthalen-2-ylmethyl-4-phenylpentanoic acid (1-oxo-1,3-dihydroisobenzofuran-5-yl)amide; 2-hydroxy-2-(3-hydroxybenzyl)-4-methyl-4-phenylpentanoic acid (1-oxo-1,3-dihydroisobenzofuran-5-yl)amide; 2-hydroxy-4-methyl-2-(2-methyl-2-phenylpropyl)-4-phenylpentanoic acid (1-oxo-1,3-dihydroisobenzofuran-5-yl)amide; 2-benzyl-4-(5-fluoro-2-methoxyphenyl)-2-hydroxy-4-methylpentanoic acid (1-oxo-1,3-dihydroisobenzofuran-5-yl)amide; 2-cyclohexylmethyl-4-(5-fluoro-2-methoxyphenyl)-2-hydroxy-4-methylpentanoic acid (1-oxo-1,3-dihydroisobenzofuran-5-yl)amide; 2-benzyl-4-(5-fluoro-2-hydroxyphenyl)-2-hydroxy-4-methylpentanoic acid (1-oxo-1,3-dihydroisobenzofuran-5-yl)amide; 2-cyclohexylmethyl-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(2-methyl-2-phenylpropyl)pentanoic acid (1-oxo-1,3-dihydroisobenzofuran-5-yl) amide; 2-(2-chloro-6-fluorobenzyl)-4-(5-fluoro-2-methoxyphenyl)-2-hydroxy-4-methylpentanoic acid (1-oxo-1,3-dihydroisobenzofuran-5-yl)amide; 2-(3-fluorobenzyl)-4-(5-fluoro-2-methoxyphenyl)-2-hydroxy-4-methylpentanoic acid (1-oxo-1,3-dihydroisobenzofuran-5-yl)amide; 2-(2-fluorobenzyl)-4-(5-fluoro-2-methoxyphenyl)-2-hydroxy-4-methylpentanoic acid (1-oxo-1,3-dihydroisobenzofuran-5-yl)amide; 2-(3,4-difluorobenzyl)-4-(5-fluoro-2-methoxyphenyl)-2-hydroxy-4-methylpentanoic acid (1-oxo-1,3-dihydroisobenzofuran-5-yl)amide; 2-(2-chloro-6-fluorobenzyl)-4-(5-fluoro-2-hydroxyphenyl)-2-hydroxy-4-methylpentanoic acid (1-oxo-1,3-dihydroisobenzofuran-5-yl)amide; 2-(3-fluorobenzyl)-4-(5-fluoro-2-hydroxyphenyl)-2-hydroxy-4-methylpentanoic acid (1-oxo-1,3-dihydroisobenzofuran-5-yl)amide; 2-(2-fluorobenzyl)-4-(5-fluoro-2-hydroxyphenyl)-2-hydroxy-4-methylpentanoic acid (1-oxo-1,3-dihydroisobenzofuran-5-yl)amide; 2-(3,4-difluorobenzyl)-4-(5-fluoro-2-hydroxyphenyl)-2-hydroxy-4-methylpentanoic acid (1-oxo-1,3-dihydroisobenzofuran-5-yl) amide; 2-(4-fluorobenzyl)-4-(5-fluoro-2-methoxyphenyl)-2-hydroxy-4-methylpentanoic acid (1-oxo-1,3-dihydroisobenzofuran-5-yl)amide; 4-(5-fluoro-2-methoxyphenyl)-2-hydroxy-4-methyl-2-(3-methylbenzyl)pentanoic acid (1-oxo-1,3-dihydroisobenzofuran-5-yl)amide; 2-(4-fluorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(3-methylbenzyl)pentanoic acid (1-oxo-1,3-dihydroisobenzofuran-5-yl)amide; 2-(3,5-difluorophenyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-methoxyphenyl)-2-hydroxy-4-methyl-2-(2-methylbenzyl)pentanoic acid (1-oxo-1,3-dihydroisobenzofuran-5-yl)amide; 2-(3,5-dimethylbenzyl)-4-(5-fluoro-2-methoxyphenyl)-2-hydroxy-4-methylpentanoic acid (1-oxo-1,3-dihydroisobenzofuran-5-yl)amide; 2-(2,5-difluorobenzyl)-4-(5-fluoro-2-methoxyphenyl)-2-hydroxy-4-methylpentanoic acid (1-oxo-1,3-dihydroisobenzofuran-5-yl)amide; 2-(2,5-difluorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-(2-methylbenzyl)pentanoic acid (1-oxo-1,3-dihydroisobenzofuran-5-yl)amide; 2-(3,5-dimethylbenzyl)-4-(5-fluoro-2-hydroxyphenyl)-2-hydroxy-4-methylpentanoic acid (1-oxo-1,3-dihydroisobenzofuran-5-yl)amide; 2-(3-chlorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-methoxyphenyl)-2-hydroxy-2-[2-(4-methoxyphenyl)ethyl]-4-methylpentanoic acid (1-oxo-1,3-dihydroisobenzofuran-5-yl)amide; 4-(5-fluoro-2-methoxyphenyl)-2-hydroxy-2-(2-methoxybenzyl)$_4$-methylpentanoic acid (1-oxo-1,3-dihydroisobenzofuran-5-yl)amide; 4-(5-fluoro-2-methoxyphenyl)-2-hydroxy-4-methyl-2-phenethylpentanoic acid (1-oxo-1,3-dihydroisobenzofuran-5-yl)amide; 2-(2-chlorobenzyl)-4-(5-fluoro-2-methoxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-4-methyl-2-phenethylpentanoic acid (1-oxo-1,3-dihydroisobenzofuran-5-yl) amide; 4-(5-fluoro-2-hydroxyphenyl)-2-hydroxy-2-[2-(4-hydroxyphenyl)ethyl]-4-methylpentanoic acid (1-oxo-1,3-dihydroisobenzofuran-5-yl)amide; 2-(2-chlorobenzyl)-4-(5-fluoro-2-hydroxyphenyl)-2-hydroxy-4-methylpentanoic acid (1-oxo-1,3-dihydroisobenzofuran-5-yl)amide; 4-(5-fluoro-2-hydroxyphenyl)-2-hydroxy-2-(2-hydroxybenzyl)-4-methylpentanoic acid (1-oxo-1,3-dihydroisobenzofuran-5-yl)amide; 2-(2-bromobenzyl)-4-(5-fluoro-2-methoxyphenyl)-2-hydroxy-4-methylpentanoic acid (1-oxo-1,3-dihydroisobenzofuran-5-yl)amide; 2-(2-bromobenzyl)-4-(5-fluoro-2-hydroxyphenyl)-2-hydroxy-4-methylpentanoic acid (-oxo-1,3-dihydroisobenzofuran-5-yl)amide; 2-(5-fluoro-2-methoxybenzyl)-2-hydroxy-4-methyl-4-phenylpentanoic acid (1-oxo-1,3-dihydroisobenzofuran-5-yl)amide; 2-(5-fluoro-2-hydroxybenzyl)-2-hydroxy-4-methyl-4-phenylpentanoic acid (1-oxo-1,3-dihydroisobenzofuran-5-yl)amide; 2-(5-fluoro-2-methoxybenzyl)-4-(5-fluoro-2-methoxyphenyl)-2-hydroxy-4-methylpentanoic acid (1-oxo-1,3-dihydroisobenzofuran-5-yl)amide; 2-(5-fluoro-2-hydroxybenzyl)-4-(5-fluoro-2-hydroxyphenyl)-2-hydroxy-4-methylpentanoic acid (1-oxo-1,3-dihydroisobenzofuran-5-yl) amide; 2-(3,5-dimethoxybenzyl)-2-hydroxy-4-methyl-4-phenylpentanoic acid (1-oxo-1,3-dihydroisobenzofuran-5-yl) amide; 2-(3,5-dihydroxybenzyl)-2-hydroxy-4-methyl-4-phenylpentanoic acid (1-oxo-1,3-dihydroisobenzofuran-5-yl)-amide; 2-hydroxy-2-(2-methoxybenzyl)$_4$-methyl-4-phenylpentanoic acid (1-oxo-1,3-dihydroisobenzofuran-5-yl)amide; 12-hydroxy-2-(2-hydroxybenzyl)-4-methyl-4-phenylpentanoic acid (1-oxo-1,3-dihydroisobenzofuran-5-yl)amide; 2-hydroxy-2-[2-(4-hydroxyphenyl)ethyl]-4-methyl-4-phenylpentanoic acid (1-oxo-1,3-dihydroisobenzofuran-5-yl)amide; 15-[2-benzyl-4-(5-fluoro-2-methoxyphenyl)-2-hydroxy-4-methylpentylamino]-3H-isobenzofuran-1-one; 4-(5-fluoro-2-methoxyphenyl)-2-hydroxy-4-methyl-2-(1-phenylvinyl)pentanoic acid (1-oxo-1,3-dihydroisobenzofuran-5-yl)amide; 2-hydroxy-4-methyl-4-phenyl-2-pyridin-2-ylmethylpentanoic acid(1-oxo-1,3-dihydroisobenzofuran-5-yl)amide; 4-(5-fluoro-2-methoxyphenyl)-2-hydroxy-4-methyl-2-(1-phenylethyl)pentanoic acid(1-oxo-1,3-dihydroisobenzofuran-5-yl) amide; 4-(5-fluoro-2-hydroxyphenyl)-2-hydroxy-4-methyl-2-(1-phenylethyl)pentanoic acid(1-oxo-1,3-dihydroisobenzofuran-5-yl)amide; 2-cyclopentyl-4-(5-fluoro-2-methoxyphenyl)-2-hydroxy-4-methylpentanoic acid(1-oxo-1,3-dihydroisobenzofuran-5-yl)amide; 2-cyclopentyl-4-(5-fluoro-2-hydroxyphenyl)-2-hydroxy-4-methylpentanoic acid(1-oxo-1,3-dihydroisobenzofuran-5-yl)amide; 2-cyclopentylmethyl-4-(5-fluoro-2-hydroxyphenyl)-2-hydroxy-4-methylpentanoic acid(1-oxo-1,3-dihydroisobenzofuran-5-yl)amide; and 2-benzyl-2-hydroxy-N-(1-oxo-1,3-dihydroisobenzofuran-5-yl)$_4$-phenylbutyramide.

In still another embodiment, said at least a DIGRA has Formula I, wherein

(a) A is an aryl or heteroaryl group, each optionally independently substituted with one to three substituent groups, which are independently selected from the group consisting of $C_1$-$C_5$ alkyl, $C_2$-$C_5$ alkenyl, $C_2$-$C_5$ alkynyl, $C_1$-$C_3$ alkanoyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, aroyl, aminocarbonyl, alkylaminocarbonyl, dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaninosulfonyl, halogen, hydroxy, carboxy, cyano, trifluoromethyl, trifluoromethoxy, nitro, amino wherein the nitrogen atom is optionally independently mono- or di-substituted by $C_1$-$C_5$ alkyl or aryl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone;

(b) $R^1$ and $R^2$ are each independently hydrogen or $C_1$-$C_5$ alkyl, or $R^1$ and $R^2$ together with the carbon atom they are commonly attached to form a $C_3$-$C_8$ spiro cycloalkyl ring;

(c) $R^3$ is the trifluoromethyl group;

(d) B is $C_1$-$C_5$ alkylene, $C_2$-$C_5$ alkenylene, or $C_2$-$C_5$ alkynylene, each optionally independently substituted with one to three substituent groups, wherein each substituent group of B is independently $C_1$-$C_3$ alkyl, hydroxy, halogen, amino, or oxo;

(e) D is absent;

(f) E is -$NR^6R^7$, wherein $R^6$ and $R^7$ are each independently hydrogen, $C_1$-$C_8$ alkyl, $C_2$-$C_8$ alkenyl, $C_2$-$C_8$ alkynyl, $C_1$-$C_8$ alkoxy, $C_2$-$C_8$ alkenyloxy, $C_2$-$C_8$ alkynyloxy, hydroxy, carbocyclyl, heterocyclyl, aryl, aryloxy, acyl, heteroaryl, carbocycle-$C_1$-$C_8$ alkyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, heteroaryl-$C_2$-$C_8$ alkenyl, or $C_1$-$C_5$ alkylthio wherein the sulfur atom is oxidized to a sulfoxide or sulfone, each optionally independently substituted with one to three substituent groups, wherein each substituent group of $R^6$ and $R^7$ are independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyl, $C_1$-$C_5$ alkylaminocarbonyl, $C_1$-$C_5$ dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, oxo, trifluoromethyl, trifluoromethoxy, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, or $C_1$-$C_5$ alkylthio wherein the sulfur atom is optionally oxidized to a sulfoxide or sulfone; and

(g) Q comprises a heteroaryl group optionally independently substituted with one to three substituent groups, wherein each substituent group of Q is independently $C_1$-$C_5$ alkyl, $C_2$-$C_5$ alkenyl, $C_2$-$C_5$ alkynyl, $C_3$-$C_8$ cycloalkyl, heterocyclyl, aryl, heteroaryl, $C_1$-$C_5$ alkoxy, $C_2$-$C_5$ alkenyloxy, $C_2$-$C_5$ alkynyloxy, aryloxy, acyl, $C_1$-$C_5$ alkoxycarbonyl, $C_1$-$C_5$ alkanoyloxy, aminocarbonyl, $C_1$-$C_5$ alkylaminocarbonyl, $C_1$-$C_5$ dialkylaminocarbonyl, aminocarbonyloxy, $C_1$-$C_5$ alkylaminocarbonyloxy, $C_1$-$C_5$ dialkylaminocarbonyloxy, $C_1$-$C_5$ alkanoylamino, $C_1$-$C_5$ alkoxycarbonylamino, $C_1$-$C_5$ alkylsulfonylamino, aminosulfonyl, $C_1$-$C_5$ alkylaminosulfonyl, $C_1$-$C_5$ dialkylaminosulfonyl, halogen, hydroxy, carboxy, cyano, trifluoromethyl, trifluoromethoxy, trifluoromethylthio, nitro, or amino wherein the nitrogen atom is optionally independently mono- or di-substituted by $C_1$-$C_5$ alkyl; or ureido wherein either nitrogen atom is optionally independently substituted with $C_1$-$C_5$ alkyl; or $C_1$-$C_5$ alkylthio wherein the sulfur atom is optionally oxidized to a sulfoxide or sulfone, wherein each substituent group of Q is optionally independently substituted with one to three substituent groups selected from $C_1$-$C_3$ alkyl, $C_1$-$C_3$ alkoxy, halogen, hydroxy, oxo, cyano, amino, or trifluoromethyl.

Examples of these compounds include 3-(5-fluoro-2-methoxyphenyl)-3-methyl-1-(pyridin-2-ylmethyl)-1-trifluoromethyl-butylamine; 3-(5-fluoro-2-methoxy-phenyl)-1-(1H-indol-2-ylmethyl)-3-methyl-1-trifluoromethyl-butylamine; 1-(2,6-dichloro-pyridin-4-ylmethyl)-3-(5-fluoro-2-methoxy-phenyl)-3-methyl-1-trifluoromethyl-butylamine; 1-(4,6-dimethyl-pyridin-2-ylmethy)-3-(5-fluoro-2-methoxy-phenyl)-3-methyl-1-trifluoromethyl-butylamine; 1-(2-chloro-pyridin-4-ylmethyl)-3-(5-fluoro-2-methoxy-phenyl)-3-methyl-1-trifluoromethyl-butylamine; 3-(5-fluoro-2-methyl-phenyl)-3-methyl-1-(3-methyl-1H-indol-2-ylmethyl)-1-trifluoromethyl-butylamine; 3-(5-fluoro-2-methoxy-phenyl)-3-methyl-1-(3-methyl-1H-indol-2-ylmethyl)-1-trifluoromethyl-butylamine; 1-(6-fluoro-1H-indol-2-ylmethyl)-3-(5-fluoro-2-methoxy-phenyl)-3-methyl-1-trifluoromethyl-butylamine; 3-(4-fluoro-phenyl)-3-methyl-1-(3-methyl-1H-indol-2-ylmethyl)-1-trifluoro-methyl-butylamine; 3-benzofuran-7-yl-1-(2,6-dichloro-pyridin-4-ylmethyl)-3-methyl-1-trifluoromethyl-butylamine; 3-(2,3-dihydro-benzofuran-7-yl)-1-(6-fluoro-1H-indol-2-ylmethyl)-3-methyl-1-trifluoromethyl-butylamine; 3-(5-fluoro-2-methoxy-phenyl)-3-methyl-1-quinolin-4-ylmethyl-1-trifluoromethyl-butylamine; 1-(2-chloro-quinolin-4-ylmethyl)-3-(5-fluoro-2-methylphenyl)-3-methyl-1-trifluoromethyl-butylamine; 3-(4-fluoro-phenyl)-3-methyl-1-quinolin-4-ylmethyl-1-trifluoromethyl-butylamine; 7-[3-amino-3-(1H-benzoimidazol-2-ylmethyl)-4,4,4-trifluoro-1,1-dimethyl-butyl]-2,3-dihydrobenzofuran-5-carbonitrile; 1-(6-fluoro-1H-benzoimidazol-2-ylmethyl)-3-(5-fluoro-2-methyl-phenyl)-3-methyl-1-trifluoromethyl-butylamine; 2-[3-amino-3-(1H-benzoimidazol-2-ylmethyl)-4,4,4-trifluoro-1,1-dimethyl-butyl]4-fluoro-phenol; 1-(1H-benzoimidazol-2-ylmethyl)-3-(4-fluoro-phenyl)-3-methyl-1-trifluoromethyl-butylamine; 1-(1H-indol-2-ylmethyl)-3-methyl-3-pyridin-3-yl-1-trifluoromethyl-butylamine; 1-(1H-benzoimidazol-2-ylmethyl)-3-methyl-3-pyridin-4-yl-1-trifluoromethyl-butylamine; 3-methyl-1-(3-methyl-1H-indol-2-ylmethyl)-3-pyridin-3-yl-1-trifluoromethyl-butylamine; 1-(6-fluoro-1H-indol-2-ylmethyl)-3-methyl-3-pyridin-3-yl-1-trifluoromethyl-butylamine; 3-(2,3-dihydrobenzofuran-7-yl)-1-(1H-indol-2-ylmethyl)-3-methyl-1-trifluoromethyl-butylamine; [3-(5-fluoro-2-methoxy-phenyl)-3-methyl-1-quinolin-4-ylmethyl-1-trifluoromethyl-butyl]-methyl-amine; ethyl-[3-(5-fluoro-2-methoxy-phenyl)-3-methyl-1-quinolin-4-ylmethyl-1-trifluoromethyl-butyl]-amine; [3-(5-fluoro-2-methoxy-phenyl)-3-methyl-1-quinolin-4-ylmethyl-1-trifluoromethyl-butyl]-propylamine; [3-(5-fluoro-2-methoxy-phenyl)-3-methyl-1-quinolin-4-ylmethyl-1-trifluoromethyl-butyl]-isobutylamine; butyl-[3-(5-fluoro-2-methoxy-phenyl)-3-methyl-1-quinolin-4-ylmethyl-1-trifluoromethyl-butyl]-amine; [3-(5-fluoro-2-methoxy-phenyl)-3-methyl- 1-quinolin-4-ylmethyl-1-trifluoromethyl-butyl]-dimethylamine; N-[3-(5-fluoro-2-methoxy-phenyl)-3-methyl-1-quinolin-4-ylmethyl-1-trifluoromethyl-butyl]-acetamide; N-[3-(5-fluoro-2-methoxy-phenyl)-3-methyl-1-quinolin-4-ylmethyl-1-trifluoromethyl-butyl]-formamide; N-[3-(5-fluoro-2-methoxy-phenyl)-3-methyl-1-quinolin-4-ylmethyl-1-trifluoromethyl-butyl]-methanesulfonamide; 1-(2,6-dimethyl-pyridin-4-ylmethyl)-3-(5-fluoro-2-methoxyphenyl)-3-methyl-1-trifluoromethyl-butylamine; 3-(5-fluoro-2-methoxy-phenyl)-3-methyl-1-(1H-pyrrolo[2,3-c]pyridin-2-ylmethyl)-1-trifluoromethyl-butylamine; 2-[2-amino-4-(5-fluoro-2-methoxy-phenyl)-4-methyl-2-trifluoromethyl-pentyl]-4-methyl-1H-indole-6-carbonitrile; N-[3-(5-fluoro-2-methoxy-phenyl)-3-methyl-1-quinolin-4-ylmethyl-1-trifluoromethyl-butyl]-hydroxylamine; and 2-(3-amino-4,4,4-trifluoro-1,1-dimethyl-3-quinolin-4-ylmethyl-butyl)-4-fluoro-phenol.

In yet another embodiment, said at least a DIGRA has Formula I, wherein A, B, D, E, $R^1$, $R^2$, $R^6$, and $R^7$ have the meanings disclosed immediately above, and $R^3$ is $C_1$-$C_8$ alkyl, $C_1$-$C_8$ alkenyl, $C_2$-$C_8$ alkynyl, carbocycle, heterocyclyl, aryl, heteroaryl, carbocycle-$C_1$-$C_8$ alkyl, carboxy, alkoxycarbonyl, aryl-$C_1$-$C_8$ alkyl, aryl-$C_1$-$C_8$ haloalkyl, heterocyclyl-$C_1$-$C_8$ alkyl, heteroaryl-$C_1$-$C_8$ alkyl, carbocycle-$C_2$-$C_8$ alkenyl, aryl-$C_2$-$C_8$ alkenyl, heterocyclyl-$C_2$-$C_8$ alkenyl, or heteroaryl-$C_2$-$C_8$ alkenyl, each optionally independently substituted with one to three substituent groups, wherein each substituent group of $R^3$ is independently $C_1$-$C_5$ alkyl, $C_2$-$C_5$ alkenyl, $C_2$-$C_5$ alkynyl, $C_3$-$C_8$ cycloalkyl, phenyl, $C_1$-$C_5$ alkoxy, phenoxy, $C_1$-$C_5$ alkanoyl, aroyl, $C_1$-$C_5$ alkoxycarbonyl, $C_1$-$C_5$ alkanoyloxy, aminocarbonyloxy, $C_1$-$C_5$ alkylaminocarbonyloxy, $C_1$-$C_5$ dialkylaminocarbonyloxy, aminocarbonyl, $C_1$-$C_5$ alkylaminocarbonyl, $C_1$-$C_5$ dialkylaminocarbonyl, $C_1$-$C_5$ alkanoylamino, $C_1$-$C_5$ alkoxycarbonylamino, $C_1$-$C_5$ alkylsulfonylamino, $C_1$-$C_5$ alkylaminosulfonyl, $C_1$-$C_5$ dialkylaminosulfonyl, halogen, hydroxy, carboxy, cyano, oxo, trifluoromethyl, nitro, amino wherein the nitrogen atom is optionally independently mono- or di-substituted by $C_1$-$C_5$ alkyl, ureido wherein either nitrogen atom is optionally independently substituted with $C_1$-$C_5$ alkyl, $C_1$-$C_5$ alkylthio wherein the sulfur atom is optionally oxidized to a sulfoxide or sulfone, wherein $R^3$ cannot be trifluoromethyl.

Examples of these compounds include 1-(2,6-dichloro-pyridin-4-ylmethyl)-3-(5-fluoro-2-methoxy-phenyl)-1,3-dimethyl-butylamine; 1-ethyl-3-(5-fluoro-2-methoxy-phenyl)-3-methyl-1-quinolin-4-ylmethyl-butylamine; 1-cyclohexylmethyl-3-(5-fluoro-2-methoxy-phenyl)-1-(1H-indol-2-ylmethyl)-3-methylbutylamine; 1-(2-chloro-quinolin-4-ylmethyl)-1-cyclopentyl-3-(5-fluoro-2-methoxyphenyl)-3-methyl-butylamine; 1-(2-chloro-pyridin-4-ylmethyl)-1-cyclopentylmethyl-3-(5-fluoro-2-methoxy-phenyl)-3-methyl-butylamine; 3-(5-fluoro-2-methoxy-phenyl)-1,3-dimethyl-1-quinolin-4-ylmethyl-butylamine; 1-cyclopropyl-3-(5-fluoro-2-methoxyphenyl)-3-methyl-1-quinolin-4-ylmethyl-butylamine; 3-(5-fluoro-2-methoxy-phenyl)-1,3-dimethyl-1-(1H-pyrrolo[2,3-c]pyridin-2-ylmethyl)-butylamine; 1-cyclopropyl-3-(5-fluoro-2-methoxy-phenyl)-3-methyl-1-(1H-pyrrolo[2,3-c]-pyridin-2-ylmethyl)-butylamine; 2-[3-amino-1,1,3-trimethyl-4-(1H-pyrrolo[2,3-c]pyridin-2-yl)-butyl]-4-fluoro-phenol; 2-[2-amino-4-(5-fluoro-2-methoxy-phenyl)-2,4-dimethyl-pentyl]-4-methyl- 1H-indole-6-carbonitrile.

Other compounds that can function as DIGRAs and methods for their manufacture are disclosed, for example, in U.S. Patent Application Publications 2004/0029932, 2004/0162321, 2004/0224992, 2005/0059714, 2005/0176706, 2005/0203128, 2005/0234091, 2005/0282881, 2006/0014787, 2006/0030561, 2006/0116396, 2006/0189646, and 2006/0189647.

In another aspect, the present invention provides an ophthalmic pharmaceutical composition for use in treating, reducing, ameliorating, or alleviating a back-of-the-eye condition or disorder as defined in the claims. In one embodiment, such a condition or disorder has an etiology in inflammation. In another embodiment, such an inflammation is a chronic inflammation. The ophthalmic pharmaceutical composition comprises at least a DIGRA, or a pharmaceutically acceptable

salt thereof as defined in the claims. In one aspect, the pharmaceutical composition further comprises a pharmaceutically acceptable carrier.

The concentration of a DIGRA, or a pharmaceutically acceptable salt thereof in such an ophthalmic composition can be in the range from about 0.0001 to about 1000 mg/ml (or, alternatively, from about 0.001 to about 500 mg/ml, or from about 0.001 to about 300 mg/ml, or from about 0.001 to about 250 mg/ml, or from about 0.001 to about 100 mg/ml, or from about 0.001 to about 50 mg/ml, or from about 0.01 to about 300 mg/ml, or from about 0.01 to about 250 mg/ml, or from about 0.01 to about 100 mg/ml, or from about 0.1 to about 100 mg/ml, or from about 0.1 to about 50 mg/ml).

In one embodiment, a composition of the present invention is in a form of a suspension or dispersion. In another embodiment, the suspension or dispersion is based on an aqueous solution. For example, a composition of the present invention can comprise sterile saline solution. In still another embodiment, micrometer- or nanometer-sized particles of a DIGRA, or a pharmaceutically acceptable salt thereof can be coated with a physiologically acceptable surfactant ( examples are disclosed below), then the coated particles are dispersed in an liquid medium. The coating can keep the particles in a suspension.

In another aspect, a composition of the present invention can further comprise a non-ionic surfactant, such as polysorbates (such as polysorbate 80 (polyoxyethylene sorbitan monooleate), polysorbate 60 (polyoxyethylene sorbitan monostearate), polysorbate 20 (polyoxyethylene sorbitan monolaurate), commonly known by their trade names of Tween® 80, Tween® 60, Tween® 20), poloxamers (synthetic block polymers of ethylene oxide and propylene oxide, such as those commonly known by their trade names of Pluronic®; e.g., Pluronic® F127 or Pluronic® F108)), or poloxamines (synthetic block polymers of ethylene oxide and propylene oxide attached to ethylene diamine, such as those commonly known by their trade names of Tetronic®; e.g., Tetronic® 1508 or Tetronic® 908, etc., other nonionic surfactants such as Brij®, Myrj®, and long chain fatty alcohols (i.e., oleyl alcohol, stearyl alcohol, myristyl alcohol, docosohexanoyl alcohol, etc.) with carbon chains having about 12 or more carbon atoms (e.g., such as from about 12 to about 24 carbon atoms). Such compounds are delineated in Martindale, 34th ed., pp 1411-1416 (Martindale, "The Complete Drug Reference," S. C. Sweetman (Ed.), Pharmaceutical Press, London, 2005) and in Remington, "The Science and Practice of Pharmacy," 21st Ed., p. 291 and the contents of chapter 22, Lippincott Williams & Wilkins, New York, 2006). The concentration of a non-ionic surfactant, when present, in a composition of the present invention can be in the range from about 0.001 to about 5 weight percent (or alternatively, from about 0.01 to about 4, or from about 0.01 to about 2, or from about 0.01 to about 1, or from about 0.01 to about 0.5 weight percent).

In addition, a composition of the present invention can include additives such as buffers, diluents, carriers, adjuvants, or other excipients. Any pharmacologically acceptable buffer suitable for application to the eye may be used. Other agents may be employed in the composition for a variety of purposes. For example, buffering agents, preservatives, co-solvents, oils, humectants, emollients, stabilizers, or antioxidants may be employed. Water-soluble preservatives which may be employed include sodium bisulfite, sodium bisulfate, sodium thiosulfate, benzalkonium chloride, chlorobutanol, thimerosal, ethyl alcohol, methylparaben, polyvinyl alcohol, benzyl alcohol, and phenylethyl alcohol. These agents may be present in individual amounts of from about 0.001 to about 5% by weight (preferably, about 0.01 % to about 2% by weight). Suitable water-soluble buffering agents that may be employed are sodium carbonate, sodium borate, sodium phosphate, sodium acetate, sodium bicarbonate, etc., as approved by the United States Food and Drug Administration ("US FDA") for the desired route of administration. These agents may be present in amounts sufficient to maintain a pH of the system of between about 2 and about 11. As such, the buffering agent may be as much as about 5% on a weight to weight basis of the total composition. Electrolytes such as, but not limited to, sodium chloride and potassium chloride may also be included in the formulation.

In one aspect, the pH of the composition is in the range from about 4 to about 11. Alternatively, the pH of the composition is in the range from about 5 to about 9, from about 6 to about 9, or from about 6.5 to about 8. In another aspect, the composition comprises a buffer having a pH in one of said pH ranges.

In another aspect, the composition has a pH of about 7. Alternatively, the composition has a pH in a range from about 7 to about 7.5.

In still another aspect, the composition has a pH of about 7.4.

In yet another aspect, a composition also can comprise a viscosity-modifying compound designed to facilitate the administration of the composition into the subject or to promote the bioavailability in the subject. In still another aspect, the viscosity-modifying compound may be chosen so that the composition is not readily dispersed after being administered into the vistreous. Such compounds may enhance the viscosity of the composition, and include: monomeric polyols, such as, glycerol, propylene glycol, ethylene glycol; polymeric polyols, such as, polyethylene glycol; various polymers of the cellulose family, such as hydroxypropylmethyl cellulose ("HPMC"), carboxymethyl cellulose ("CMC") sodium, hydroxypropyl cellulose ("HPC"); polysaccharides, such as hyaluronic acid and its salts, chondroitin sulfate and its salts, dextrans, such as, dextran 70; water soluble proteins, such as gelatin; vinyl polymers, such as, polyvinyl alcohol, polyvinylpyrrolidone, povidone; carbomers, such as carbomer 934P, carbomer 941, carbomer 940, or carbomer 974P; and acrylic acid polymers. In general, a desired viscosity can be in the range from about 1 to about 400 centipoises ("cps").

Also disclosed is a composition for use in treating, reducing, ameliorating, or alleviating a back-of-the-eye

condition or disorder. In one embodiment, the condition or disorder has an etiology in inflammation. In another embodiement, such inflammation is chronic inflammation. In still another aspect, the back-of-the-eye condition or disorder is selected form the group consisting of DR, AMD, DME, posterior uveitis, and combinations thereof. In a further aspect, such posterior uveitis is selected from the group consisting of one of choroiditis, retinitis, vasculitis, optic neuritis, and combinations thereof. The composition comprises: (a) at least a DIGRA, a prodrug thereof, or a pharmaceutically acceptable salt thereof; and (b) a material selected from the group consisting of (i) anti-inflammatory agents other than a DIGRA, a prodrug thereof, and a pharmaceutically acceptable salt thereof; (ii) anti-angiogenic agents; and (iii) combinations thereof; said DIGRA, prodrug thereof, or pharmaceutically acceptable salt thereof, anti-inflammatory agent, or anti-angiogenic agent being present in amounts effective to treat, reduce, ameliorate, or alleviate said back-of-the-eye condition or disorder. In one embodiment, such an anti-inflammatory agent is selected from the group consisting of non-steroidal anti-inflammatory drugs ("NSAIDs"), peroxisome proliferator-activated receptor-γ ("PPARγ") ligands, combinations thereof, and mixtures thereof.

Examples of the NSAIDs are: aminoarylcarboxylic acid derivatives (e.g., enfenamic acid, etofenamate, flufenamic acid, isonixin, meclofenamic acid, mefenamic acid, niflumic acid, talniflumate, terofenamate, tolfenamic acid), arylacetic acid derivatives (e.g., aceclofenac, acemetacin, alclofenac, amfenac, amtolmetin guacil, bromfenac, bufexamac, cinmetacin, clopirac, diclofenac sodium, etodolac, felbinac, fenclozic acid, fentiazac, glucametacin, ibufenac, indomethacin, isofezolac, isoxepac, lonazolac, metiazinic acid, mofezolac, oxametacine, pirazolac, proglumetacin, sulindac, tiaramide, tolmetin, tropesin, zomepirac), arylbutyric acid derivatives (e.g., bumadizon, butibufen, fenbufen, xenbucin), arylcarboxylic acids (e.g., clidanac, ketorolac, tinoridine), arylpropionic acid derivatives (e.g., alminoprofen, benoxaprofen, bermoprofen, bucloxic acid, carprofen, fenoprofen, flunoxaprofen, flurbiprofen, ibuprofen, ibuproxam, indoprofen, ketoprofen, loxoprofen, naproxen, oxaprozin, piketoprolen, pirprofen, pranoprofen, protizinic acid, suprofen, tiaprofenic acid, ximoprofen, zaltoprofen), pyrazoles (e.g., difenamizole, epirizole), pyrazolones (e.g., apazone, benzpiperylon, feprazone, mofebutazone, morazone, oxyphenbutazone, phenylbutazone, pipebuzone, propyphenazone, ramifenazone, suxibuzone, thiazolinobutazone), salicylic acid derivatives (e.g., acetaminosalol, aspirin, benorylate, bromosaligenin, calcium acetylsalicylate, diflunisal, etersalate, fendosal, gentisic acid, glycol salicylate, imidazole salicylate, lysine acetylsalicylate, mesalamine, morpholine salicylate, 1-naphthyl salicylate, olsalazine, parsalmide, phenyl acetylsalicylate, phenyl salicylate, salacetamide, salicylamide o-acetic acid, salicylsulfuric acid, salsalate, sulfasalazine), thiazinecarboxamides (e.g., ampiroxicam, droxicam, isoxicam, lornoxicam, piroxicam, tenoxicam), ε-acetamidocaproic acid, S-(5'-adenosyl)-L-methionine, 3-amino-4-hydroxybutyric acid, amixetrine, bendazac, benzydamine, α-bisabolol, bucolome, difenpiramide, ditazol, emorfazone, fepradinol, guaiazulene, nabumetone, nimesulide, oxaceprol, paranyline, perisoxal, proquazone, superoxide dismutase, tenidap, zileuton, their physiologically acceptable salts, combinations thereof, and mixtures thereof.

In another aspect of the present invention, an anti-inflammatory agent is a PPAR-binding molecule. In one embodiment, such a PPAR-binding molecule is a PPARα-, PPARδ-, or PPARγ-binding molecule. In another embodiment, such a PPAR-binding molecule is a PPARα, PPARδ, or PPARγ agonist. Such a PPAR ligand binds to and activates PPAR to modulate the expression of genes containing the appropriate peroxisome proliferator response element in its promoter region.

PPARγ agonists can inhibit the production of TNF-α and other inflammatory cytokines by human macrophages (C-Y. Jiang et al., Nature, Vol. 391, 82-86 (1998)) and T lymphocytes (A.E. Giorgini et al., Horm. Metab. Res. Vol. 31, 1-4 (1999)). More recently, the natural PPARγ agonist 15-deoxy-Δ-12,14-prostaglandin J2 (or "15-deoxy-Δ-12,14-PG J2"), has been shown to inhibit neovascularization and angiogenesis (X. Xin et al., J. Biol. Chem. Vol. 274:9116-9121 (1999)) in the rat cornea. Spiegelman et al., in U.S. Patent 6,242,196, disclose methods for inhibiting proliferation of PPARγ-responsive hyperproliferative cells by using PPARγ agonists; numerous synthetic PPARγ agonists are disclosed by Spiegelman et al., as well as methods for diagnosing PPARγ-responsive hyperproliferative cells. PPARs are differentially expressed in diseased versus normal cells. PPARγ is expressed to different degrees in the various tissues of the eye, such as some layers of the retina and the cornea, the choriocapillaris, uveal tract, conjunctival epidermis, and intraocular muscles (see, e.g., U.S. Patent 6,316,465).

In one aspect, a PPARγ agonist used in a composition of the present invention is a thiazolidinedione, a derivative thereof, or an analog thereof. Examples of thiazolidinedione-based PPARγ agonists include pioglitazone, troglitazone, ciglitazone, englitazone, rosiglitazone, and chemical derivatives thereof. Other PPARγ agonists include Clofibrate (ethyl 2-(4-chlorophenoxy)-2-methylpropionate), clofibric acid (2-(4-chlorophenoxy)-2-methylpropanoic acid), GW 1929 (N-(2-benzoylphenyl)-O-{2-(methyl-2-pyridinylamino)ethyl}-L-tyrosine), GW 7647 (2-{{4-{2-{{(cyclohexylamino)carbonyl}(4-cyclohexylbutyl)amino}ethyl}phenyl}thio}-2-methylpropanoic acid), and WY 14643 ({{4-chloro-6-{(2,3-dimethylphenyl) amino}-2-pyrimidinyl}thio}acetic acid). GW 1929, GW 7647, and WY 14643 are commercially available, for example, from Koma Biotechnology, Inc. (Seoul, Korea). In one embodiment, the PPARγ agonist is 15-deoxy-Δ-12, 14-PG J2.

Examples of PPAR-α agonists include the fibrates, such as fenofibrate and gemfibrozil. An example of PPAR-δ agonist is GW501516 (available from Axxora LLC, San Diego, California or EMD Biosciences, Inc., San Diego, California).

In a further aspect, an anti-angiogenic agent included in a pharmaceutical composition of the present disclosure is selected from the group consisting of: (i) compounds that interact with and inhibit a downstream activity of extracellular VEGF; (ii) compounds that interact with at least a VEGF receptor and render it substantially unavailable for interacting with VEGF; (iii) compounds that reduce a level of expression of VEGF; and (iv) combinations thereof.

In one embodiment, compounds that interact with and inhibit a downstream activity of extracellular VEGF comprise a nucleic acid ligand that binds to extracellular VEGF and substantially prevents it from participating in the angiogenic cascade. Examples of such a nucleic acid ligand are the VEGF aptamers disclosed in U.S. Patents 6,426,335; 6,168,778; 6,147,204; 6,051,698; and 6,051,020. In one embodiment, such a nucleic acid ligand comprises the VEGF antagonist aptamer known by its trade name "Macugen®", being marketed by OSI EyeTech Pharmaceuticals (Melleville, New York). In another embodiment, a compound that interacts with and inhibits a downstream activity of extracellular VEGF comprises an anti-VEGF antibody, such as the recombinant monoclonal antibody known as Lucentis® (ranibizumab, developed by Genentech, South San Francisco, California).

In one aspect of the present disclosure, compounds that interact with at least a VEGF receptor and render it substantially unavailable for interacting with VEGF comprises VEGF tyrosine kinase inhibitors, which can be a small synthetic molecule or protein or protein fragment that binds to the transmembrane VEGF receptors and neutralizes their activation, such as rendering them incapable of initiating or participating further in the expression of VEGF or other angiogenic factors.

Examples of synthetic VEGF tyrosine kinase inhibitors include the compounds disclosed in U.S. Patents 6,958,340; 6,514,971; 6,448,077; and U.S. Patent Application Publications 2005/0233921, 2005/0244475, 2005/0143442, and 2006/0014252.

In another aspect, a level of VEGF can be reduced by interfering with the transcription of the VEGF gene by binding a small organic VEGF-gene inhibitor to said gene, such as one of the compounds disclosed in U.S. Patent Application Publication 2003/0282849.

Other suitable anti-angiogenic agents that can be used in a composition of the present disclosure are disclosed in U.S. Patent Application having Serial No. 60/797,608 filed May 7, 2006.

In still another aspect, a method for preparing a composition of the present disclosure comprises combining: (i) at least a DIGRA, a prodrug thereof, or a pharmaceutically acceptable salt thereof; and (ii) a material selected from the group consisting of anti-inflammatory agent other than a DIGRA, anti-angiogenic agents, and combinations thereof; and (iii) a pharmaceutically acceptable carrier. In one embodiment, such a carrier can be a sterile saline solution or a physiologically acceptable buffer. In another embodiment, such a carrier comprises a hydrophobic medium, such as a pharmaceutically acceptable oil. In still another embodiment, such as carrier comprises an emulsion of a hydrophobic material and water.

Physiologically acceptable buffers include, but are not limited to, a phosphate buffer or a Tris-HCl buffer (comprising tris(hydroxymethyl)aminomethane and HCl). For example, a Tris-HCl buffer having pH of 7.4 comprises 3 g/l of tris(hydroxymethyl)aminomethane and 0.76 g/l of HCl. In yet another aspect, the buffer is 10X phosphate buffer saline ("PBS") or 5X PBS solution.

Other buffers also may be found suitable or desirable in some circumstances, such as buffers based on HEPES (N-{2-hydroxyethyl}peperazine-N'-{2-ethanesulfonic acid}) having $pK_a$ of 7.5 at 25 °C and pH in the range of about 6.8-8.2; BES (N,N-bis{2-hydroxyethyl}2-aminoethanesulfonic acid) having $pK_a$ of 7.1 at 25°C and pH in the range of about 6.4-7.8; MOPS (3-{N-morpholino}propanesulfonic acid) having $pK_a$ of 7.2 at 25°C and pH in the range of about 6.5-7.9; TES (N-tris{hydroxymethyl}-methyl-2-aminoethanesulfonic acid) having $pK_a$ of 7.4 at 25°C and pH in the range of about 6.8-8.2; MOBS (4-{N-morpholino}butanesulfonic acid) having $pK_a$ of 7.6 at 25°C and pH in the range of about 6.9-8.3; DIPSO (3-(N,N-bis{2-hydroxyethyl}amino)-2-hydroxypropane) ) having $pK_a$ of 7.52 at 25°C and pH in the range of about 7-8.2; TAPSO (2-hydroxy-3{tris(hydroxymethyl)methylamino}-1-propanesulfonic acid)) having $pK_a$ of 7.61 at 25°C and pH in the range of about 7-8.2; TAPS ({(2-hydroxy-1,1-bis(hydroxymethyl)ethyl)amino}-1-propanesulfonic acid) ) having $pK_a$ of 8.4 at 25°C and pH in the range of about 7.7-9.1; TABS (N-tris(hydroxymethyl)methyl-4-aminobutanesulfonic acid) having $pK_a$ of 8.9 at 25°C and pH in the range of about 8.2-9.6; AMPSO (N-(1,1-dimethyl-2-hydroxyethyl)-3-amino-2-hydroxypropanesulfonic acid)) having $pK_a$ of 9.0 at 25°C and pH in the range of about 8.3-9.7; CHES (2-cyclohexylamino)ethanesulfonic acid) having $pK_a$ of 9.5 at 25°C and pH in the range of about 8.6-10.0; CAPSO (3-(cyclohexylamino)-2-hydroxy-1-propanesulfonic acid) having $pK_a$ of 9.6 at 25°C and pH in the range of about 8.9-10.3; or CAPS (3-(cyclohexylamino)-1-propane sulfonic acid) having $pK_a$ of 10.4 at 25°C and pH in the range of about 9.7-11.1.

In certain embodiments, a composition of the present invention is formulated in a buffer having a slight acidic pH, such as from about 6 to about 6.8. In such embodiments, the buffer capacity of the composition desirably allows the composition to come rapidly to a physiological pH after being administered to into the patient.

Reference EXAMPLE 1

Two mixtures I and II are made separately by mixing the ingredients listed in Table 1. Five parts (by weight) of

mixture I are mixed with twenty parts (by weight) of mixture II for 15 minutes or more. The pH of the combined mixture is adjusted to 6.2-6.4 using 1 N NaOH.

Table 1

| Ingredient | Amount |
|---|---|
| Mixture I | |
| Macugen® | 0.2g |
| Carbopol 934P NF | 0.25 g |
| Purified water | 99.55 g |
| Mixture II | |
| Propylene glycol | 5 g |
| EDTA | 0.1 mg |
| Compound of Formula IV | 50 g |

Reference EXAMPLE 2:

Two mixtures I and II are made separately by mixing the ingredients listed in Table 2. Five parts (by weight) of mixture I are mixed with twenty parts (by weight) of mixture II for 15 minutes or more. The pH of the combined mixture is adjusted to 6.2-6.4 using 1 N NaOH.

Table 2

| Ingredient | Amount |
|---|---|
| Mixture I | |
| Macugen® | 0.2g |
| 4-(2-phenyl-1H-imidazol-1-yl)-N-pyridin-4-ylpyrimidin-2-amine (a tyrosine kinase inhibitor) | 0.3g |
| Carbopol 934P NF | 0.25 g |
| Purified water | 99.25 g |
| Mixture II | |
| Propylene glycol | 5 g |
| EDTA | 0.1 mg |
| Compound of Formula IV | 50 g |

Reference EXAMPLE 3:

Two mixtures I and II are made separately by mixing the ingredients listed in Table 3. Five parts (by weight) of mixture I are mixed with twenty parts (by weight) of mixture II for 15 minutes or more. The pH of the combined mixture is adjusted to 6.2-6.4 using 1 N NaOH.

Table 3

| Ingredient | Amount |
|---|---|
| Mixture I | |
| Lucentis® | 0.2g |
| Carbopol 934P NF | 0.25 g |
| Purified water | 99.55 g |

(continued)

| | |
|---|---|
| Mixture II | |
| Propylene glycol | 3 g |
| Triacetin | 7g |
| Compound of Formula II | 50 g |
| EDTA | 0.1 mg |

Reference EXAMPLE 4:

Two mixtures I and II are made separately by mixing the ingredients listed in Table 4. Five parts (by weight) of mixture I are mixed with twenty parts (by weight) of mixture II for 15 minutes or more. The pH of the combined mixture is adjusted to 6.2-6.4 using 1 N NaOH.

Table 4

| Ingredient | Amount |
|---|---|
| Mixture I | |
| Lucentis® | 0.3g |
| N-isoxazol-3-yl-4-(2-phenyl-1H-imidazol-1-yl)pyrimidin-2-amine (a tyrosine kinase inhibitor) | 0.3g |
| Carbopol 934P NF | 0.25 g |
| Olive oil | 99.15 g |
| Mixture II | |
| Propylene glycol | 7 g |
| Glycerin | 3 g |
| Compound of Formula III | 50 g |
| Cyclosporine A | 5 g |
| HAP (30%) | 0.5 mg |
| Alexidine 2HCl | 1-2 ppm |
| Note: "HAP" denotes hydroxyalkyl phosphonates, such as those known under the trade name Dequest®. | |

Reference EXAMPLE 5:

The ingredients listed in Table 5 are mixed together for at least 15 minutes. The pH of the mixture is adjusted to 6.2-6.4 using 1 N NaOH.

Table 5

| Ingredient | Amount (% by weight) |
|---|---|
| Povidone | 1 |
| HAP (30%) | 0.05 |
| Glycerin | 3 |
| Propylene glycol | 3 |
| Compound of Formula IV | 0.5 |
| Lucentis® | 0.1 |
| Tyloxapol | 0.25 |
| BAK | 10-100 ppm |

(continued)

| Ingredient | Amount (% by weight) |
|---|---|
| Purified water | q.s. to 100 |
| Note: "BAK" denotes benzalkonium chloride. | |

Reference EXAMPLE 6:

The ingredients listed in Table 6 are mixed together for at least 15 minutes. The pH of the mixture is adjusted to 6.2-6.4 using 1 N NaOH.

Table 6

| Ingredient | Amount (% by weight) |
|---|---|
| Povidone | 1.5 |
| HAP (30%) | 0.05 |
| Glycerin | 3 |
| Propylene glycol | 3 |
| Compound of Formula IV | 0.75 |
| VEGF ribozyme | 0.1 |
| Tyloxapol | 0.25 |
| Alexidine 2HCl | 1-2 ppm |
| Purified water | q.s. to 100 |

Reference EXAMPLE 7:

The ingredients listed in Table 7 are mixed together for at least 15 minutes. The pH of the mixture is adjusted to 6.2-6.4 using 1 N NaOH.

Table 7

| Ingredient | Amount (% by weight) |
|---|---|
| CMC (MV) | 0.5 |
| HAP (30%) | 0.05 |
| Glycerin | 3 |
| Propylene glycol | 3 |
| Compound of Formula IV | 0.75 |
| Lucentis® | 0.1 |
| N-(3-methylisoxazol-5-yl)-4-(2-phenyl-1H-imidazol-1-yl)pyrimidin-2-amine (a tyrosine kinase inhibitor) | 0.3 |
| Tyloxapol (a surfactant) | 0.25 |
| Alexidine 2HCl | 1-2 ppm |
| Sunflower oil | q.s. to 100 |

Reference EXAMPLE 8:

The ingredients listed in Table 8 are mixed together for at least 15 minutes. The pH of the mixture is adjusted to 6.2-6.4 using 1 N NaOH.

Table 8

| Ingredient | Amount (% by weight) |
|---|---|
| CMC (MV) | 0.5 |
| HAP (30%) | 0.05 |
| Glycerin | 3 |
| Propylene glycol | 3 |
| Compound of Formula IV | 0.75 |
| dsRNA having sequence corresponding to single-stranded VEGF mRNA | 0.2 |
| N-(3-methylisoxazol-5-yl)-4-(2-phenyl-1H-imidazol-1-yl)pyrimidin-2-amine (a tyrosine kinase inhibitor) | 0.3 |
| Tyloxapol (a surfactant) | 0.25 |
| Alexidine 2HCl | 1-2 ppm |
| Purified water | q.s. to 100 |

Reference EXAMPLE 9:

The ingredients listed in Table 9 are mixed together for at least 15 minutes. The pH of the mixture is adjusted to 6.2-6.4 using 1 N NaOH.

Table 9

| Ingredient | Amount (% by weight) |
|---|---|
| CMC (MV) | 0.5 |
| HAP (30%) | 0.05 |
| Glycerin | 3 |
| Propylene glycol | 3 |
| Compound of Formula IV | 0.75 |
| Lucentis® | 0.2 |
| dsRNA having sequence corresponding to single-stranded VEGF mRNA | 0.2 |
| N-(3-methylisoxazol-5-yl)-4-(2-phenyl-1H-imidazol-1-yl)pyrimidin-2-amine (a tyrosine kinase inhibitor) | 0.3 |
| Tyloxapol (a surfactant) | 0.25 |
| Alexidine 2HCl | 1-2 ppm |
| Corn oil | q.s. to 100 |

Reference EXAMPLE 10:

The ingredients listed in Table 10 are mixed together for at least 15 minutes. The pH of the mixture is adjusted to 6.2-6.4 using 1 N NaOH.

Table 10

| Ingredient | Amount (% by weight) |
|---|---|
| CMC (MV) | 0.5 |
| HAP (30%) | 0.05 |
| Glycerin | 3 |

(continued)

| Ingredient | Amount (% by weight) |
|---|---|
| Propylene glycol | 3 |
| Compound of Formula IV | 0.75 |
| Macugen® | 0.2 |
| polypeptide antibody against VEGFR-2 | 0.3 |
| N-(3-methylisoxazol-5-yl)-4-(2-phenyl-1H-imidazol-1-yl)pyrimidin-2-amine (a tyrosine kinase inhibitor) | 0.3 |
| Tyloxapol (a surfactant) | 0.25 |
| Alexidine 2HCl | 1-2 ppm |
| Purified water | q.s. to 100 |

In another aspect, a DIGRA or a pharmaceutically acceptable salt thereof, and an anti-inflammatory agent are incorporated into a formulation for topical administration, systemic administration, periocular injection, or intravitreal injection. An injectable intravitreal formulation can desirably comprise a carrier that provides a sustained-release of the active ingredients, such as for a period longer than about 1 week (or longer than about 1, 2, 3, 4, 5, or 6 months). In certain embodiments, the sustained-release formulation desirably comprises a carrier that is insoluble or only sparingly soluble in the vitreous. Such a carrier can be an oil-based liquid, emulsion, gel, or semisolid. Examples of oil-based liquids include castor oil, peanut oil, olive oil, coconut oil, sesame oil, cottonseed oil, corn oil, sunflower oil, fish-liver oil, arachis oil, and liquid paraffin.

In another aspect of the present disclosure, the compound or composition can be injected intravitreally, for example through the pars plana of the ciliary body, to treat or prevent glaucoma or progression thereof using a fine-gauge needle, such as 25-30 gauge. Typically, an amount from about 25 μl to about 100 μl of a composition comprising a DIGRA, a prodrug thereof, or a pharmaceutically acceptable salt thereof is administered into a patient. A concentration of such DIGRA, prodrug thereof, or pharmaceutically acceptable salt thereof is selected from the ranges disclosed above.

In another aspect, a DIGRA, or a pharmaceutically acceptable salt thereof is incorporated into an ophthalmic device that comprises a biodegradable material, and the device is implanted into a subject to provide a long-term (e.g., longer than about 1 week, or longer than about 1, 2, 3, 4, 5, or 6 months) treatment of the chronic inflammatory condition. Such a device may be implanted by a skilled physician in the subject's ocular or periocular tissue.

In still another aspect of the present disclosure, the composition is for use in a method for treating, reducing, ameliorating, or alleviating a back-of-the-eye condition or disorder, which has an etiology in inflammation, wherein the composition comprises a DIGRA, a prodrug thereof, or a pharmaceutically acceptable salt thereof; and is to be administered to a subject in an amount at a frequency sufficient to treat, reduce, ameliorate, or alleviate the condition or disorder in the subject.

In one embodiment, the DIGRA is selected from among those disclosed above.

In another embodiment, such inflammation is a chronic inflammation.

In still another embodiment, such as condition or disorder is selected from the group consisting of DR. AMD, DME, posterior uveitis, and combinations thereof.

In another embodiment of the present disclosure, the composition further comprises: (i) an anti-inflammatory agent other than a DIGRA, a prodrug thereof, and a pharmaceutically acceptable thereof; (ii) an anti-angiogenic agent; or (iii) a combination thereof. Such an anti-inflammatory agent or anti-angiogenic agent is selected from among those disclosed above. The concentration of the DIGRA, a prodrug thereof, a pharmaceutically acceptable salt thereof, the anti-inflammatory agent or anti-angiogenic agent is selected from among the ranges disclosed above.

In another aspect of the present disclosure, a DIGRA, a prodrug thereof, or a pharmaceutically acceptable salt thereof, with or without an additional anti-inflamatory agent and/or an anti-angiogenic agent, is incorporated into a formulation for topical administration, systemic administration, periocular injection, or intravitreal injection. An injectable intravitreal formulation can desirably comprise a carrier that provides a sustained-release of the active ingredients, such as for a period longer than about 1 week (or longer than about 1, 2, 3, 4, 5, or 6 months).

In still another aspect, a DIGRA, or a pharmaceutically acceptable salt thereof is incorporated into an ophthalmic device that comprises a biodegradable material, and the device is implanted into a subject to provide a long-term (e.g., longer than about 1 week, or longer than about 1, 2, 3, 4, 5, or 6 months) treatment of a back-of-the-eye disease. Such a device may be implanted by a skilled physician in the back of the eye of the patient for the sustained release of the active ingredient or ingredients. A typical implant system or device suitable for use in a method of the present invention

comprises a biodegradable matrix with the active ingredient or ingredients impregnated or dispersed therein. Examples of ophthalmic implant systems or devices for the sustained-release of an active ingredient are disclosed in U.S. Patents 5,378,475; 5,773,019; 5,902,598; 6,001,386; 6,051,576; and 6,726,918.

In yet another aspect, a composition of the present invention is administered once a week, once a month, once a year, twice a year, four times a year, or at a suitable frequency that is determined to be appropriate for treating, reducing, ameliorating, or alleviating the condition or disorder.

Also disclosed is a composition for use in a method for treating, reducing, ameliorating, or alleviating a back-of-the-eye condition or disorder that has an etiology in inflammation (in particular, chronic inflammation). The following steps are to be applied: (a) an amount of a composition comprising a DIGRA, a prodrug thereof, or a pharmaceutically acceptable salt thereof is to be administered to a subject at a first frequency sufficient to treat, reduce, ameliorate, or alleviate the condition or disorder in the subject; and (b) a procedure selected from the group consisting of protocoagulation, photodynamic therapy, and a combination thereof is to be prepared in the subject at a second frequency sufficient to treat, reduce, ameliorate, or alleviate the condition or disorder in the subject. In one embodiment, the composition further comprises an anti-inflammatory agent other than a DIGRA, an anti-angiogenic agent, or a combination thereof. Examples of these materials are disclosed herein above.

In one embodiment, the first frequency and the second frequency are the same. In another embodiment, the first frequency and the second frequency are different. In still another embodiment, said administering and said performing are carried out sequentially. In yet another embodiment, said performing is carried out before said administering. In a further embodiment, said performing is carried out after said administering. The first frequency and the second frequency can be, for example, once a week, once a month, once a year, twice a year, four times a year, or other frequencies, said first frequency and second frequency being chosen as deemed appropriate for the condition and treatment objective.

In photocoagulation therapy, high-energy light from a laser is directed to the leaky vasculature to coagulate the fluid in and around the new leaky vessels, relying on the transfer of thermal energy generated by the laser to the pathological tissue. Photocoagulation systems are currently available.

In photodynamic therapy ("PDT"), a photosensitizer (light-activated drug) is administered into the patient, typically via the intravenous route followed by application of light of appropriate wavelength directed at the pathological tissue, such as the leaky vasculature. The light sources most commonly used are non-thermal lasers or light-emitting diodes ("LEDs"). After exposure to light at a wavelength absorbed by the photosensitizer, an energy transfer cascade is initiated, culminating in the formation of reactive oxygen, which generates free radicals. These free radicals, in turn, disrupt cellular structures or functions, leading to death of endothelial cells and, thus, prevention of further neovascularization. Examples of photosensitizers include those disclosed in U.S. Patents 7,015,240 and 7,060,695.

COMPARISON OF GLUCOCORTICOIDS AND DIGRAS

One of the most frequent undesirable actions of a glucocorticoid therapy is steroid diabetes. The reason for this undesirable condition is the stimulation of gluconeogenesis in the liver by the induction of the transcription of hepatic enzymes involved in gluconeogenesis and metabolism of free amino acids that are produced from the degradation of proteins (catabolic action of glucocorticoids). A key enzyme of the catabolic metabolism in the liver is the tyrosine aminotransferase ("TAT"). The activity of this enzyme can be determined photometrically from cell cultures of treated rat hepatoma cells. Thus, the gluconeogenesis by a glucocorticoid can be compared to that of a DIGRA by measuring the activity of this enzyme. For example, in one procedure, the cells are treated for 24 hours with the test substance (a DIGRA or glucocorticoid), and then the TAT activity is measured. The TAT activities for the selected DIGRA and glucocorticoid are then compared. Other hepatic enzymes can be used in place of TAT, such as phosphoenolpyruvate carboxykinase, glucose-6-phosphatase, or fructose-2,6-biphosphatase. Alternatively, the levels of blood glucose in an animal model may be measured directly and compared for individual subjects that are treated with a glucocorticoid for a selected condition and those that are treated with a DIGRA for the same condition.

Another undesirable result of glucocorticoid therapy is GC-induced cataract. The cataractogenic potential of a compound or composition may be determined by quantifying the effect of the compound or composition on the flux of potassium ions through the membrane of lens cells (such as mammalian lens epithelial cells) in vitro. Such an ion flux may be determined by, for example, electrophysiological techniques or ion-flux imaging techniques (such as with the use of fluorescent dyes). An exemplary in-vitro method for determining the cataractogenic potential of a compound or composition is disclosed in U.S. Patent Application Publication 2004/0219512.

Still another undesirable result of glucocorticoid therapy is hypertension. Blood pressure of similarly matched subjects treated with glucocorticoid and DIGRA for an inflammatory condition may be measured directly and compared.

Yet another undesirable result of glucocorticoid therapy is increased IOP. IOP of similarly matched subjects treated with glucocorticoid and DIGRA for an inflammatory condition may be measured directly and compared.

TESTING: Comparison of the DIGRA Having Formula IV With Two Corticosteroids and One NSAID in Treating Inflammation

I . INTRODUCTION

Inflammatory processes are multidimensional in origin, and are characterized by complex cellular and molecular events involving numerous components all of which have not been identified. Prostaglandins are among these mediators and play an important role in certain forms of ocular inflammation. Paracentesis of the anterior chamber in the rabbit eye induces inflammatory reaction due to the disruption of the blood-aqueous barrier ("BAB"), which is mediated, at least in part, by prostaglandin $E_2$ [References 1 -3 below]. Intraocular or topical administration of $PGE_2$ disrupts the BAB. [Reference 4, below] The treatment schedule adopted in this study was similar to the clinical NSAIDs (Ocufen) treatment schedule used by surgeons for patients before cataract surgery. We investigated a dissociated glucocorticoid receptor agonist ("BOL-303242-X", compound having Formula IV above) at different doses on rabbit paracentesis model evaluating aqueous biomarkers levels, and iris-ciliary body MPO activity in comparison with vehicle, dexamethasone, loteprednol and flurbiprofen.

2. METHODS

2.1 Drugs and Materials

2.1.1. Test articles

BOL-303242-X (0.1%. 0.5% and 1% topical formulations), lot 2676-MLC-107, Bauch & Lomb Incorporated ("B&L") Rochester, USA.

Vehicle (10% PEG 3350; 1% Tween 80; phosphate buffer pH 7.00), lot 2676-MLC-107, B&L Rochester, USA.

Visumetazone® (0.1% Dexamethasone topical formulation), lot T253, Visufarma, Rome, Italy.

Lotemax® (0.5% Loteprednol topical formulation), lot 078061, B&L IOM, Macherio, Italy.

Ocufen® (0.03% Flurbiprofen topical formulation), lot E45324, Allergan, Westport, Ireland.

2.2 Animals

Species: Rabbit

Breed: New Zealand

Source: Morini (Reggio Emila, Italy)

Sex: Male

Age at Experimental Start: 10 weeks.

Weight Range at Experimental Start: 2.0-2.4 Kg

Total Number of Animals: 28

Identification: Ear tagged with an alphanumeric code (i.e. A1 means test article A and animal 1).

Justification: The rabbit is a standard non-rodent species used in pharmacodynamic studies. The number of animals used in this study is, in judgment of the investigators involved, the minimum number necessary to properly perform this type of study and it is consistent with world wide regulatory guidelines.

Acclimation/Quarantine: Following arrival, a member of the veterinary staff assessed animals as to their general health. Seven days elapsed between animal receipt and the start of experiment in order to acclimate animals to the laboratory environment and to observe them for the development of infection disease.

Animal Husbandry: All the animals were housed in a cleaned and disinfected room, with a constant temperature (22±1 °C), humidity (relative, 30%) and under a constant light-dark cycle (light on between 8.00 and 20.00). Commercial food and tap water were available *ad libitum*. Their body weights were measured just before the experiment (Table T-1). All the animals had a body weight inside the central part of the body weight distribution curve (10%).

Four rabbits were replaced with animals of similar age and weight from the same vendor because three of them showed signs of ocular inflammation and one was dead upon arrival.

Animals Welfare Provisions: All experiments were carried out according to the ARVO (Association for Research in Vision and Ophthalmology) guidelines on the use of animals in research. No alternative test system exists which have been adequately validated to permit replacement of the use of live animals in this study. Every effort has been made to obtain the maximum amount of information while reducing to a minimum the number of animals required for this study. To the best of our knowledge, this study is not unnecessary or duplicative. The study protocol was reviewed and approved by the Institutional Animal Care and Use Committee (IACUC) of the University of Catania and complies with the acceptable standards of animal welfare care.

2.3 Experimental Preparations

2.3.1 Study design and randomization

Twenty-eight rabbits were randomly allocated into 7 groups (4 animals/each) as shown in the table below.

Table 8

| Group | No of rabbits | Treatment | | Observations and measurements | Termination and assays |
|---|---|---|---|---|---|
| I | 4 | CTR | 50 μl drops at 180, 120, 90, and 30 min prior to first paracentesis, and at 15, 30, 90 min after the first paracentesis. | Clinical observations and pupillary diameter at 180 and 5 min before the first paracentesis, and at 5 min before the second paracentesis.<br>Paracentesis at 0 and 2 hours. | Termination immediately after the second paracentesis.<br>Aqueous humor collected for $PGE_2$, protein, leukocytes and $LTB_4$ measurements.<br>Iris-ciliary body collected for MPO activity measurement. |
| II | 4 | 1% BOL | | | |
| III | 4 | 0.5% BOL | | | |
| IV | 4 | 0.1% BOL | | | |
| V | 4 | 0.5% LE | | | |
| VI | 4 | 0.1% Dex | | | |
| VII | 4 | 0.03% F | | | |
| CTR=vehicle; BOL=BOL-303242-X; LE=loteprednol etabonate; Dex=dexamethasone; F=flurbiprofen | | | | | |

To each test article was randomly assigned a letter from A to G

A = vehicle (10% PEG3350/1% Tween 80/PB pH 7.00)

B = Ocufen (Fluorbiprofen 0.03%)

C = Visumetazone (Desmethasone 0.1%)

D = Lotemax (Loetprednol etabonate 0.5%)

E = BOL-303242-X 0.1% (1 mg/g)

F = BOL-303242-X 0.5% (5 mg/g)

G = BOL-303242-X 1% (10 mg/g)

2.3.2 Reagent preparation for MPO assay

2.3.2.1 Phosphate buffer (50mM; pH=6)

3.9g of $NaH_2PO_4$ $2H_2O$ were dissolved in a volumetric flask to 500ml with water. The pH was adjusted to pH=6

with 3N NaOH.

2.3.2.2 Hexa-decyl-trimethyl-ammonium bromide (0.5%)

0.5g of hexa-decyl-trimethyl-ammonium bromide was dissolved in 100ml phosphate buffer.

2.3.2.3 o-dianisidine 2HCl (0.0167%) / $H_2O_2$ (0.0005%) solution

The solution was prepared freshly. Ten microliters of $H_2O_2$ (30 wt.%) were diluted to 1ml with water (solution A). 7.5mg o-dianisidine 2HCl were dissolved in 45ml of phosphate buffer and 75μl of solution A were added.

2.4 Experimental Protocols

2.4.1 Animals treatment and sample collection

Each rabbit was placed in a restraint device and tagged with the alphanumeric code. The formulations were instilled (50 μl) into the conjunctival sac of both eyes 180, 120, 90 and 30 min before the first paracentesis; then 15, 30, 90 min after the first paracentesis. To perform the first paracentesis the animals were anaesthetized by intraveneous injection of 5mg/kg Zoletil® (Virbac; 2.5mg/kg tiletamine HCl and 2.5mg/kg zolazepam HCl) and one drop of local anesthetic (Novesina®, Novartis) was administered to the eye. Anterior chamber paracentesis was performed with a 26 G needle attached to a tuberculin syringe; the needle was introduced into the anterior chamber through the cornea, taking care not to damage the tissues. Two hours after the first paracentesis, the animals were sacrificed with 0.4 ml Tanax® (Intervet International B.V.) and the second paracentesis was performed. About 100 μl of aqueous humor were removed at the second paracentesis. Aqueous humor was immediately split in four aliquots and stored at -80 °C until analysis. Then both eyes were enucleated and the iris-ciliary body was carefully excised, placed in polypropylene tubes, and stored at -80 °C until analysis.

2.4.2 Pupillary diameter measurement

The pupillary diameter of both eyes was measured with a Castroviejo caliper 180 min and 5 min before the first paracentesis and 5 min before the second paracentesis.

2.4.3 Clinical evaluation

The clinical evaluation of both eyes was performed by a slit lamp (4179-T; Sbisa, Italy) at 180 min and 5 min before the first paracentesis and 5 min before the second paracentesis. The clinical score was assigned according to the following scheme:

0 = normal
1 = discrete dilatation of iris and conjunctival vessels
2 = moderate dilatation of iris and conjunctival vessels
3 = intense iridal hyperemia with flare in the anterior chamber
4 = intense iridal hyperemia with flare in the anterior chamber and presence of fibrinous exudates.

2.4.4 Prostaglandin $E_2$ ($PGE_2$) measurement

For the quantitative determination of $PGE_2$ in the aqueous humor we used the $PGE_2$ Immunoassay kit (R&D Systems; Cat.No. KGE004; Lot.No. 240010). Eleven microliters or 16μl of aqueous humor were diluted to 110μl or 160μl with the calibrator diluent solution provided with the kit. One hundred microliters of samples and of standards were load into a 96-well plate and recorded in a plate layout. Samples were treated following the assay procedure described in the kit. A microplate reader (GDV, Italy; model DV 990 B/V6) set at 450 nm (wavelength correction at 540 nm) was used for making the calibration and analyzing the samples.

2.4.5 Protein measurement

For protein concentration determination in the aqueous humor we used the Protein Quantification Kit (Fluka; Cat.No. 77371; Lot.No. 1303129). Five microliters of aqueous humor were diluted to 100μl with water. Twenty microliters of samples and of standards were load into a 96-well plate and recorded in a plate layout. Samples were treated following

the assay procedure described in the kit. A microplate reader (GDV, Italy; model DV 990 B/V6) set at 670 nm was used for making the calibration and analyzing the samples.

2.4.6 Leukocytes (PMN) measurement

For the determination of the number of leukocytes we used a haemocytometer (Improved Neubauer Chamber; Brigth-line, Hausser Scientific) and a Polyvar 2 microscope (Reichert-Jung).

2.4.7 Leucotriene $B_4$ ($LTB_4$) measurement

For the quantitative determination of $LTB_4$ concentration in the aqueous humor we used the $LTB_4$ Immunoassay kit (R&D Systems; Cat.No. KGE006; Lot.No. 243623). 11μl of aqueous humor were diluted to 110μl with the calibrator diluent solution provided with the kit. 100 μl of samples and of standards were load into a 96-well plate and recorded in a plate layout. Samples were treated following the assay procedure described in the kit. A microplate reader (GDV, Italy; model DV 990 B/V6) set at 450 nm (wavelength correction at 540 nm) was used for making the calibration and analyzing the samples.

2.4.8 Myeloperoxidase (MPO) measurement

The activity of MPO was measured as previously described by Williams et al.[5] The iris-ciliary bodies were carefully dried, weighed and immersed in 1ml of hexa-decyl-trimethyl-ammonium bromide solution. Then, the samples were sonicated for 10 sec on ice by a ultrasound homogenizer (HD 2070, Bandelin electronic), freeze-thawed three times, sonicated for 10 sec and centrifuged at 14,000 g for 10 min to remove cellular debris. An aliquot of the supernatant (40-200μl) was diluted to 3ml with the *o*-dianisidine 2HCl / $H_2O_2$ solution. The change in absorbance at 460nm was continuously monitored for 5 min by a spectrophotometer (UV/Vis Spectrometer Lambda EZ 201; Perkin Elmer). The slope of the line (Δ/min) was determined for each sample and used to calculate the number of units of MPO in the tissue as follows:

$$MPOunit/g = \frac{(\Delta/\min)\cdot 10^{6}}{\varepsilon \cdot \mu l \cdot mg}$$

were $\varepsilon = 11.3\ mM^{-1}$.
Values were expressed as units of MPO/g of tissue.

2.5 Data Analysis

Pupillary diameter, $PGE_2$, protein, PMN, and MPO were expressed as mean ± SEM. Statistical analysis was performed using one way ANOVA followed by a Newman-Keuls post hoc test. Clinical score was expressed as % of eyes and the statistical analysis was performed using Kruskal-Wallis followed by a Dunn post hoc test. P<0.05 was considered statistically significant in both cases. Prism 4 software (GraphPad Software, Inc.) was used for the analysis and graphs.

3. RESULTS

3.1 Pupillary diameter measurement

The raw data are displayed in Tables T-2 and T-3. No statistical significance was found between the CRT and all the treatments.

3.2 Clinical evaluation

The raw data are displayed in Tables T-4 and T-5. Only the 0.5% LE group showed a significant difference vesus CTR (p<0.05).

3.3 Prostaglandin $E_2$ ($PGE_2$) measurement

The raw data are displayed in Tables T-6 and T-7. The treatments 0.03% F, 0.5% LE, 0.1% BOL, and 0.5% BOL were statistically significant versus CTR (p<0.05).

3.4 Protein measurement

The raw data are displayed in Tables T-8 and T-9. It has been found a statistical significance for the treatments 0.03% F and 1% BOL vs CTR with p<0.001, and 0.5% BOL vs CTR with p<0.05.

3.5 Leukocytes (PMN) measurement

The raw data are displayed in Tables T-10 and T-11. All the treatments were statistically significant vs CTR (p<0.001).

3.6 Leucotriene $B_4$ ($LTB_4$) measurement

All samples were under the limit of quantification (about 0.2 ng/ml) of the assay.

3.7 Myeloperoxidase (MPO) measurement

The raw data are displayed in Tables T-12 and T-13. It has been found a statistical significance for the all the treatments vs CTR with p<0.01 for 0.03% F, and p<0.001 for 0. 1% Dex, 0.5% LE, 0.1% BOL, 0.5% BOL and 1% BOL.

4. DISCUSSION

The preliminary conclusions from the data generated are:

- BOL-303242-X is active in this model.
- There was not a large difference between these concentrations of BOL-303242-X and NSAID and steroid positive controls.

There was not a profound dose-response for BOL-303242-X, perhaps because we are at either maximal efficacy or maximal drug exposure at these doses. However, the results show that BOL-303242-X is as effective an anti-inflammatory drug as some of the commonly accepted prior-art steroids or NSAID. Some other very preliminary data (not shown) suggest that BOL-303242-X does not have some of the side effects of corticosteroids.

5. REFERENCES

1. Eakins KE (1977). Prostaglandin and non prostaglandin-mediated breakdown of the blood-aqueous barrier. Exp Eye Res, 25, 483-498.
2. Neufeld AH, Sears ML (1973). The site of action of prostaglandin E2 on the disruption of the blood-aqueous barrier in the rabbit eye. Exp Eye Res, 17, 445-448.
3. Unger WG, Cole DP, Hammond B (1975). Disruption of the blood-aqueous barrier following paracentesis in the rabbit. Exp Eye Res, 20, 255-270.
4. Stjemschantz J (1984). Autacoids and Neuropeptides. In: Sears, ML (ed) Pharmacology of the Eye. Springer-Verlag, New York, pp 311-365.
5. Williams RN, Paterson CA, Eakins KE, Bhattacherjee P (1983) Quantification of ocular inflammation: evaluation of polymorphonuclear leukocyte infiltration by measuring myeloperoxidase activity. Curr Eye Res 2:465-469.

Table T-1: Rabbit body weight measured just before the experiment

| Rabbit ID | Sex | Body weight (g) |
|---|---|---|
| A1 | M | 2090 |
| A2 | M | 2140 |

(continued)

| Rabbit ID | Sex | Body weight (g) |
|---|---|---|
| A3 | M | 2100 |
| A4 | M | 2320 |
| B1 | M | 2270 |
| B2 | M | 2190 |
| B3 | M | 2340 |
| B4 | M | 2300 |
| C1 | M | 2160 |
| C2 | M | 2160 |
| C3 | M | 2280 |
| C4 | M | 2400 |
| D1 | M | 2220 |
| D2 | M | 2200 |
| D3 | M | 2180 |
| D4 | M | 2260 |
| E1 | M | 2170 |
| E2 | M | 2330 |
| E3 | M | 2350 |
| E4 | M | 2300 |
| F1 | M | 2190 |
| F2 | M | 2240 |
| F3 | M | 2120 |
| F4 | M | 2200 |
| G1 | M | 2410 |
| G2 | M | 2270 |
| G3 | M | 2310 |
| G4 | M | 2130 |
| Mean ± S.D. | | 2236.8 ± 89.2 |

Table T-2 Raw data of pupillary diameter at -180 min (basal), -5 min (5 min before the first paracentesis) and at +115 min (5 min before the second paracentesis), and calculated difference between the value at +115 min and the value at -180 min.

| Treatment | Rabbit ID | Eye | Diameter (mm) | | | |
|---|---|---|---|---|---|---|
| | | | T1: -180 min | T2: -5 min | T3: +115 min | Δ(T3 - T1) |
| CTR | A1 | DX | 6.0 | 5.5 | 4.0 | -2.0 |
| | | SX | 5.5 | 5.5 | 4.0 | -1.5 |
| | A2 | DX | 6.0 | 6.5 | 4.5 | -1.5 |
| | | SX | 6.0 | 6.5 | 5.0 | -1.0 |
| | A3 | DX | 6.5 | 6.5 | 5.0 | -1.5 |
| | | SX | 6.5 | 6.5 | 5.0 | -1.5 |
| | A4 | DX | 6.0 | 6.5 | 5.0 | -1.0 |
| | | SX | 6.0 | 6.5 | 5.0 | -1.0 |
| | | | | | | |

(continued)

| Treatment | Rabbit ID | Eye | Diameter (mm) | | | |
|---|---|---|---|---|---|---|
| | | | T1: -180 min | T2: -5 min | T3: +115 min | Δ(T3 - T1) |
| 0.03% F | B1 | DX | 5.0 | 6.0 | 4.0 | -1.0 |
| | | SX | 5.0 | 6.0 | 3.5 | -1.5 |
| | B2 | DX | 7.0 | 6.5 | 5.5 | -1.5 |
| | | SX | 6.0 | 7.0 | 5.0 | -1.0 |
| | B3 | DX | 6.0 | 6.5 | 4.5 | -1.5 |
| | | SX | 6.0 | 6.5 | 6.0 | 0.0 |
| | B4 | DX | 5.5 | 6.0 | 5.5 | 0.0 |
| | | SX | 6.0 | 5.5 | 5.0 | -1.0 |
| | | | | | | |
| 0.1% Dex | C1 | DX | 6.0 | 5.5 | 5.5 | -0.5 |
| | | SX | 7.0 | 6.5 | 5.5 | -1.5 |
| | C2 | DX | 5.5 | 6.5 | 6.0 | 0.5 |
| | | SX | 5.5 | 6.0 | 5.5 | 0.0 |
| | C3 | DX | 6.5 | 6.0 | 4.5 | -2.0 |
| | | SX | 6.5 | 6.5 | 5.0 | -1.5 |
| | C4 | DX | 6.5 | 7.0 | 6.0 | -0.5 |
| | | SX | 7.0 | 7.5 | 6.5 | -0.5 |
| | | | | | | |
| 0.5% LE | D1 | DX | 6.0 | 6.0 | 4.5 | -1.5 |
| | | SX | 6.0 | 6.0 | 5.0 | -1.0 |
| | D2 | DX | 6.5 | 6.5 | 5.5 | -1.0 |
| | | SX | 6.5 | 6.5 | 5.5 | -1.0 |
| | D3 | DX | 6.0 | 6.0 | 6.0 | 0.0 |
| | | SX | 6.5 | 6.5 | 6.0 | -0.5 |
| | D4 | DX | 6.5 | 6.5 | 6.0 | -0.5 |
| | | SX | 6.5 | 6.5 | 5.0 | -1.5 |
| | | | | | | |
| 0.1% BOL | E1 | DX | 6.5 | 6.5 | 5.0 | -1.5 |
| | | SX | 6.5 | 6.5 | 6.0 | -0.5 |
| | E2 | DX | 6.5 | 7.0 | 5.0 | -1.5 |
| | | SX | 6.5 | 7.0 | 6.0 | -0.5 |
| | E3 | DX | 7.0 | 7.0 | 6.0 | -1.0 |
| | | SX | 7.5 | 7.5 | 6.5 | -1.0 |
| | E4 | DX | 7.0 | 6.5 | 5.5 | -1.5 |
| | | SX | 7.0 | 7.0 | 5.5 | -1.5 |
| | | | | | | |

(continued)

| Treatment | Rabbit ID | Eye | Diameter (mm) | | | |
|---|---|---|---|---|---|---|
| | | | T1: -180 min | T2: -5 min | T3: +115 min | Δ(T3 - T1) |
| 0.5% BOL | F1 | DX | 8.0 | 8.0 | 6.5 | -1.5 |
| | | SX | 8.0 | 8.0 | 6.5 | -1.5 |
| | F2 | DX | 7.0 | 7.0 | 6.5 | -0.5 |
| | | SX | 7.0 | 7.0 | 6.0 | -1.0 |
| | F3 | DX | 7.5 | 7.5 | 7.0 | -0.5 |
| | | SX | 8.0 | 8.0 | 7.0 | -1.0 |
| | F4 | DX | 7.0 | 7.0 | 6.0 | -1.0 |
| | | SX | 7.5 | 7.0 | 6.5 | -1.0 |
| | | | | | | |
| 1% BOL | G1 | DX | 6.0 | 6.0 | 5.5 | -0.5 |
| | | SX | 6.5 | 6.5 | 5.0 | -1.5 |
| | G2 | DX | 6.0 | 6.5 | 5.0 | -1.0 |
| | | SX | 6.0 | 6.5 | 5.0 | -1.0 |
| | G3 | DX | 6.5 | 7.0 | 5.5 | -1.0 |
| | | SX | 6.5 | 7.0 | 5.0 | -1.5 |
| | G4 | DX | 6.5 | 6.5 | 6.0 | -0.5 |
| | | SX | 6.5 | 6.0 | 6.0 | -0.5 |

Table T-3 Difference between the value of pupillary diameter at T3=+1 15 min (5 min before the second paracentesis) and the value at T1=-180 min (basal) (Mean ± SEM).

| Treatment | Rabbit Group ID | Mean (mm) Δ(T3 - T1) | SEM | n |
|---|---|---|---|---|
| CTR | A | -1.4 | 0.12 | 8 |
| 0.03% F | B | -0.9 | 0.22 | 8 |
| 0.1% Dex | C | -0.8 | 0.30 | 8 |
| 0.5% LE | D | -0.9 | 0.18 | 8 |
| 0.1% BOL | E | -1.1 | 0.16 | 8 |
| 0.5% BOL | F | -1.0 | 0.13 | 8 |
| 1% BOL | G | -0.9 | 0.15 | 8 |

Table T-4 Raw data of clinical score at -180 min (basal), -5 min (5 min before the first paracentesis) and at +115 min (5 min before the second paracentesis).

| Treatment | Rabbit ID | Eye | Clinical Score | | |
|---|---|---|---|---|---|
| | | | -180 min | -5 min | +115 min |
| CTR | A1 | DX | 0 | 1 | 3 |
| | | SX | 0 | 1 | 3 |
| | A2 | DX | 0 | 0 | 2 |
| | | SX | 0 | 0 | 2 |
| | A3 | DX | 0 | 0 | 3 |
| | | SX | 0 | 0 | 3 |
| | A4 | DX | 0 | 0 | 3 |
| | | SX | 0 | 0 | 3 |
| | | | | | |
| 0.03% F | B1 | DX | 0 | 0 | 2 |
| | | SX | 0 | 0 | 2 |
| | B2 | DX | 0 | 0 | 2 |
| | | SX | 0 | 0 | 2 |
| | B3 | DX | 0 | 0 | 2 |
| | | SX | 0 | 0 | 2 |
| | B4 | DX | 0 | 0 | 2 |
| | | SX | 0 | 0 | 2 |
| | | | | | |
| 0.1% Dex | C1 | DX | 0 | 0 | 1 |
| | | SX | 0 | 0 | 1 |
| | C2 | DX | 0 | 0 | 1 |
| | | SX | 0 | 0 | 1 |
| | C3 | DX | 0 | 1 | 3 |
| | | SX | 0 | 1 | 3 |
| | C4 | DX | 0 | 0 | 1 |
| | | SX | 0 | 0 | 1 |
| | | | | | |
| 0.5% LE | D1 | DX | 0 | 0 | 2 |
| | | SX | 0 | 0 | 2 |
| | D2 | DX | 0 | 0 | 1 |
| | | SX | 0 | 0 | 1 |
| | D3 | DX | 0 | 0 | 1 |
| | | SX | 0 | 0 | 1 |
| | D4 | DX | 0 | 0 | 1 |
| | | SX | 0 | 0 | 1 |
| | | | | | |

(continued)

| Treatment | Rabbit ID | Eye | Clinical Score | | |
|---|---|---|---|---|---|
| | | | -180 min | -5 min | +115 min |
| 0.1% BOL | E1 | DX | 0 | 0 | 2 |
| | | SX | 0 | 0 | 2 |
| | E2 | DX | 0 | 0 | 2 |
| | | SX | 0 | 0 | 2 |
| | E3 | DX | 0 | 0 | 2 |
| | | SX | 0 | 0 | 2 |
| | E4 | DX | 0 | 0 | 3 |
| | | SX | 0 | 0 | 3 |
| | | | | | |
| 0.5% BOL | F1 | DX | 0 | 0 | 2 |
| | | sx | 0 | 0 | 2 |
| | F2 | DX | 0 | 0 | 1 |
| | | SX | 0 | 0 | 2 |
| | F3 | DX | 0 | 0 | 1 |
| | | SX | 0 | 0 | 1 |
| | F4 | DX | 0 | 0 | 2 |
| | | SX | 0 | 0 | 2 |
| | | | | | |
| 1% BOL | G I | DX | 0 | 0 | 2 |
| | | SX | 0 | 0 | 2 |
| | G2 | DX | 0 | 0 | 2 |
| | | SX | 0 | 0 | 2 |
| | G3 | DX | 0 | 0 | 2 |
| | | SX | 0 | 0 | 2 |
| | G4 | DX | 0 | 0 | 2 |
| | | SX | 0 | 0 | 2 |

Table T-5 Clinical score expressed as percentage of eyes at -180 min (basal), -5 min (5 min before the first paracentesis) and at +115 min (5 min before the second paracentesis).

| Treatment | Rabbit Group ID | N (eyes) | Score (%) | | | | |
|---|---|---|---|---|---|---|---|
| | | | 0 | 1 | 2 | 3 | 4 |
| -180 min | | | | | | | |
| CTR | A | 8 | 100 | - | - | - | - |
| 0.03% F | B | 8 | 100 | - | - | - | - |
| 0.1% Dex | C | 8 | 100 | - | - | - | - |
| 0.5% LE | D | 8 | 100 | - | - | - | - |
| 0.1% BOL | E | 8 | 100 | - | - | - | - |

(continued)

| | | | | | | | |
|---|---|---|---|---|---|---|---|
| -180 min | | | | | | | |
| 0.5% BOL | F | 8 | 100 | - | - | - | - |
| 1% BOL | G | 8 | 100 | - | - | - | - |
| -5 min | | | | | | | |
| CTR | A | 8 | 75 | 25 | - | - | - |
| 0.03% F | B | 8 | 100 | - | - | - | - |
| 0.1% Dex | C | 8 | 75 | 25 | - | - | - |
| 0.5% LE | D | 8 | 100 | - | - | - | - |
| 0.1% BOL | E | 8 | 100 | - | - | - | - |
| 0.5% BOL | F | 8 | 100 | - | - | - | - |
| 1 % BOL | G | 8 | 100 | - | - | - | - |
| + 115 min | | | | | | | |
| CTR | A | 8 | - | - | 25 | 75 | - |
| 0.03% F | B | 8 | - | - | 100 | - | - |
| 0.1% Dex | C | 8 | - | 75 | - | 25 | - |
| 0.5% LE | D | 8 | - | 75 | 25 | - | - |
| 0.1% BOL | E | 8 | - | - | 75 | 25 | -- |
| 0.5% BOL | F | 8 | - | 37.5 | 62.5 | - | - |
| 1% BOL | G | 8 | - | - | 100 | - | - |

Table T-6 Raw data of $PGE_2$ levels in aqueous humor samples collected at the second paracentesis

| Treatment | Sample | $PGE_2$ (ng/ml) |
|---|---|---|
| CTR | 2-A1-DX | 3.81 |
| | 2-A1-SX | 2.91 |
| | 2-A2-DX | 4.77 |
| | 2-A2-SX | [1]N/A |
| | 2-A3-DX | 1.46 |
| | 2-A3-SX | 3.00 |
| | 2-A4-DX | 1.87 |
| | 2-A4-SX | 1.88 |
| | | |
| 0.03% F | 2-B1-DX | 1.04 |
| | 2-B1-SX | 0.75 |
| | 2-B2-DX | 0.85 |
| | 2-B2-SX | 1.11 |
| | 2-B3-DX | 2.11 |
| | 2-B3-SX | 0.93 |
| | 2-B4-DX | 0.61 |
| | 2-B4-SX | 2.11 |

(continued)

| Treatment | Sample | $PGE_2$ (ng/ml) |
|---|---|---|
| | | |
| 0.1% Dex | 2-C1-DX | 2.51 |
| | 2-C1-SX | N/A |
| | 2-C2-DX | 2.32 |
| | 2-C2-SX | N/A |
| | 2-C3-DX | 2.10 |
| | 2-C3-SX | 3.03 |
| | 2-C4-DX | 2.32 |
| | 2-C4-SX | 1.30 |
| | | |
| 0.5% LE | 2-D1-DX | [2]N/D |
| | 2-D1-SX | N/D |
| | 2-D2-DX | N/D |
| | 2-D2-SX | 0.23 |
| | 2-D3-DX | N/D |
| | 2-D3-SX | 0.68 |
| | 2-D4-DX | N/D |
| | 2-D4-SX | 1.10 |
| | | |
| 0.1% BOL | 2-E1-DX | 1.62 |
| | 2-E1-SX | 1.88 |
| | 2-E2-DX | 2.15 |
| | 2-E2-SX | 0.70 |
| | 2-E3-DX | 1.34 |
| | 2-E3-SX | 1.03 |
| | 2-E4-DX | N/D |
| | 2-E4-SX | N/D |
| | | |
| 0.5% BOL | 2-F1-DX | 2.31 |
| | 2-F1-SX | 2.59 |
| | 2-F2-DX | N/D |
| | 2-F2-SX | 0.53 |
| | 2-F3-DX | 0.75 |
| | 2-F3-SX | 0.80 |
| | 2-F4-DX | 1.62 |
| | 2-F4-SX | 1.09 |
| | | |

(continued)

| Treatment | Sample | $PGE_2$ (ng/ml) |
|---|---|---|
| 1% BOL | 2-G1-DX | 0.50 |
| | 2-G1-SX | 1.87 |
| | 2-G2-DX | 1.71 |
| | 2-G2-SX | 4.04 |
| | 2-G3-DX | 1.11 |
| | 2-G3-SX | 3.78 |
| | 2-G4-DX | N/D |
| | 2-G4-SX | N/D |
| [1]N/A = not available<br>[2]N/D = not detectable, under the limit of quantification | | |

Table T-7 Levels of $PGE_2$ in aqueous humor samples collected at the second paracentesis (Mean ± SEM).

| Treatment | Sample Group | Mean (ng/ml) | SEM | n |
|---|---|---|---|---|
| CTR | A | 2.815 | 0.449 | 7 |
| 0.03% F | B | 1.189 | 0.209 | 8 |
| 0.1% Dex. | C | 2.263 | 0.232 | 6 |
| 0.5% LE | D | 0.672 | 0.250 | 3 |
| 0.1% BOL | E | 1.452 | 0.221 | 6 |
| 0.5% BOL | F | 1.384 | 0.306 | 7 |
| 1% BOL | G | 2.168 | 0.586 | 6 |

Table T-8 Raw data of protein levels in aqueous humor samples collected at the second paracentesis

| Treatment | Sample | Protein (mg/ml) |
|---|---|---|
| CTR | 2-A1-DX | 50.24 |
| | 2-A1-SX | 53.51 |
| | 2-A2-DX | 28.73 |
| | 2-A2-SX | [1]N/A |
| | 2-A3-DX | 40.09 |
| | 2-A3-SX | 30.84 |
| | 2-A4-DX | 41.79 |
| | 2-A4-SX | 30.35 |
| | | |

(continued)

| Treatment | Sample | Protein (mg/ml) |
|---|---|---|
| 0.03% F | 2-B1-DX | 20.78 |
| | 2-B1-SX | 28.80 |
| | 2-B2-DX | N/A |
| | 2-B2-SX | 23.41 |
| | 2-B3-DX | 20.21 |
| | 2-B3-SX | 17.53 |
| | 2-B4-DX | 15.12 |
| | 2-B4-SX | 20.52 |
| | | |
| 0.1% Dex | 2-C1-DX | 31.31 |
| | 2-C1-SX | N/A |
| | 2-C2-DX | 31.81 |
| | 2-C2-SX | N/A |
| | 2-C3-DX | 35.95 |
| | 2-C3-SX | 37.15 |
| | 2-C4-DX | 32.12 |
| | 2-C4-SX | 32.40 |
| | | |
| 0.5% LE | 2-D1-DX | 36.14 |
| | 2-D1-SX | 39.10 |
| | 2-D2-DX | 34.69 |
| | 2-D2-SX | 26.10 |
| | 2-D3-DX | 26.30 |
| | 2-D3-SX | 28.16 |
| | 2-D4-DX | 40.90 |
| | 2-D4-SX | 39.85 |
| | | |
| 0.1% BOL | 2-E1-DX | 34.87 |
| | 2-E1-SX | 34.41 |
| | 2-E2-DX | 31.14 |
| | 2-E2-SX | 22.82 |
| | 2-E3-DX | 29.46 |
| | 2-E3-SX | 31.69 |
| | 2-E4-DX | 35.70 |
| | 2-E4-SX | 49.25 |
| | | |

(continued)

| Treatment | Sample | Protein (mg/ml) |
|---|---|---|
| 0.5% BOL | 2-F1-DX | 33.98 |
| | 2-F1-SX | 33.65 |
| | 2-F2-DX | 19.99 |
| | 2-F2-SX | 27.11 |
| | 2-F3-DX | 19.72 |
| | 2-F3-SX | 36.35 |
| | 2-F4-DX | 27.71 |
| | 2-F4-SX | 32.24 |
| | | |
| 1% BOL | 2-G1-DX | 20.99 |
| | 2-G1-SX | 21.48 |
| | 2-G2-DX | 15.1 |
| | 2-G2-SX | 20.28 |
| | 2-G3-DX | 20.94 |
| | 2-G3-SX | 21.89 |
| | 2-G4-DX | 20.03 |
| | 2-G4-SX | 30.76 |
| [1]N/A = not available | | |

Table T-9 Protein levels in aqueous humor samples collected at the second paracentesis (Mean ± SEM).

| Treatment | Sample Group | Mean (mg/ml) | SEAM | n |
|---|---|---|---|---|
| CTR | A | 39.364 | 3.754 | 7 |
| 0.03% F | B | 20.910 | 1.648 | 7 |
| 0.1% Dex | C | 33.457 | 1.001 | 6 |
| 0.5% LE | D | 33.905 | 2.190 | 8 |
| 0.1% BOL | E | 33.667 | 2.655 | 8 |
| 0.5% BOL | F | 28.844 | 2.249 | 8 |
| 1% BOL | G | 21.435 | 1.529 | 8 |

Table T-10 Raw data of PMN numbers in aqueous humor samples collected at the second paracentesis

| Treatment | Sample | PMN (number/μl) |
|---|---|---|
| CTR | 2-A1-DX | 90 |
| | 2-A1-SX | 80 |
| | 2-A2-DX | 70 |
| | 2-A2-SX | [1]N/A |
| | 2-A3-DX | 70 |
| | 2-A3-SX | 80 |
| | 2-A4-DX | 50 |
| | 2-A4-SX | 40 |
| | | |
| 0.03% F | 2-B1-DX | 50 |
| | 2-B1-SX | 40 |
| | 2-B2-DX | N/A |
| | 2-B2-SX | 20 |
| | 2-B3-DX | 10 |
| | 2-B3-SX | 40 |
| | 2-B4-DX | 30 |
| | 2-B4-SX | 20 |
| | | |
| 0.1% Dex | 2-C1-DX | 20 |
| | 2-C1-SX | N/A |
| | 2-C2-DX | 20 |
| | 2-C2-SX | N/A |
| | 2-C3-DX | 50 |
| | 2-C3-SX | 40 |
| | 2-C4-DX | 20 |
| | 2-C4-SX | 30 |
| | | |
| 0.5% LE | 2-D1-DX | N/A |
| | 2-D1-SX | N/A |
| | 2-D2-DX | 40 |
| | 2-D2-SX | 20 |
| | 2-D3-DX | 20 |
| | 2-D3-SX | 30 |
| | 2-D4-DX | 40 |
| | 2-D4-SX | 20 |

(continued)

| Treatment | Sample | PMN (number/μl) |
|---|---|---|
| 0.1% BOL | 2-E1-DX | N/A |
| | 2-E1-SX | 20 |
| | 2-E2-DX | 40 |
| | 2-E2-SX | 50 |
| | 2-E3-DX | 20 |
| | 2-E3-SX | 20 |
| | 2-E4-DX | 20 |
| | 2-E4-SX | N/A |
| | | |
| 0.5% BOL | 2-F1-DX | 40 |
| | 2-F1-SX | 20 |
| | 2-F2-DX | 20 |
| | 2-F2-SX | 10 |
| | 2-F3-DX | 10 |
| | 2-F3-SX | 10 |
| | 2-F4-DX | 20 |
| | 2-F4-SX | 40 |
| | | |
| 1%BOL | 2-G1-DX | 30 |
| | 2-G1-SX | 20 |
| | 2-G2-DX | 30 |
| | 2-G2-SX | 40 |
| | 2-G3-DX | 20 |
| | 2-G3-SX | 30 |
| | 2-G4-DX | 40 |
| | 2-G4-SX | 20 |
| [1]N/A = not available | | |

Table T-11 PMN numbers in aqueous humor samples collected at the second paracentesis (Mean ± SEM).

| Treatment | Sample Group | Mean (number/μl) | SEM | n |
|---|---|---|---|---|
| CTR | A | 68.571 | 6.701 | 7 |
| 0.03% F | B | 30.000 | 5.345 | 7 |
| 0.1%Dex | C | 30.000 | 5.164 | 6 |
| 0.5% LE | D | 28.333 | 4.014 | 6 |
| 0.1% BOL | E | 28.333 | 5.426 | 6 |
| 0.5% BOL | F | 21.250 | 4.407 | 8 |
| 1% BOL | G | 28.750 | 2.950 | 8 |

Table T-12 Raw data of MPO activity in iris-ciliary body samples collected after the second paracentesis.

| Treatment | Sample | Iris-ciliary body weight (mg) | [1]Volume (μl) | [2]Δ/min | MPO Unit/g |
|---|---|---|---|---|---|
| CTR | A1-DX | 41.7 | 40 | 0.021 | 1.11 |
| | A1-SX | 42.3 | 40 | 0.024 | 1.26 |
| | A2-DX | 46.6 | 40 | 0.039 | 1.85 |
| | A2-SX | 40.5 | 40 | 0.037 | 2.02 |
| | A3-DX | 48.9 | 40 | 0.075 | 3.39 |
| | A3-SX | 51.1 | 40 | 0.049 | 2.12 |
| | A4-DX | 36.6 | 40 | 0.013 | 0.79 |
| | A4-SX | 38.8 | 40 | 0.019 | 1.08 |
| | | | | | |
| 0.03% F | B1-DX | 39.5 | 100 | 0.049 | 1.10 |
| | B1-SX | 42.7 | 100 | 0.082 | 1.70 |
| | B2-DX | 34.1 | 100 | 0.013 | 0.34 |
| | B2-SX | 36.6 | 100 | 0.031 | 0.75 |
| | B3-DX | 45.6 | 100 | 0.038 | 0.74 |
| | B3-SX | 38.0 | 100 | 0.027 | 0.63 |
| | B4-DX | 40.1 | 100 | 0.033 | 0.73 |
| | B4-SX | 42.6 | 100 | 0.061 | 1.27 |
| | | | | | |
| 0.1% Dex | C1-DX | 36.4 | 100 | 0.029 | 0.71 |
| | C1-SX | 45.8 | 100 | 0.031 | 0.60 |
| | C2-DX | 42.9 | 100 | 0.064 | 1.32 |
| | C2-SX | 42.7 | 100 | 0.023 | 0.48 |
| | C3-DX | 43.0 | 100 | 0.019 | 0.39 |
| | C3-SX | 46.8 | 140 | 0.024 | 0.45 |
| | C4-DX | 42.3 | 100 | 0.023 | 0.48 |
| | C4-SX | 36.1 | 100 | 0.021 | 0.51 |
| | | | | | |
| 0.5% LE | D1-DX | 38.9 | 200 | 0.026 | 0.30 |
| | D1-SX | 44.7 | 200 | 0.053 | 0.51 |
| | D2-DX | 35.9 | 200 | 0.067 | 0.81 |
| | D2-SX | 40.7 | 200 | 0.055 | 0.60 |
| | D3-DX | 46.3 | 200 | 0.076 | 0.73 |
| | D3-SX | 41.9 | 200 | 0.096 | 1.01 |
| | D4-DX | 46.7 | [3]N/A | N/A | N/A |
| | D4-SX | 32.9 | N/A | N/A | N/A |

(continued)

| Treatment | Sample | Iris-ciliary body weight (mg) | [1]Volume (μl) | [2]Δ/min | MPO Unit/g |
|---|---|---|---|---|---|
| 0.1% BOL | E1-DX | 43.6 | 100 | 0.051 | 1.04 |
| | E1-SX | 37.2 | 100 | 0.042 | 1.00 |
| | E2-DX | 32.6 | 100 | 0.042 | 1.14 |
| | E2-SX | 37.4 | 100 | 0.045 | 1.06 |
| | E3-DX | 36.2 | 100 | 0.050 | 1.22 |
| | E3-SX | 45.1 | 100 | 0.031 | 0.61 |
| | E4-DX | 30.4 | 100 | 0.036 | 1.05 |
| | E4-SX | 42.3 | 100 | 0.031 | 0.65 |
| | | | | | |
| 0.5% BOL | F1-DX | 45.8 | 100 | 0.044 | 0.85 |
| | F1-SX | 38.2 | 100 | 0.040 | 0.93 |
| | F2-DX | 34.9 | 100 | 0.031 | 0.79 |
| | F2-SX | 42.0 | 100 | 0.049 | 1.03 |
| | F3-DX | 39.1 | 100 | 0.033 | 0.75 |
| | F3-SX | 40.6 | 100 | 0.034 | 0.74 |
| | F4-DX | 36.2 | 100 | 0.022 | 0.54 |
| | F4-SX | 39.5 | 100 | 0.026 | 0.58 |
| | | | | | |
| 1% BOL | G1-DX | 32.4 | 100 | 0.024 | 0.66 |
| | G1-SX | 43.1 | 100 | 0.033 | 0.68 |
| | G2-DX | 30.6 | 100 | 0.017 | 0.49 |
| | G2-SX | 39.9 | 100 | 0.018 | 0.40 |
| | G3-DX | 41.3 | 100 | 0.016 | 0.34 |
| | G3-SX | 44.9 | 100 | 0.052 | 1.02 |
| | G4-DX | 36.6 | 100 | 0.013 | 0.31 |
| | G4-SX | 36.9 | 100 | 0.018 | 0.43 |

[1]Volume = aliquot (μl) of the supernatant diluted to 3ml for the analysis.
[2]Δ/min = mean of the slope of the line recorded every 15 sec for 5 min
[3]N/A = not available

Table T-13 MPO activity in iris-ciliary body samples collected after the second paracentesis (Mean ± SEM).

| Treatment | Sample Group | Mean MPO Unit/g | SEM | n |
|---|---|---|---|---|
| CTR | A | 1.703 | 0.297 | 8 |
| 0.03% F | B | 0.906 | 0.151 | 8 |
| 0.1% Dex | c | 0.618 | 0.106 | 8 |
| 0.5% LE | D | 0.661 | 0.102 | 6 |
| 0.1% BOL | E | 0.971 | 0.079 | 8 |
| 0.5% BOL | F | 0.775 | 0.058 | 8 |

(continued)

| Treatment | Sample Group | Mean MPO Unit/g | SEM | n |
|---|---|---|---|---|
| 1% BOL | G | 0.542 | 0.083 | 8 |

## Claims

1. A composition comprising: (a) a dissociated glucocorticoid receptor agonist ("DIGRA") having Formula I or a pharmaceutically acceptable salt thereof

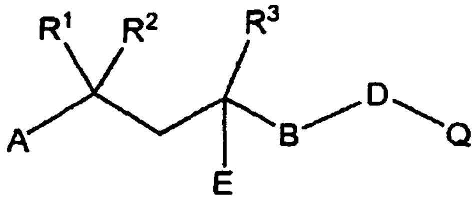

(I)

wherein A and Q are independently selected from the group consisting of aryl and heteroaryl groups substituted with at least a halogen atom, cyano group, hydroxy group, or $C_1$-$C_{10}$ alkoxy group; $R^1$ and $R^2$, and $R^3$ are independently selected from the group consisting of unsubstituted and substituted $C_1$-$C_5$ alkyl groups; B is a $C_1$-$C_5$ alkylene group; D is the -NH- or -NR'- group, wherein R' is a $C_1$-$C_5$ alkyl group; and E is the hydroxy group; and (b) an anti-inflammatory agent other than said DIGRA and pharmaceutically acceptable salt thereof;
wherein said DIGRA or a pharmaceutically acceptable salt thereof and said anti-inflammatory agent are present in said composition in an amount effective for use in treating, reducing, or ameliorating a condition or disorder selected from the group consisting of diabetic retinopathy ("DR"), age-related macular degeneration ("AMD"), diabetic macular edema ("DME"), or a combination thereof,
and wherein the composition further comprises a pharmaceutically acceptable carrier.

2. A composition comprising: (a) a dissociated glucocorticoid receptor agonist ("DIGRA") having Formula I or a pharmaceutically acceptable salt thereof

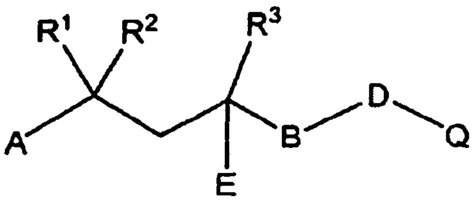

(I)

wherein A comprises a dihydrobenzofuranyl group substituted with a halogen atom; Q comprises a quinolinyl or isoquinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group; D is the -NH- group; E is the hydroxy group; and $R^3$ comprises a completely halogenated $C_1$-$C_5$ alkyl group; and (b) an anti-inflammatory agent other than said DIGRA and pharmaceutically acceptable salt thereof;
wherein said DIGRA or a pharmaceutically acceptable salt thereof and said anti-inflammatory agent are present in said composition in an amount effective for use in treating, reducing, or ameliorating a condition or disorder selected from the group consisting of diabetic retinopathy ("DR"), age-related macular degeneration ("AMD"), diabetic macular edema ("DME"), or a combination thereof,
and wherein the composition further comprises a pharmaceutically acceptable carrier.

3. The composition of claim 2 for use as in claim 2, wherein the DIGRA has Formula I

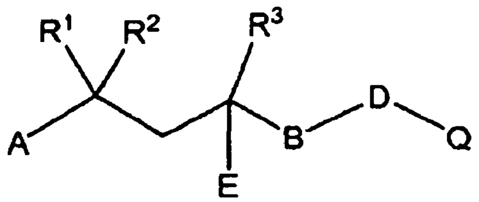

(I)

wherein A comprises a dihydrobenzofuranyl group substituted with a fluorine atom; Q comprises a quinolinyl or isoquinolinyl group substituted with a methyl group; $R^1$ and $R^2$ are independently selected from the group consisting of unsubstituted and substituted $C_1$-$C_5$ alkyl groups; B is a $C_1$-$C_3$ alkylene group; D is the -NH- group; E is the hydroxy group; and $R^3$ comprises a trifluoromethyl group.

**4.** The composition of claim 1 for use as in claim 1, wherein the DIGRA has Formula II

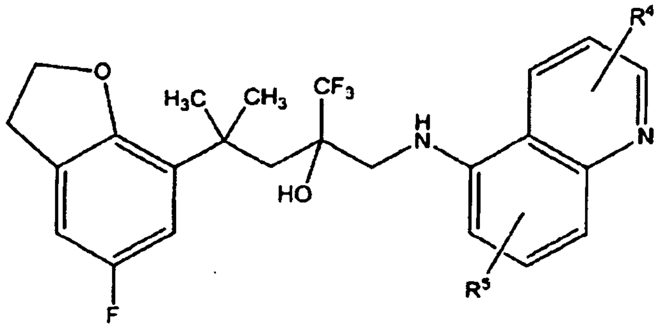

(II)

wherein $R^4$ and $R^5$ are independently selected from the group consisting of halogen, cyano, hydroxy, and $C_1$-$C_{10}$ alkoxy groups.

**5.** The composition of claim 1 for use as in claim 1, wherein the DIGRA has Formula III

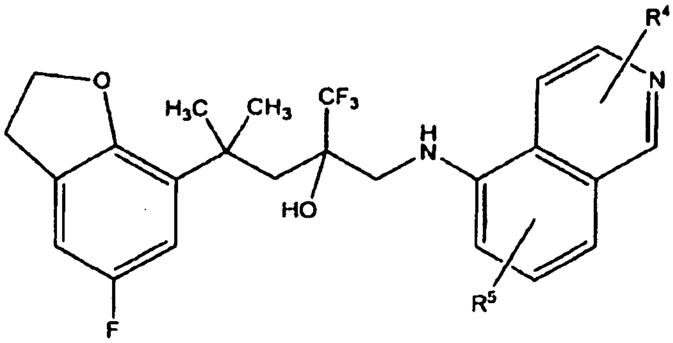

(III)

wherein $R^4$ and $R^5$ are independently selected from the group consisting of halogen, cyano, hydroxy, and $C_1$-$C_{10}$ alkoxy groups.

**6.** The composition of claim 2 for use as in claim 2, wherein the DIGRA has Formula IV

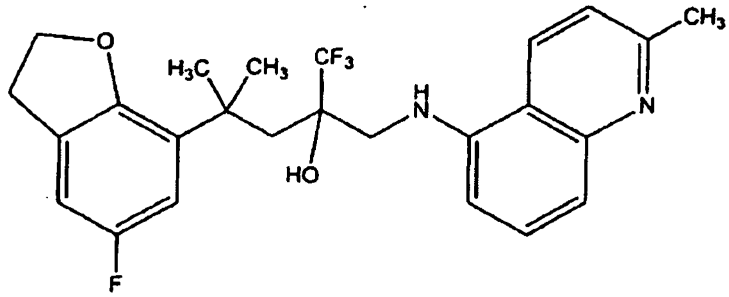

(IV)

7. The composition of claim 1 or 2 for use as in claim 1 or 2, wherein the composition causes a lower level of at least an adverse side effect in a subject than at least a glucocorticoid used to treat, reduce, or ameliorate the same condition or disorder.

8. The composition of claim 7 for use as in claim 7, wherein the anti-inflammatory agents other than said DIGRA and pharmaceutically acceptable salts thereof are selected from the group consisting of non-steroidal anti-inflammatory drugs ("NSAIDs"), peroxisome proliferator-activated receptor ("PPAR") ligands, combinations thereof, and mixtures thereof.

9. Use of: (a) a DIGRA having Formula I or a pharmaceutically acceptable salt thereof and (b) an anti-inflammatory agent other than said DIGRA and pharmaceutically acceptable salts thereof, for the preparation of a medicament for treating, reducing, or ameliorating diabetic retinopathy ("DR"), age-related macular degeneration ("AMD"), diabetic macular edema ("DME"), or a combination thereof

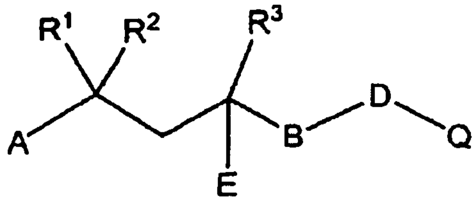

(I)

wherein A and Q are independently selected from the group consisting of aryl and heteroaryl groups substituted with at least a halogen atom, cyano group, hydroxy group, or $C_1$-$C_{10}$ alkoxy group; $R^1$, $R^2$, and $R^3$ are independently selected from the group consisting of unsubstituted and substituted $C_1$-$C_5$ alkyl groups; B is a $C_1$-$C_5$ alkylene group; D is the -NH- or NR'- group, wherein R' is a $C_1$-$C_5$ alkyl group; and E is the hydroxy group.

10. Use of: (a) a DIGRA having Formula I or a pharmaceutically acceptable salt thereof and (b) an anti-inflammatory agent other than said DIGRA and pharmaceutically acceptable salts thereof, for the preparation of a medicament for treating, reducing, or ameliorating diabetic retinopathy ("DR"), age-related macular degeneration ("AMD"), diabetic macular edema ("DME"), or a combination thereof

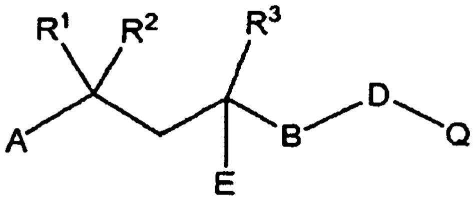

(I)

wherein A comprises a dihydrobenzofuranyl group substituted with a halogen atom; Q comprises a quinolinyl or isoquinolinyl group substituted with a $C_1$-$C_{10}$ alkyl group; $R^1$ and $R^2$ are independently selected from the group consisting of unsubstituted and substituted $C_1$-$C_5$ alkyl groups; B is a $C_1$-$C_3$ alkylene group; D is the -NH- group; E is the hydroxy group; and $R^3$ comprises a completely halogenated $C_1$-$C_5$ alkyl group.

11. The use of claim 10, wherein A comprises a dihydrobenzofuranyl group substituted with a fluorine atom; Q comprises a quinolinyl or isoquinolinyl group substituted with a methyl group; $R^1$ and $R^2$ are independently selected from the

group consisting of unsubstituted and substituted $C_1$-$C_5$ alkyl groups; B is a $C_1$-$C_3$ alkylene group; D is the -NH- group; E is the hydroxy group; and $R^3$ comprises a trifluoromethyl group.

**12.** The use of claim 9, wherein the DIGRA has Formula II

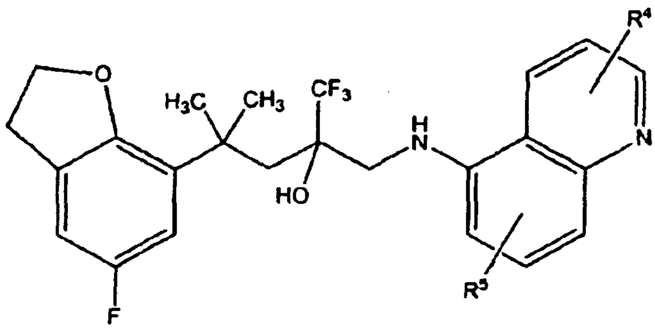

(II)

wherein $R^4$ and $R^5$ are independently selected from the group consisting of halogen, cyano, hydroxy, and $C_1$-$C_{10}$ alkoxy groups.

**13.** The use of claim 9, wherein the DIGRA has Formula III

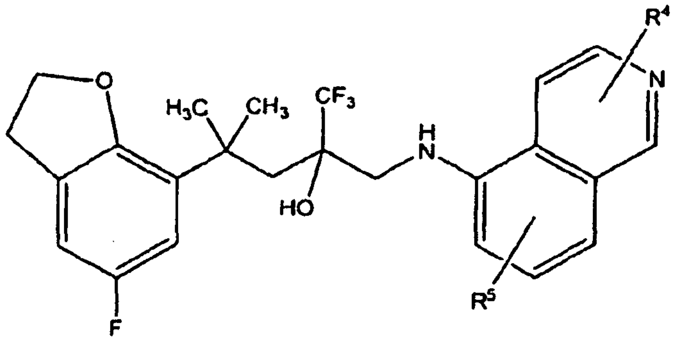

(III)

wherein $R^4$ and $R^5$ are independently selected from the group consisting of halogen, cyano, hydroxy, and $C_1$-$C_{10}$ alkoxy groups.

**14.** The use of claim 10, wherein the DIGRA has Formula IV

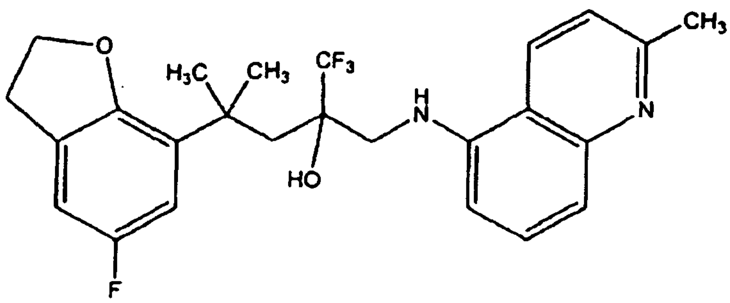

(IV)

**15.** The use of claim 9 or 10, wherein the composition causes a lower level of at least an adverse side effect in a subject than at least a glucocorticoid used to treat, reduce, or ameliorate the same condition or disorder.

**Patentansprüche**

**1.** Eine Zusammensetzung umfassend: (a) einen dissoziierten Glucocorticoid-Rezeptor-Agonisten ("DIGRA") der Formel I oder ein pharmazeutisch zulässiges Salz davon

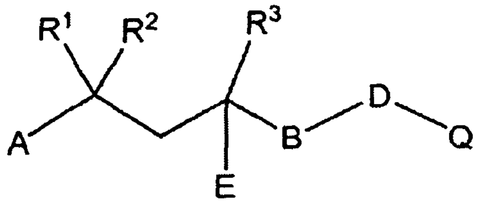

(I)

wobei A und Q unabhängig voneinander ausgewählt sind aus der Gruppe bestehend aus Aryl- oder Heteroarylgruppen substituiert mit wenigstens einem Halogenatom, Cyanogruppe, Hydroxygruppe oder $C_1$-$C_{10}$ Alkoxygruppe; $R^1$ und $R^2$ und $R^3$ unabhängig voneinander ausgewählt sind aus der Gruppe bestehend aus unsubstituierten und substituierten $C_1$-$C_5$ Alkylgruppen; B eine $C_1$-$C_5$ Alkylengruppe ist; D eine -NH- oder -NR'-Gruppe ist, wobei R' eine $C_1$-$C_5$ Alkylgruppe ist und E eine Hydroxygruppe ist und (b) einen entzündungshemmenden Wirkstoff der von besagtem DIGRA und pharmazeutisch zulässigem Salz davon verschieden ist;
wobei besagtes DIGRA oder pharmazeutisch zulässiges Salz davon und besagter entzündungshemmender Wirkstoff in besagter Zusammensetzung in Mengen vorhanden sind, die für die Behandlung, Reduzierung oder Verbesserung von Beschwerden oder einer Erkrankung ausgewählt aus der Gruppe bestehend aus diabetischer Rethinopathie ("DR"), altersbedingter Makuladegeneration ("AMD"), diabetischen Makulaödemen oder einer Kombination daraus wirksam sind,
und wobei die Zusammensetzung weiterhin einen pharmazeutisch zulässigen Träger umfasst.

**2.** Eine Zusammensetzung umfassend: (a) einen dissoziierten Glucocorticoid-Rezeptor-Agonisten ("DIGRA") der Formel I oder ein pharmazeutisch zulässiges Salz davon

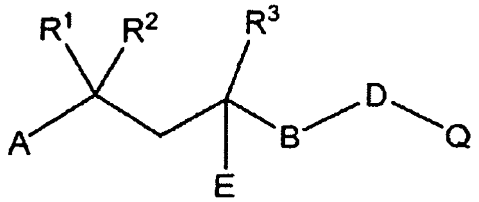

(I)

wobei A eine Dihydrobenzofuranylgruppe substituiert mit einem Halogenatom umfasst; Q eine Quinolinyl- oder Isoquinolinylgruppe substituiert mit einer $C_1$-$C_{10}$ Alkylgruppe umfasst; $R^1$ und $R^2$ unabhängig voneinander ausgewählt sind aus der Gruppe bestehend aus unsubstituierten und substituierten $C_1$-$C_5$ Alkylgruppen; B eine $C_1$-$C_3$ Alkylengruppen ist; D eine -NH-Gruppe ist; E eine Hydroxygruppe ist und $R^3$ eine vollständig halogenierte $C_1$-$C_5$ Alkylgruppe umfasst und (b) einen entzündungshemmenden Wirkstoff der von besagtem DIGRA und pharmazeutisch zulässigem Salz davon verschieden ist;
wobei besagtes DIGRA oder pharmazeutisch zulässiges Salz davon und besagter entzündungshemmender Wirkstoff in besagter Zusammensetzung in Mengen vorhanden sind, die für die Behandlung, Reduzierung oder Verbesserung von Beschwerden oder einer Krankheit ausgewählt aus der Gruppe bestehend aus diabetischer Rethinopathie ("DR"), altersbedingter Makuladegeneration ("AMD"), diabetischen Makulaödemen oder einer Kombination daraus wirksam sind,
und wobei die Zusammensetzung weiterhin einen pharmazeutisch zulässigen Träger umfasst.

**3.** Die Zusammensetzung gemäß Anspruch 2 zur Verwendung gemäß Anspruch 2, wobei DIGRA die Formel I aufweist

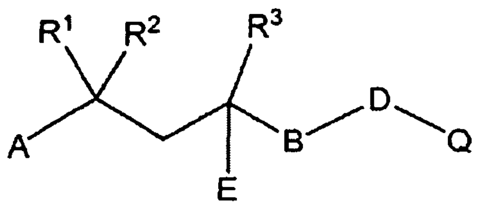

(I)

wobei A eine Dihydrobenzofuranylgruppe substituiert mit einem Fluoratom umfasst; Q eine Quinolinyl- oder Isoquinolinylgruppe substituiert mit einer Methylgruppe umfasst; $R^1$ und $R^2$ unabhängig voneinander ausgewählt sind aus der Gruppe bestehend aus unsubstituierten und substituierten $C_1$-$C_5$ Alkylgruppen; B eine $C_1$-$C_3$ Alkylengruppen ist; D eine -NH-Gruppe ist; E eine Hydroxygruppe ist und $R^3$ eine Trifluormethylgruppe umfasst.

**4.** Die Zusammensetzung gemäß Anspruch 1 zur Verwendung gemäß Anspruch 1, wobei DIGRA die Formel II aufweist

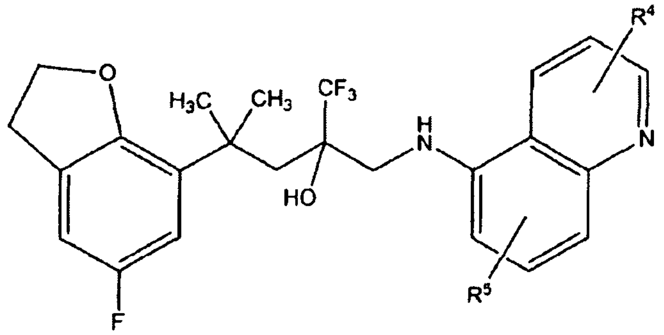

(II)

wobei $R^4$ und $R^5$ unabhängig voneinander ausgewählt sind aus der Gruppe bestehend aus Halogen, Cyano, Hydroxy und $C_1$-$C_{10}$ Alkoxygruppen.

**5.** Die Zusammensetzung gemäß Anspruch 1 zur Verwendung gemäß Anspruch 1, wobei DIGRA die Formel III aufweist

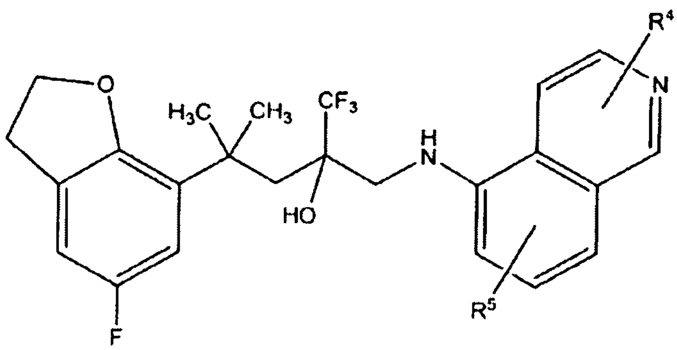

(III)

wobei $R^4$ und $R^5$ unabhängig voneinander ausgewählt sind aus der Gruppe bestehend aus Halogen, Cyano, Hydroxy und $C_1$-$C_{10}$ Alkoxygruppen.

**6.** Die Zusammensetzung gemäß Anspruch 2 zur Verwendung gemäß Anspruch 2, wobei DIGRA die Formel IV aufweist

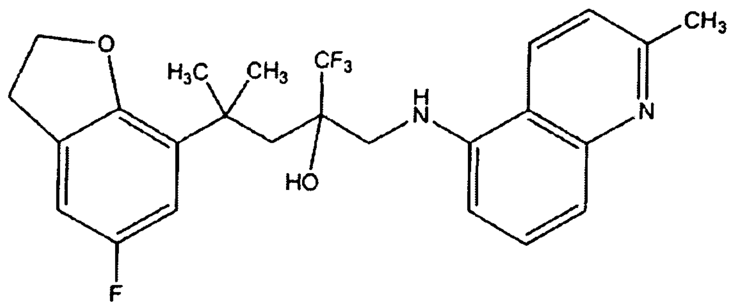

(IV).

7. Die Zusammensetzung gemäß Anspruch 1 oder 2 zur Verwendung gemäß Anspruch 1 oder 2, wobei die Zusammensetzung einen geringeren Grad an wenigstens einer unerwünschten Nebenwirkung in einem Subjekt verursacht als wenigstens ein Glucocorticoid, das zur Behandlung, Reduzierung oder Verbesserung derselben Beschwerden oder derselben Krankheit verwendet wird.

8. Die Zusammensetzung gemäß Anspruch 7 zur Verwendung gemäß Anspruch 7, wobei der entzündungshemmende Wirkstoff der von besagtem DIGRA und pharmazeutische zulässigem Salz davon verschieden ist, ausgewählt ist aus der Gruppe bestehend aus nicht-steroidalen entzündungshemmenden Arzneimitteln ("NSAIDs"), Peroxisome-Proliferator aktivierten Rezeptor ("PPAR") Liganden, Kombinationen daraus und Mischungen davon.

9. Verwendung von: (a) einem DIGRA der Formel I oder einem pharmazeutisch zulässigem Salz davon und (b) einem entzündungshemmenden Wirkstoff der von besagtem DIGRA und pharmazeutische zulässigem Salz davon verschieden ist, für die Herstellung eines Medikaments zur Behandlung, Reduzierung oder Vorbesserung diabetischer Rethinopathie ("DR"), altersbedingter Makuladegeneration ("AMD"), diabetischen Makulaödemen oder einer Kombination daraus

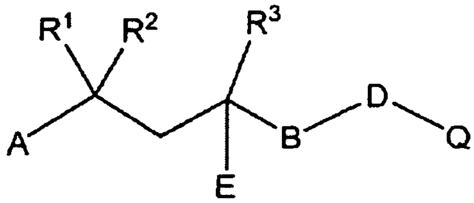

(I)

wobei A und Q unabhängig voneinander ausgewählt sind aus der Gruppe bestehend aus Aryl- oder Heteroarylgruppen substituiert mit wenigstens einem Halogenatom, Cyanogruppe, Hydroxygruppe oder $C_1$-$C_{10}$ Alkoxygruppe; $R^1$, $R^2$ und $R^3$ unabhängig voneinander ausgewählt sind aus der Gruppe bestehend aus unsubstituierten und substituierten $C_1$-$C_5$ Alkylgruppen; B eine $C_1$-$C_5$ Alkylengruppe ist; D eine -NH- oder -NR'-Gruppe ist, wobei R' eine $C_1$-$C_5$ Alkylgruppe ist und E eine Hydroxygruppe ist.

10. Verwendung von: (a) einem DIGRA der Formel I oder einem pharmazeutisch zulässigem Salz davon und (b) einem entzündungshemmenden Wirkstoff der von besagtem DIGRA und pharmazeutisch zulässigem Salz davon verschieden ist, für die Herstellung eines Medikaments zur Behandlung, Reduzierung oder Verbesserung von diabetischer Rethinopathie ("DR"), altersbedingter Makuladegeneration ("AMD"), diabetischen Makulaödemen oder einer Kombination daraus,

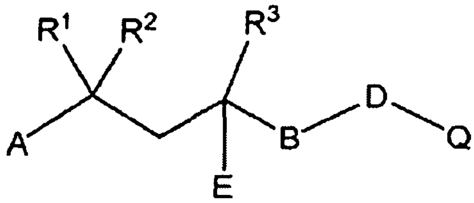

(I)

wobei A eine Dihydrobenzofuranylgruppe substituiert mit einem Halogenatom umfasst; Q eine Quinolinyl- oder Isoquinolinylgruppe substituiert mit einer $C_1$-$C_{10}$ Alkylgruppe umfasst; $R^1$ und $R^2$ unabhängig voneinander ausgewählt sind aus der Gruppe bestehend aus unsubstituierten und substituierten $C_1$-$C_5$ Alkylgruppen; B eine $C_1$-$C_3$ Alkylengruppen ist; D eine -NH-Gruppe ist; E eine Hydroxygruppe ist und $R^3$ eine vollständig halogenierte $C_1$-$C_5$ Alkylgruppe umfasst.

**11.** Die Verwendung gemäß Anspruch 10, wobei A eine Dihydrobenzofuranylgruppe substituiert mit einem Fluoratom umfasst; Q eine Quinolinyl- oder Isoquinolinylgruppe substituiert mit einer Methylgruppe umfasst; $R^1$ und $R^2$ unabhängig voneinander ausgewählt sind aus der Gruppe bestehend aus unsubstituierten und substituierten $C_1$-$C_5$ Alkylgruppen; B eine $C_1$-$C_3$ Alkylengruppe ist; D eine -NH-Gruppe ist; E eine Hydroxygruppe ist und $R^3$ eine Trifluormethylgruppe umfasst.

**12.** Die Verwendung gemäß Anspruch 9, wobei DIGRA die Formel II aufweist

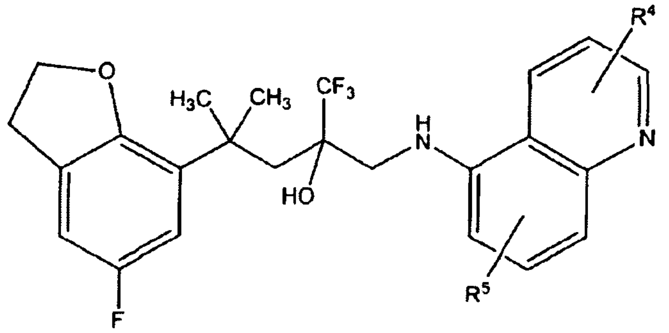

(II)

wobei $R^4$ und $R^5$ unabhängig voneinander ausgewählt sind aus der Gruppe bestehend aus Halogen, Cyano, Hydroxy und $C_1$-$C_{10}$ Alkoxygruppen.

**13.** Die Verwendung gemäß Anspruch 9, wobei DIGRA die Formel III aufweist

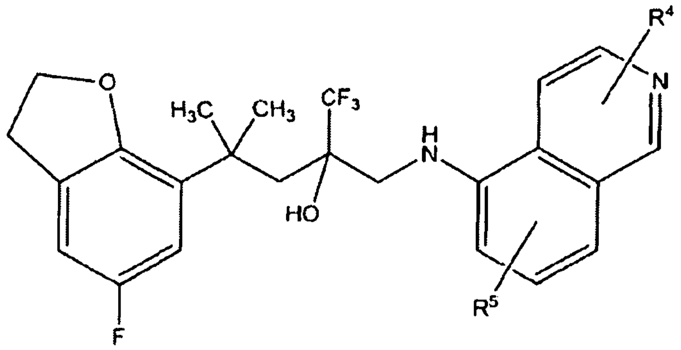

(III)

wobei $R^4$ und $R^5$ unabhängig voneinander ausgewählt sind aus der Gruppe bestehend aus Halogen, Cyano, Hydroxy und $C_1$-$C_{10}$ Alkoxygruppen.

**14.** Die Verwendung gemäß Anspruch 10, wobei DIGRA die Formel IV aufweist

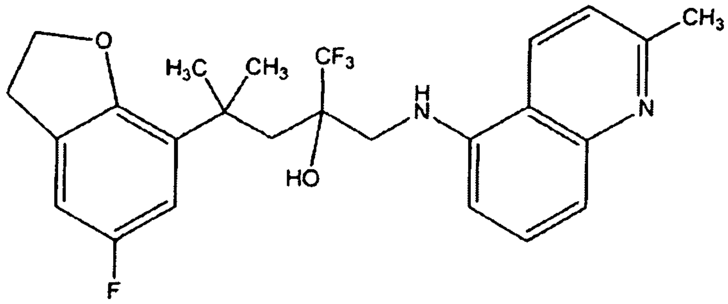

(IV)

**15.** Die Verwendung gemäß Anspruch 9 oder 10, wobei die Zusammensetzung einen geringeren Grad an wenigstens einer unerwünschten Nebenwirkung in einem Subjekt verursacht als wenigstens ein Glucocorticoid, das zur Behandlung, Reduzierung oder Verbesserung derselben Beschwerden oder derselben Krankheit verwendet wird.

**Revendications**

**1.** Composition comprenant : (a) un agoniste des récepteurs des glucocorticoïdes dissociés (« DIGRA ») de formule 1 ou un sel pharmaceutiquement acceptable de celui-ci

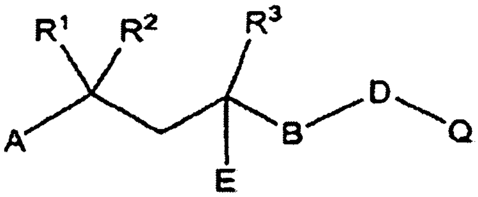

(I)

dans laquelle A et Q sont indépendamment sélectionnés dans le groupe constitué par les groupes aryles et hétéroaryles substitués par au moins un atome d'halogène, un groupe cyano, un groupe hydroxy ou un groupe alcoxy en $C_1$-$C_{10}$; $R^1$, $R^2$ et $R^3$ sont indépendamment sélectionnés dans le groupe constitué par les groupes alkyles en $C_1$-$C_5$ non substitués et substitués ; B est un groupe alkylène en $C_1$-$C_5$ ; D est le groupe -NH- ou -NR'-, où R' est un groupe alkyle en $C_1$-$C_5$ ; et E est le groupe hydroxy ; et (b) un agent anti-inflammatoire autre que ledit DIGRA et un sel pharmaceutiquement acceptable de celui-ci ;
dans laquelle ledit DIGRA ou un sel pharmaceutiquement acceptable de celui-ci et ledit agent anti-inflammatoire sont présents dans ladite composition dans une quantité efficace pour traiter, réduire ou améliorer une pathologie ou un trouble sélectionné dans le groupe constitué par la rétinopathie diabétique (« RD »), la dégénérescence maculaire liée à l'âge (« DMA »), l'oedème maculaire diabétique (« OMD ») ou une combinaison de ceux-ci,
et dans laquelle la composition comprend en outre un support pharmaceutiquement acceptable.

**2.** Composition comprenant : (a) un agoniste des récepteurs des glucocorticoïdes dissociés (« DIGRA ») de formule 1 ou un sel pharmaceutiquement acceptable de celui-ci

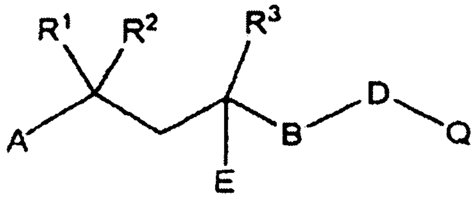

(I)

dans laquelle A comprend un groupe dihydrobenzofuranyle substitué par un atome d'halogène ; Q comprend un groupe quinoléinyle ou isoquinoléinyle substitué par un groupe alkyle en $C_1$-$C_{10}$ ; $R^1$ et $R^2$ sont indépendamment sélectionnés dans le groupe constitué par les groupes alkyles en $C_1$-$C_5$ non substitués et substitués ; B est un groupe alkylène en $C_1$-$C_3$ ; D est le groupe -NH- ; E est le groupe hydroxy ; et $R^3$ comprend un groupe alkyle en $C_1$-$C_5$ complètement halogéné ; et (b) un agent anti-inflammatoire autre que ledit DIGRA et un sel pharmaceutiquement acceptable de celui-ci ;
dans laquelle ledit DIGRA ou un sel pharmaceutiquement acceptable de celui-ci et ledit agent anti-inflammatoire sont présents dans ladite composition dans une quantité efficace pour traiter, réduire ou améliorer une pathologie ou un trouble sélectionné dans le groupe constitué par la rétinopathie diabétique (« RD »), la dégénérescence maculaire liée à l'âge (« DMA »), l'oedème maculaire diabétique (« OMD ») ou une combinaison de ceux-ci,
et dans laquelle la composition comprend en outre un support pharmaceutiquement acceptable.

**3.** Composition de la revendication 2, pour une utilisation telle que dans la revendication 2, dans laquelle le DIGRA est de formule I

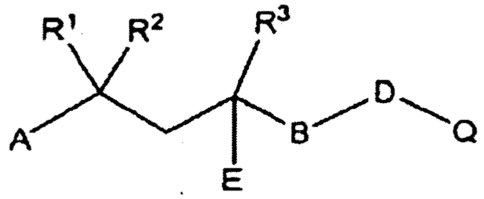

(I)

dans laquelle A comprend un groupe dihydrobenzofuranyle substitué par un atome de fluor ; Q comprend un groupe quinoléinyle ou isoquinoléinyle substitué par un groupe méthyle ; $R^1$ et $R^2$ sont indépendamment sélectionnés dans le groupe constitué par les groupes alkyles en $C_1$-$C_5$ non substitués et substitués ; B est un groupe alkylène en $C_1$-$C_3$ ; D est le groupe NH- ; E est le groupe hydroxy ; et $R^3$ comprend un groupe trifluorométhyle.

**4.** Composition de la revendication 2, pour une utilisation telle que dans la revendication 2, dans laquelle le DIGRA est de formule II

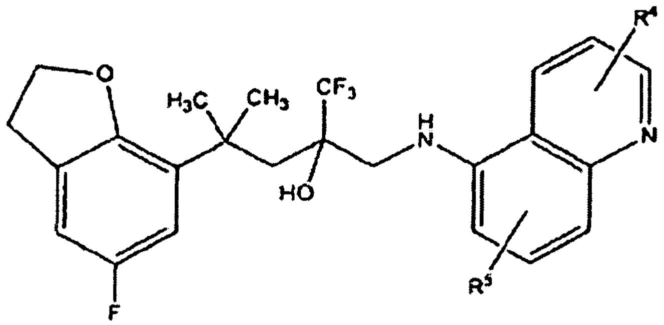

(II)

dans laquelle $R^4$ et $R^5$ sont indépendamment sélectionnés dans le groupe constitué par l'halogène, le cyano, l'hydroxy, et les groupes alcoxy en $C_1$-$C_{10}$.

**5.** Composition de la revendication 1, pour une utilisation telle que dans la revendication 1, dans laquelle le DIGRA est de formule III

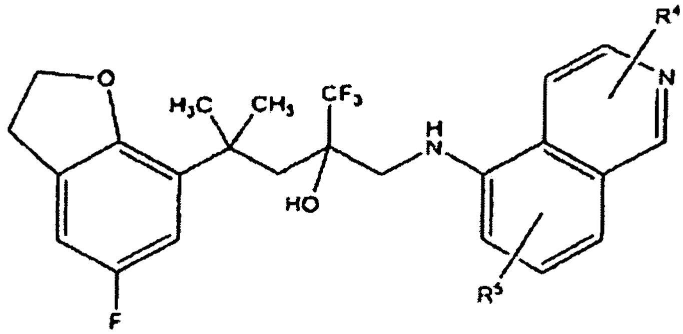

(III)

dans laquelle $R^4$ et $R^5$ sont indépendamment sélectionnés dans le groupe constitué par l'halogène, le cyano,

l'hydroxy, et les groupes alcoxy en $C_1$-$C_{10}$.

6. Composition de la revendication 2, pour une utilisation telle que dans la revendication 2, dans laquelle le DIGRA est de formule IV

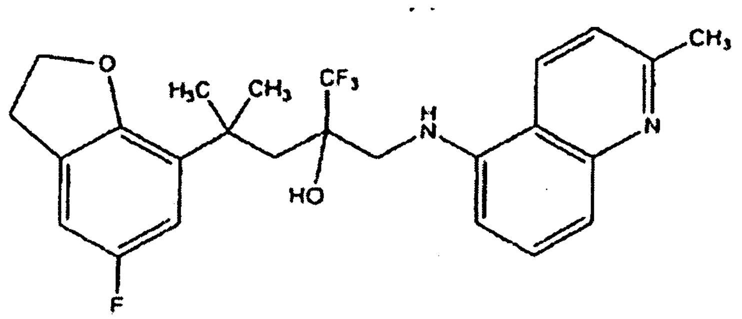

(IV)

7. Composition de la revendication 1 ou 2, pour une utilisation telle que dans la revendication 1 ou 2, dans laquelle la composition provoque, chez un sujet, un niveau d'au moins un effet secondaire indésirable inférieur à celui d'au moins un glucocorticoïde utilisé pour traiter, réduire ou améliorer cette même pathologie ou ce même trouble.

8. Composition de la revendication 7, pour une utilisation telle que dans la revendication 7, dans laquelle les agents anti-inflammatoires autres que ledit DIGRA et les sels pharmaceutiquement acceptables de celui-ci sont sélectionnés dans le groupe constitué par les anti-inflammatoire non stéroïdiens (« AINS »), les ligands des récepteurs activés par les proliférateurs de peroxysomes (« RAPP »), des combinaisons de ceux-ci et des mélanges de ceux-ci.

9. Utilisation de : (a) un DIGRA de formule I ou un sel pharmaceutiquement acceptable de celui-ci et de (b) un agent anti-inflammatoire autre que ledit DIGRA et de sels pharmaceutiquement acceptables de celui-ci, pour la préparation d'un médicament pour traiter, réduire ou améliorer la rétinopathie diabétique (« RD »), la dégénérescence maculaire liée à l'âge (« DMA »), l'oedème maculaire diabétique (« OMD ») ou une combinaison de ceux-ci

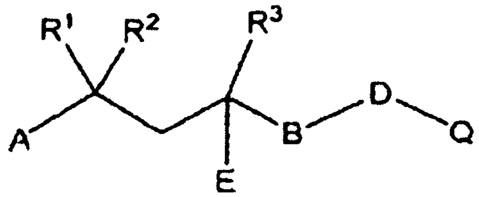

(I)

dans laquelle A et Q sont indépendamment sélectionnés dans le groupe constitué par les groupes aryles et hétéroaryles substitués par au moins un atome d'halogène, un groupe cyano, un groupe hydroxy ou un groupe alcoxy en $C_1$-$C_{10}$ ; $R^1$, $R^2$ et $R^3$ sont indépendamment sélectionnés dans le groupe constitué par les groupes alkyles en $C_1$-$C_5$ non substitués et substitués ; B est un groupe alkylène en $C_1$-$C_5$ ; D est le groupe - NH- ou -NR'-, où R' est un groupe alkyle en $C_1$-$C_5$ ; et E est le groupe hydroxy.

10. Utilisation de : (a) un DIGRA de formule I ou un sel pharmaceutiquement acceptable de celui-ci et de (b) un agent anti-inflammatoire autre que ledit DIGRA et de sels pharmaceutiquement acceptables de celui-ci, pour la préparation d'un médicament pour traiter, réduire ou améliorer la rétinopathie diabétique (« RD »), la dégénérescence maculaire liée à l'âge (« DMA »), l'oedème maculaire diabétique (« OMD ») ou une combinaison de ceux-ci

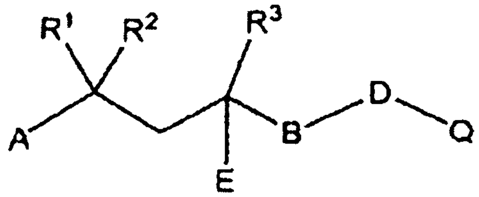

(I)

dans laquelle A comprend un groupe dihydrobenzofuranyle substitué par un atome d'halogène ; Q comprend un groupe quinoléinyle ou isoquinoléinyle substitué par un groupe alkyle en $C_1$-$C_{10}$ ; $R^1$ et $R^2$ sont indépendamment sélectionnés dans le groupe constitué par les groupes alkyles en $C_1$-$C_5$ non substitués et substitués ; B est un groupe alkylène en $C_1$-$C_3$ ; D est le groupe NH- ; E est le groupe hydroxy ; et $R^3$ comprend un groupe alkyle en $C_1$-$C_5$ complètement halogéné.

**11.** Utilisation de la revendication 10, dans laquelle A comprend un groupe dihydrobenzofuranyle substitué par un atome de fluor ; Q comprend un groupe quinoléinyle ou isoquinoléinyle substitué par un groupe méthyle ; $R^1$ et $R^2$ sont indépendamment sélectionnés dans le groupe constitué par les groupes alkyles en $C_1$-$C_5$ non substitués et substitués ; B est un groupe alkylène en $C_1$-$C_3$ ; D est le groupe - NH- ; E est le groupe hydroxy ; et $R^3$ comprend un groupe trifluorométhyle.

**12.** Utilisation de la revendication 9, dans laquelle le DIGRA est de formule II

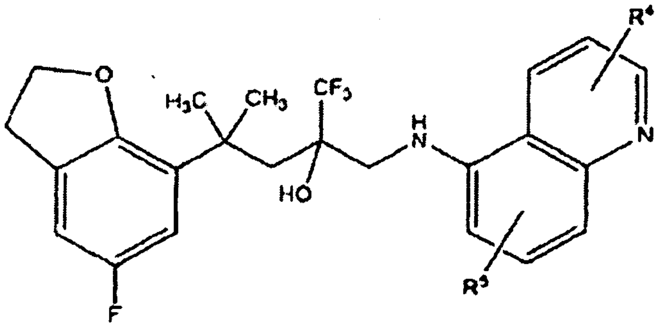

(II)

dans laquelle $R^4$ et $R^5$ sont indépendamment sélectionnés dans le groupe constitué par l'halogène, le cyano, l'hydroxy, et les groupes alcoxy en $C_1$-$C_{10}$.

**13.** Utilisation de la revendication 9, dans laquelle le DIGRA est de formule III

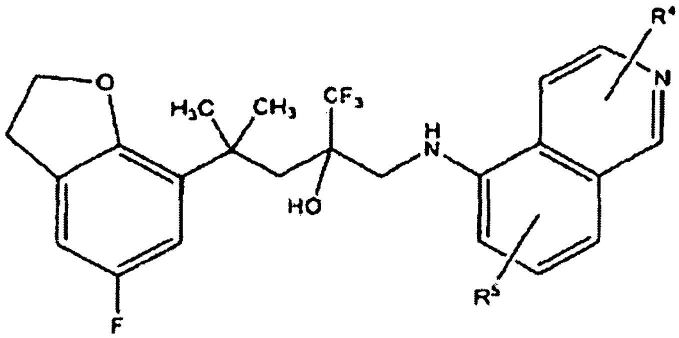

(III)

dans laquelle $R^4$ et $R^5$ sont indépendamment sélectionnés dans le groupe constitué par l'halogène, le cyano, l'hydroxy, et les groupes alcoxy en $C_1$-$C_{10}$.

**14.** Utilisation de la revendication 10, dans laquelle le DIGRA est de formule IV

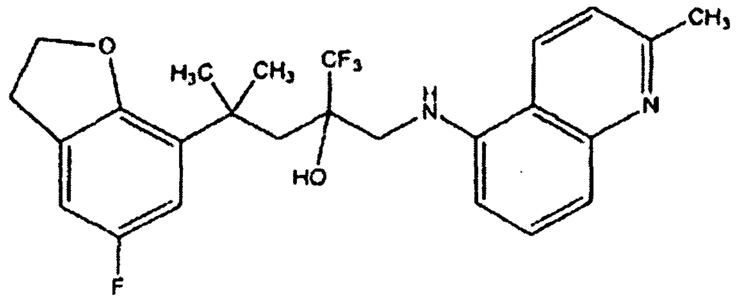

(IV)

**15.** Utilisation de la revendication 9 ou 10, dans laquelle la composition provoque, chez un sujet, un niveau d'au moins un effet secondaire indésirable inférieur à celui d'au moins un glucocorticoïde utilisé pour traiter, réduire ou améliorer cette même pathologie ou ce même trouble.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006050998 A
- US 6897224 B
- US 6903215 B
- US 6960581 B
- US 20060116396 A
- WO 2006050998A1 A
- US 20040029932 A
- US 20040162321 A
- US 20040224992 A
- US 20050059714 A
- US 20050176706 A
- US 20050203128 A
- US 20050234091 A
- US 20050282881 A
- US 20060014787 A
- US 20060030561 A
- US 20060189646 A
- US 20060189647 A
- US 6242196 B, Spiegelman
- US 6316465 B
- US 6426335 B
- US 6168778 B
- US 6147204 B
- US 6051698 B
- US 6051020 B
- US 6958340 B
- US 6514971 B
- US 6448077 B
- US 20050233921 A
- US 20050244475 A
- US 20050143442 A
- US 20060014252 A
- US 20030282849 A
- US 79760806 P
- US 5378475 A
- US 5773019 A
- US 5902598 A
- US 6001386 A
- US 6051576 A
- US 6726918 A
- US 7015240 B
- US 7060695 B
- US 20040219512 A


### Non-patent literature cited in the description


- **A.P. Adamis.** *British J. Ophthalmol.,* 2002, vol. 86, 363
- **S. Ishida et al.** *Invest. Ophthalmol. & Visual Sci.,* 2003, vol. 44 (5), 2155
- **I. Kim et al.** *Biol. Chem.,* 2001, vol. 276 (10), 7614
- **J.F. Navarro ; C. Mora.** *Nephrol. Dial. Transplant,* 2005, vol. 20, 2601
- **A.M. Joussen et al.** *FASEB J.,* 2004, vol. 18, 1450
- *Science,* 2006, vol. 311, 1704
- **M.A. Speicher et al.** *Expert Opinion on Emerging Drugs,* 2003, vol. 8 (1), 239
- **E.A. Felinski ; D.A. Antonetti.** *Curr. Eye Research,* 2005, vol. 30 (11), 49
- **G.M. Corner ; T.A. Ciulla.** *Expert Opinion on Emerging Drugs,* 2005, vol. 10 (2), 441
- **V. Vasumathy et al.** *Ophthalmic Practice,* 2006, vol. 54 (2), 133
- **P. Massin et al.** *Ophthalmology,* 2004, vol. 111 (2), 218
- **W.W. Lai et al.** *Clin. Experiment Ophthalmol.,* 2004, vol. 32 (6), 563
- **Johns Hopkins.** *University School of Medicine and Oregon Health Science University,* 2006, www.clinicaltrials.gov
- **P.J. Barnes.** *Clin. Sci.,* 1998, vol. 94, 557-572
- **S. Wissink et al.** *Mol. Endocrinol.,* 1998, vol. 12 (3), 354-363
- **P.J. Barnes ; M. Karin.** *New Engl. J. Med.,* 1997, vol. 336, 1066-1077
- **H. Schäcke et al.** *Pharmacol. Ther.,* 2002, vol. 96, 23-43
- The Complete Drug Reference. **Martindale.** Martindale. Pharmaceutical Press, London, 2005, 1411-1416
- **Remington.** The Science and Practice of Pharmacy. Lippincott Williams & Wilkins, 2006, 291
- **C-Y. Jiang et al.** *Nature,* 1998, vol. 391, 82-86
- **A.E. Giorgini et al.** *Horm. Metab. Res.,* 1999, vol. 31, 1-4
- **X. Xin et al.** *J. Biol. Chem.,* 1999, vol. 274, 9116-9121
- **Eakins KE.** Prostaglandin and non prostaglandin-mediated breakdown of the blood-aqueous barrier. *Exp Eye Res,* 1977, vol. 25, 483-498

- **Neufeld AH ; Sears ML.** The site of action of prostaglandin E2 on the disruption of the blood-aqueous barrier in the rabbit eye. *Exp Eye Res,* 1973, vol. 17, 445-448
- **Unger WG ; Cole DP ; Hammond B.** Disruption of the blood-aqueous barrier following paracentesis in the rabbit. *Exp Eye Res,* 1975, vol. 20, 255-270
- Autacoids and Neuropeptides. **Stjemschantz J.** Pharmacology of the Eye. Springer-Verlag, 1984, 311-365
- **Williams RN ; Paterson CA ; Eakins KE ; Bhattacherjee P.** Quantification of ocular inflammation: evaluation of polymorphonuclear leukocyte infiltration by measuring myeloperoxidase activity. *Curr Eye Res,* 1983, vol. 2, 465-469